(19) Europäisches Patentamt
European Patent Office
Office européen des brevets

(11) EP 1 505 942 B1

(12) EUROPEAN PATENT SPECIFICATION

(45) Date of publication and mention of the grant of the patent:
**13.08.2008 Bulletin 2008/33**

(21) Application number: **03722135.5**

(22) Date of filing: **12.05.2003**

(51) Int Cl.:
***A61K 6/00*** *(2006.01)*

(86) International application number:
**PCT/CA2003/000680**

(87) International publication number:
**WO 2003/094829 (20.11.2003 Gazette 2003/47)**

# (54) PATHOGEN VACCINES AND METHODS FOR USING THE SAME

PATHOGENE IMPSTOFFE UND VERFAHREN ZUR DEREN VERWENDUNG

VACCINS CONTRE DES MALADIES PRODUITES PAR LES PATHOGENES ET METHODES D'UTILISATION DESDITS VACCINS

(84) Designated Contracting States:
**AT BE BG CH CY CZ DE DK EE ES FI FR GB GR HU IE IT LI LU MC NL PT RO SE SI SK TR**

(30) Priority: **10.05.2002 US 379343 P**
**07.11.2002 US 290545**
**12.03.2003 US 454298 P**

(43) Date of publication of application:
**16.02.2005 Bulletin 2005/07**

(73) Proprietor: **Tekmira Pharmaceuticals Corporation**
**Burnaby BC V5J 5J8 (CA)**

(72) Inventors:
- **SEMPLE, Sean**
  **Vancouver, British Columbia V6G 3E3 (CA)**
- **CHIKH, Ghania**
  **Vancouver, British Columbia, V6G 1M9 (CA)**
- **HOPE, Michael, J.**
  **Vancouver, British Columbia V6R 2K2 (CA)**
- **TAM, Ying, K.**
  **Vancouver, British Columbia V6S 1C3 (CA)**

(74) Representative: **Lee, Nicholas John et al**
**Kilburn & Strode**
**20 Red Lion Street**
**London WC1R 4PJ (GB)**

(56) References cited:
**WO-A-01/15726** **WO-A-03/026688**
**WO-A-03/039595**

- **SEMPLE SEAN C ET AL: "Efficient encapsulation of antisense oligonucleotides in lipid vesicles using ionizable aminolipids: Formation of novel small multilamellar vesicle structures." BIOCHIMICA ET BIOPHYSICA ACTA, vol. 1510, no. 1-2, 2001, pages 152-166, XP004248775 ISSN: 0006-3002**
- **MUI B ET AL: "Immune stimulation by a CpG-containing oligodeoxynucleotide is enhanced when encapsulated and delivered in lipid particles" JOURNAL OF PHARMACOLOGY AND EXPERIMENTAL THERAPEUTICS, AMERICAN SOCIETY FOR PHARMACOLOGY AND, US, vol. 298, no. 3, September 2001 (2001-09), pages 1185-1192, XP002224996 ISSN: 0022-3565**
- **MCCLUSKIE M J ET AL: "The potential of oligodeoxynucleotides as mucosal and parenteral adjuvants" VACCINE, BUTTERWORTH SCIENTIFIC. GUILDFORD, GB, vol. 19, no. 17-19, 21 March 2001 (2001-03-21), pages 2657-2660, XP004231093 ISSN: 0264-410X**

EP 1 505 942 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

### TECHNICAL FIELD

This invention relates generally to the field of vaccines against pathogens, and more specifically to vaccine formulations that include antigenic epitopes from a pathogen associated with a lipid nucleic acid particle that enhances stimulation of a Th1 type humoral immune response.

### BACKGROUND OF THE INVENTION

The immune system is an extraordinarily complex combination of cells and compositions that protects a mammalian host against a wide variety of pathogens, while surveiling the body against deleterious aberrations. One branch of the immune system involves the cells that carry out immune system functions, including both (a) lymphocytes, such as the bone marrow-derived B-lymphocytes, the thymus-derived T lymphocytes and natural-killer (NK) cells, and (b) the mononuclear phagocytes, including both monocytes and macrophages. Lymphocytes are primarily associated with specific immune responses, due to their ability to specifically recognize and distinguish antigenic determinants, while the mononuclear phagocytes are most often involved in the general removal of foreign microbes through phagocytosis as well as the production and secretion of cytokines induced by a microbe itself or in response to antigen-stimulated T lymphocytes. The functions of lymphocytic cells and the mononuclear phagocytes are highly interconnected and essential for proper immune system function.

One important subset of lymphocytic cells are T lymphocytes, which derive their designation from the fact that they are processed by the thymus. T lymphocytes are a complex group of cells which may be cytotoxic, having numerous mechanisms for inducing cell death, or activating, by secreting various cytokines that function to activate other cells. Cytotoxic T lymphocytes ("CTLs") act by being restricted to a particular major histocompatibility complex (MHC) antigen and express a cell surface T cell receptor which has specific affinity for a particular MHC complex associated with a peptide in the groove of the MHC. Where the MHC is foreign or the peptide in the groove is foreign to the host, CTLs will attack such cell and kill it. Importantly, however, CTLs have been screened during thymic development so that they do not normally act against cells where the peptide in the groove is endogenous to the host.

The combination of B lymphocytes and T lymphocytes establish the underlying operation of the humoral and cellular immune responses, respectively, which together form the basis for creating protective vaccines against pathogens and cancer cells. The humoral and cellular immune responses each proceed by activation of their respective cell types in response to stimulation from an antigen and the consequent secretions of various cytokines. The presentation of antigenic peptide to naïve CD4+ T helper cells causes the cells to differentiate into two distinct subsets of helper cells (Th-1 and Th-2) which can be distinguished by their function and cytokine expression profiles. Mosman et al., Annu. Rev. Immunol., 7:145-173 (1989); Paul et al., Cell, 76: 241-251 (1994); O'Garra, Immunity, 8:275-283 (1998).

The specific patterns of cytokines secreted by the CD4+ Th cells steer the immune response to a predominantly cellular, type-1 response (including IFN-γ, IL-1, IL-2, IL-12, and TNF-α) or a mainly humoral, type-2 response (including IL-4, IL-5, IL-6, IL-9, IL-10 and IL-13). Glimcher and Murphy, Genes Dev., 14:1693-1711 (2000); Abbas et al., Nature, 383:787-793 (1996). The Th-1 subset promotes both cell-mediated immunity through activation of CTL and NK cells, as well as humoral immunity characterized by immunoglobulin class switching from IgM to IgG and IgA in humans, and to IgG2a in mice. Th-1 responses may also be associated with delayed-type hypersensitivity and autoimmune disease. The Th-2 subset induces primarily humoral immunity and induces class switching to igG1 in mice and IgE in humans. The antibody isotypes associated with Th-1 responses generally have good neutralizing and opsonizing capabilities whereas those associated with Th-2 responses are generally more associated with allergic responses.

Vaccines have been formulated to provide protective immunity against pathogens by introducing antigens and adjuvants that stimulate the humoral and cellular immune responses and the production of memory B cells. Memory B cells are one form of differentiated B cells that proliferate as a component of the humoral immune response. Memory B cells remain in preactivated state so that they proliferate quickly in response to binding the same antigen (cognate antigen) without need of the initial burst of multiple cytokines required for the initial humoral response. Memory B cells quickly differentiate into antibody-secreting cells upon binding their cognate antigen. Memory T cells, on the other hand, are produced as a component of the cellular response. Memory T cells are analogous to memory B cells in that they are the progeny of an antigen-specific activation and maturation of helper T cells. Memory T cells secrete IFN γ upon exposure to the cognate antigen that enhances differentiation of the memory B cells into antibody secreting cells. A strong Th1 type response is central to a good vaccine.

The goal of vaccine formulation is provide a combination of antigens and adjuvants capable of generating a strong enough humoral and cellular immune response to induce a sufficient population of memory T cells and B cells to react quickly to a new challenge by a pathogen bearing the cognate antigen. There are four basic strategies for creating vaccines against pathogens.

The first strategy iuses whole attenuated and/or inactivated bacteria or viruses, which are particles that have been treated with some denaturing condition to render them ineffective or inefficient in mounting a pathogenic invasion. Some vaccines based on this strategy have been very effective, exemplified for example, by polio, measles yellow fever influenza, rabies and Japanese encephalitis vaccines. On the other hand, vaccines based on this strategy also have significant problems including inducing limited protection, being effective for only short periods of time, having toxic side effects, or ineffective attenuation or inactivation such that a reversion to a pathogenic infection may result.

A second strategy is to use purified antigens, which are typically naturally-produced antigens purified from a cell culture of the pathogen or a tissue sample containing the pathogen. Examples of vaccines based on this strategy include antigens derived from diphtheria, tetanus, pneumococcus and haempohilius influenza and cholera as well as subunits of hepatitis B and influenza viruses. One drawback to purified antigen systems is the need to derive the antigen from cultures of the pathogen, which comes with the risk that other viral pathogens may contaminate the purification. Another drawback is that in some cases these types of vaccines are inefficient in stimulating the development of memory cells.

A third strategy is to produce partial antigens from recombinantly engineered-organisms expressing antigenic epitopes from a recombinantly engineered gene. A typical antigenic epitope is a small polypeptide of about 8 to 20 amino acids in length. Exemplary vaccines based on this strategy include various components of hepatitis B virus, hepatitis C virus, herpes simplex virus and foot and mouth disease virus (animal vaccines). Typically, however, small recombinant epitope vaccines are not sufficiently immunogenic to stimulate the immune responses alone and often require cross-linking or genetic fusion to a more immunogenic protein sequence such as E. coli enterotoxin and the like. Moreover, even full-length recombinantly-expressed proteins may alone not be sufficiently immunogenic to provide a protective response against the pathogen of interest without additional stimulation.

A fourth strategy uses live viral delivery vectors to express and/or secrete pathogenic antigens in the host cells of the subject. These strategies rely on genetically engineering the viral vectors to be non-pathogenic and non-toxic. Exemplary viral delivery vectors used for this purpose include vaccina virus, adenovirus, adeno-associated virus, rinovirus, and integrating retroviruses including various versions of disarmed retoviruses with recombinant viral genomes obtained from viruses that infect humans such as HIV alone or combined with sequences from viruses that infect other species such as simian or feline viruses. Gene therapy strategies sometimes rely on targeting the viral vector to specific cell types such as antigen presenting cells, T cells and the like. All of these strategies also involve genetically engineering the chosen antigen for expression in the targeted cell and sometimes for secretion therefrom. Unfortunately however, live viral vaccine vectors pose a number of dangers including the possibility of activating silenced oncogenes, of mutation of the virus into a pathogenic form, and of generating overly-stimulated and uncontrolled immune responses that can cause severe or fatal consequences.

There is therefore, a need in the art to develop alternative vaccine formulations capable of eliciting an enhanced, Th-1 biased immune response to pathogens. There is a particular need for the development of such vaccines capable of eliciting improved humoral responses to antigens from a variety of different pathogens.

## SUMMARY OF THE INVENTION

In accordance with the foregoing needs and objectives, the present invention provides improved vaccines against pathogens and their use in stimulating immune responses to microbial antigens, and preferably humoral immune responses. The vaccines and uses described herein provide enhanced, Th-1 biased humoral immune responses to one or more epitopes of one or more target antigens, through the combined delivery of immunostimulatory nucleic acids using lipid-nucleic acid ("LNA") formulations along with the antigens of interest. Significantly, the present invention makes possible enhancement of the humoral component of the immune response to antigenic stimulation, including improved maturation of the humoral response and increased antibody isotype switching to IgG and IgA isotypes in humans.

In a first aspect, the present invention provides a pathogen vaccine comprising a lipid-nucleic acid (LNA) formulation in combination with at least one microbial antigen, wherein said at least one microbial antigen is mixed with or associated with said LNA formulation, said LNA formulation comprising: a) a lipid component comprising at least one cationic lipid; and b) a nucleic acid component comprising at least one oligonucleotide having at least one CpG dinucleotide with a methylated cytosine; wherein said vaccine is capable of stimulating a Th-1 biased immune response in *vivo* to said at least one microbial antigen. The epitope(s) of the microbial antigen(s) preferably are associated with the formulation, e.g. either attached to or encapsulated within the liposomal particle. The at least one microbial antigen may comprise a single epitope, a plurality of epitopes from the same antigen, or a plurality of epitopes from different antigens. In a second aspect, there is provided a polytope pathogen vaccine comprising a lipid-nucleic acid (LNA) formulation in combination with a plurality of microbial antigens, wherein said plurality of microbial antigens are associated with said LNA formulation, said formulation comprising: a) a lipid component comprising at least one cationic lipid; and b) a nucleic acid component comprising at least one oligonucleotide having at least one CpG dinucleotide with a methylated cytosine, wherein said vaccine is capable of simultaneously delivering said plurality of antigens to antigen presenting cells in

conjunction with adjuvant immune stimulation by said CpG dinucleotide to induce a Th-1 biased immune response.

In a third aspect, the invention provides the use of a vaccine of the first and second aspects in the manufacture of a medicament for stimulating an enhanced immune response to a microbial antigen. The use may be in a mammal.

Microbial antigens finding advantageous use in the present invention may generally be derived from infectious microbes such as virus, bacteria, parasites and fungi, and include intact microorganisms and natural isolates, fragments and derivatives thereof, as well as synthetic and recombinant molecules. In a preferred embodiment, the pathogen comprises hepatitis B virus. In a further preferred embodiment, the microbial antigen comprises the hepatitis B surface antigen (HBsAg), which may be recombinantly produced. Alternative specific embodiments are described herein.

In a fourth aspect, the invention provides the use of a lipid-nucleic acid (LNA) formulation associated with a target antigen, said LNA formulation comprising: a) a lipid component comprising at least one cationic lipid; and b) a nucleic acid component comprising at least one oligonucleotide having at least one CpG dinucleotide with a methylated cytosine; in the manufacture of a medicament for simultaneously delivering antigenic and adjuvant immune stimulation to antigen presenting cells to enhance Th-1 biased immune response.

In a specific embodiment, the nucleic acid component comprises the sequence 5' TAACGTTGAGGGGCAT 3' (ODN1m). In an alternative embodiment, the nucleic acid component comprises at least two CpG dinucleotides, wherein at least one cytosine in the CpG dinucleotides is methylated. In a further embodiment, each cytosine in the CpG dinucleotides present in the sequence is methylated. In another specific embodiment, the nucleic acid sequence comprises the sequence 5' TTCCATGACGTTCCTGACGTT 3' (ODN2m). In particularly preferred embodiments, the ODN is selected from a group of ODNs consisting of ODN #1, ODN #2, ODN #3, ODN #4, ODN #5, ODN #6, ODN #7, ODN #8, and ODN #9.

The enhanced Th-1 biased immune response obtained with the subject invention may be characterized by the induction of a first peak amount of IFN- y *in vivo* within 24 hours of administering the immunostimulatory composition, and by the induction of a larger, second peak amount of IFN-γ *in vivo* between about 2 days and about 7 days after administration of the immunostimulatory composition. Preferably, the use of an LNA formulation comprising a modified phosphate backbone provides a second peak that is at least twice the amount obtained upon administration of an LNA formulation containing a phosphodiester backbone.

The enhanced Th-1 biased immune response obtained with the subject invention may be characterized by at least a three-fold increase in Th-1 type antigen-specific antibody titers *in vivo* in comparison with conventional adjuvants or with free CpG ODN, preferably at least a four-or five-fold increase, still more preferably a six-to eight-fold increase, most preferably a ten-fold increase, and more generally at least a two-fold increase.

In a fifth aspect, the invention provides the use of an immunostimulatory composition comprising an encapsulated oligonucleotide having a modified phosphate backbone and at least one CpG dinucleotide with a methylated cytosine in the manufacture of a medicament for enhancing the humoral component of a host immune response to antigenic stimulation. Preferably, the modified phosphate backbone comprises a phosphorothioate backbone. In a further embodiment, the humoral component can be enhanced by associating the antigen with the LNA formulations The immunostimulatory composition may be associated with at least one target antigen. The target antigen may be associated with the LNA by at least one of chemical coupling, hydrophobic bonding or ionic bonding to a surface of the LNA.

The enhanced humoral response may be characterized by the induction of a first peak amount of IFN-γ *in vivo* within 24 hours of administering the immunostimulatory composition, and by the induction of a larger, second peak amount of IFN-γ *in vivo* between about 2 days and about 7 days after administration of the immunostimulatory composition. Preferably, the use of an LNA formulation comprising a phosphorothioate backbone provides a second peak that is at least twice the amount obtained upon administration of an LNA formulation containing a phosphodiester backbone.

In a sixth aspect, the invention provides the use of an immunostimulatory composition comprising an encapsulated oligonucleotide having a modified phosphate backbone and at least one CpG dinucleotide with a methylated cytosine in the manufacture of a medicament for improving the maturation of the humoral component of a host immune response to antigenic stimulation or for increasing antigen-specific antibody isotype switching in response to antigenic stimulation. Preferably, the use of an LNA formulation comprising a phosphorothioate backbone induces antigen-specific isotype switching from IgM to IgG and IgA isotypes in the host. In a seventh aspect, the invention provides the use of an immunostimulatory composition comprising an encapsulated oligonucleotide having a modified phosphate backbone and at least one CpG dinucleotide with a methylated cytosine in the manufacture of a medicament for inducing increased Th-1 type cytokine secretion in a host in response to antigenic stimulation.

In an eighth aspect, the invention provides an improved vaccine for stimulating a host immune response against a hepatitis B virus, comprising a lipid-nucleic acid (LNA) formulation in combination with at least one hepatitis B antigen, wherein said at least one hepatitis B antigen is mixed with or associated with said LNA formulation, said LNA formulation comprising: a) a lipid component comprising at least one cationic lipid; and b) a nucleic acid component comprising at least one oligonucleotide having at least one CpG dinucleotide with a methylated cytosine; wherein said vaccine is effective in inducing increased Th-1 type antibody titers *in vivo.*

In certain embodiments, the nucleic acid is comprised of a phosphodiester backbone. In alternative and preferred embodiments, the nucleic acid is comprised of a modified phosphate backbone. In a particularly preferred embodiment

discussed above, the modified phosphate backbone is a phosphorothioate backbone which, as demonstrated herein, is capable of improved humoral stimulation when encapsulated in the subject LNA formulations.

In one embodiment, the lipid component of the LNA formulation comprises a cationic lipid. In a further embodiment, the cationic lipid is selected from a group of cationic lipids consisting of DDAB, DODAC, DOTAP, DMRIE, DOSPA, DMDMA, DC-Chol, DODMA, and DODAP. In a further embodiment, the lipid component of the LNA formulation comprises a neutral lipid. In a further embodiment, the neutral lipid is selected from a group of neutral lipids consisting of DOPE, DSPC, POPC, diacylphosphatidylcholine, diacylphosphatidylethanolamine, ceramide, cholesterol sphingomyelin, cephalin, cholesterol and cerebrosides.

In other embodiments the lipid particle further includes a steric barrier lipid component on the surface of the lipid particle. In certain embodiments, the steric barrier lipid component is selected from the group consisting of PEG-DMG, PEG-PE, and a PEG ceramide. In one embodiment, the PEG ceramide is PEG-ceramide C-14. In another embodiment the PEG ceramide is PEG-ceramide C-20. In preferred embodiments, the lipid component of the LNA formulation comprises DSPC, DODMA, Chol, and PEG-DMG and the ratio of the DSPC to the DODMA to the Chol to the PEG-DMG is about 20:25:45:10 mol/mol.

The ratio of the lipid component to the nucleic component of the LNA formulations of the compositions and uses of the present invention may be about 0.01-0.25 wt/wt. The lipid component of the LNA formulations of the compositions and uses of the present invention may comprise a lipid membrane encapsulating said oligonucleotide.

In various embodiments, the lipid-nucleic acid formulation further comprises a pharmaceutically acceptable carrier, buffer or diluent.

A host suffering from a pathogen infection may be treated by the administration to said host of the subject LNA formulations in combination with at least one microbial antigen derived from said pathogen. A host may be prophylactically immunized against a target pathogen, utilizing the subject compositions and uses. Preferably, administration of the subject compositions is capable of stimulating one or more dendritic cells present in the animal's immune system. In one embodiment, the microbial antigen is administered in association with the lipid-nucleic acid formulations described herein, and more preferably with a liposomal particle. In a particularly preferred embodiment, the antigen is encapsulated in the liposomal particle. As described herein, in preferred embodiments the vaccine comprises a plurality of epitopes from one or more microbial antigens.

## BRIEF DESCRIPTION OF THE DRAWINGS

Figure 1 illustrates *in vitro* stimulation of leukocytes bearing the activation marker CD69 results from treating whole blood with free oligonucleotides. Mouse whole blood was treated *in vitro* with either the free oligonucleotide herein designated ODN 1 or with the oligonucleotide designated ODN2.

Figure 2 illustrates *in vivo* treatment of mice by injection with encapsulated or free ODN1 and ODN2 oligonucleotides produces results that are contrary to those obtained *in vitro.*

Figure 3 shows that when encapsulated in a lipid vesicle the methylated ODN1m was more active than the unmethylated counterpart ODN1 in stimulating activation of dendritic cells *in vivo.*

Figure 4A shows that both the methylated ODN1m and the unmethylated ODN1 stimulated the expansion of CD11c positive cells in spleen and whole blood.

Figure 4B shows that both ODN1 and ODN1m stimulate the expansion of DEC205 positive cells in spleen, whole blood and lymph node.

Figure 5 shows that the methylated ODN1 m was more active than the unmethylated counterpart ODN1. in stimulating CD86 expression when either ODN was lipid encapsulated.

Figure 6 shows that *in vivo* administration of free oligonucleotide had no affect on stimulation of IL-6, IL-12 IFN-gamma or MCP-1. In contrast, in vivo administration of lipid encapsulated oligonucleotides stimulated production of each of these cytokines.

Figure 7A illustrates increased IL-12 induction by treatment of mice with either encapsulated PO or PS oligonucleotide ODN1 in comparison to free oligonuchiotide ODN1 measured over an oligonucleotide dosage scale. Figure 7B shows that treatment with encapsulated PO oligonucleotides stimulates a strong early induction of IFN-gamma while treatment with encapsulated PS oligonucleotides stimulates a smaller but still effective induction of IFN-gamma.

Figure 8 shows a comparison of IgM titres indicative of a Th-1 response upon administration of free PS or PO oligonucleotides.

Figure 9 shows a comparison of IgG production indicative of a Th-2 response upon administration of free PS or PO oligonucleotide, including methylated oligonucleotides

Figure 10 shows that over a series of screenings of animals treated with methylated or unmethylated lipid encapsulated oligonucleotides, the methylated oligonucleotides are about the same or better than the unmethylated oligonucleotide in stimulating proliferation of dendritic cells, NK cells and CD8+ T-cells.

Figures 11 A and B show that over a series of screenings of animals treated with methylated or unmethylated

lipid encapsulated oligonucleotides, the methylated oligonucleotides are better than the unmethylated oligonucleotide in stimulating proliferation of cytotoxic T lymphocytes and Ag-specific lymphocytes.

Figure 11C illustrates data from a representative tetramer study that was included in the overall screenings described in Figures 11 A and 11B.

Figure 12 illustrates that when administered to an animal as free oligonucleotides, methylated versions have less therapeutic efficacy than methylated nucleotides In reducing tumor growth.

Figure 13 illustrates that encapsulation of oligonucleotides provides improved efficacy of methylated and unmethylated oligonucleotides over free ODN, particularly when the oligonucleotides contain a natural phosphorothioate (PS) backbone.

Figure 14 shows that encapsulation of oligonucleotides provides improved efficacy of methylated and unmethylated oligonucleotides over free ODN, when the oligonucleotides contain a phosphodiester (PO) backboneFigure 15 shows that lipid encapsulated PS oligonucleotides ODN2and ODN2m each exhibit therapeutic efficacy.

Figure 16 illustrates an adjuvant effect and therapeutic efficacy of administering the methylated ODN1m to an animal inoculated with a B16 melanoma tumor. Encapsulation of the ODN1m oligonucleotide in a lipid particle increased its efficacy in reducing tumor volume.

Figure 17 shows that for a series of mice inoculated with the B16 melanoma and subsequently treated by administration of a 20mg/kg dose of oligonucleotide, the average tumor size of tumors in mice treated with encapsulated free oligonucleotides ODN1 and ODN1m.

Figure 18 shows the reduction in tumor volume when mice were treated with encapsulated methylated ODN1m and the unmethylated counterpart ODN1.

Figure 19 shows survival rates of mice treated with the encapsulated methylated ODN1m in comparison to treatment with the unmethylated ODN1 in two different studies.

Figure 20 illustrates the efficacy in terms of tumor volume when methylated ODN1m and the unmethylated counterpart ODN1are encapsulated in a lipid particle.

Figure 21 shows the survival rate of mice treated with encapsulated methylated ODN1m relative to treatment with the unmethylated encapsulated ODN1.

Figure 22 illustrates that encapsulated PS oligonucleotides ODN1 and ODN2produced an IFN-gamma peak that is not produced by encapsulated PO oligonucleotides 6 days after treatment.

Figure 23 shows the effect on blood clearance in mice methylated or unmethylated oligonucleotides encapsulated in lipid particles having different PEG-ceramide steric coatings.

Figure 24 illustrates therapeutic efficacy of liposomal particles encapsulating unmethylated or methylated CpG oligonucleotide in treating a tumor by administering the composition to an animal having the tumor.

Figure 25 illustrates that lipid encapsulation of methylated PS-ODN5m provided a more effective therapeutic benefit than encapsulation of the equivalent unmethylated PS-ODN5 at reducing tumor growth over time.

Figure 26 shows the survival rate of the mice treated with free and encapsulated methylated ODN5m relative to treatment with the unmethylated encapsulated and free ODN5.

Figure 27 illustrates efficacy in terms of tumor volume when treated with free unmethylated and methylated PS and PO ODN7 and encapsulated PO-ODN7m.

Figure 28 shows survival rates of mice treated with the free unmethylated and methylated PS and PO ODN7 in comparison to treatment with the encapsulated PO-ODN7m.

Figure 29 demonstrates increase in titers of HBsAg specific IgG upon co-administration of Alum-based vaccine with LNA/ODN 1 . IgG levels in plasma were obtained after 6 weeks upon IM prime-boost immunization and were measured by end-point dilutions ELISA.

Figure 30 confirms the result in Figure 29. Mice were immunized with 2$\mu$g/dose of recombinant HBsAg with Alum or LNA/ODN 1. A 10-fold increase in specific IgG titer was observed for LNA/ODN 1 compared to Alum (Figure 34A).

Figure 31 illustrates the effect of co-administration of the Recombivax-HB vaccine with LNA /ODN 1 in a single immunization setting. Plasma obtained 6 weeks following an IM single dose immunization is compared to plasma obtained from mice immunized with either Engerix B or RecombiVax-HB in a prime-boost regimen.

Figure 32 illustrates anti-HBsAg plasma antibody titers following an IM immunization with RecombiVax-HB or Engerix-B (0.5 $\mu$g of HBsAg, 25 $\mu$g Alum per dose) alone or mixed with LNA /ODN 1 (100 $\mu$g ODN) as an adjuvant on a q14d x 2 prime-boost dosing schedule.

Figure 33 illustrates anti-HBsAg plasma antibody titers following an IM immunization with recombinant HBsAg (2 of HBsAg, 25) alone or mixed with Alum (25$\mu$g per dose), encapsulated (LNA /ODN 1) ISS ODN (100 $\mu$g) or a combination of Alum and encapsulated ODN (LNA /ODN 1) in a q14d x 2 prime-boost dosing schedule.

Figure 34 illustrates the adjuvant effect of LNA co-administered with BSA. Either BSA alone or LNA + BSA was administered as a single dose intravenously to mice. The titer of antibodies in the serum was measured after 5 weeks. A strong IgG1 response is observed in either case. However, CpG-containing particles direct the immune system to produce higher titers of the IgG2a isotype, as seen in the bars on the right hand side of the graph.

Figure 35 illustrates the immunostimulatory effect of LNA on IgA production. OVA + LNA/ODN1 PS,, OVA + ODN 1 PS, or CT mixed with OVA were administered intranasally to mice once a week for three weeks. IgA titers were measured in serum and lung. In both cases, IgA levels were higher when OVA +LNA/ODN1 PS was used.

Figure 36 illustrates the immunostimulatory effect of the LNA. Alum, CFA/IFA, TitreMax™, ImmuneEasy™ (CpG1826+Alum), LNA (CpG1826), and OVA-conjugated LNA (CpG1826) were administered intravenously to mice, and IgG levels were measured after two weeks. LNA-OVA particles (solid squares) induced the highest levels of OVA-specific IgG two weeks after a single injection.

Figure 37 depicts the titer of anti-OVA IgG (Fig. 37A), anti-OVA IgA (Fig. 37B), and anti-OVA IgM (Fig. 37C) in serum on day 28 following the initial immunization of C57BL/6 mice (6 weeks old) with 20 μl of test formulations by intranasal administration.

Figure 38 depicts the titer of anti-OVA IgG (Fig. 38A), anti-OVA IgA (Fig. 38B), and anti-OVA IgM (Fig. 38C) in lung washes on day 28 following the initial immunization of C57BU6 mice (6 weeks old) with 20 μl of test formulations by intranasal administration.

Figure 39 depicts the titer of anti-OVA IgG (Fig. 39A) and anti-OVA IgA (Fig. 39B) in vaginal washes on day 28 following the initial immunization of C57BU6 mice (6 weeks old) with 20 μl of test formulations by intranasal administration.

Figure 40 depicts humoral immunity as indicated by the titer of anti-OVA IgG in serum (Fig. 40A), lung wash (Fig. 40C), and vaginal wash (Fig. 40A) on day 28 following the initial immunization of C57BL/6 mice (6 weeks old) with 20 μl of the test formulations test formulations by intranasal administration.

Figure 41 depicts humoral immunity as indicated by the titer of anti-OVA IgA in serum (Fig. 41 A), lung wash (Fig. 41 B), and vaginal wash (Fig. 40C) on day 28 following the initial immunization of C57BU6 mice (6 weeks old) with 20 μl of test formulations by intranasal administration.

Figure 42 depicts the titer of anti-OVA IgA in lung washes was on day 28 following the initial immunization of C57BU6 mice (6 weeks old) with 20 μl of test formulations by intranasal administration.

Figure 43 depicts the titer of anti-OVA IgA in vaginal washes was on day 28 following the initial immunization of C57BU6 mice (6 weeks old) with 20 μl of test formulations by intranasal administration.

Figure 44 depicts the titer of anti-OVA IgA in lung washes (Fig. 44A) and vaginal washes (Fig. 44B) on day 28 following the initial immunization of C57BU6 mice (6 weeks old) with 20 μl of test formulations by intranasal administration.

Figure 45 depicts the titer of anti-OVA IgG in lung washes (Fig. 45A) and vaginal washes (Fig. 45B) on day 28 following the initial immunization of C57BU6 mice (6 weeks old) with 20 μl of test formulations by intranasal administration.

Figure 46 depicts the titer of anti-OVA IgG in plasma on day following the initial immunization of C57BU6 mice (6 weeks old) with 20 μl of test formulations by intranasal administration.

Figure 47 shows the CTL response to a B16 cell target after immunization with a multiple epitope cancer vaccine using encapsulated ODN 1 m.

Figure 48 shows the CTL response to a B16 cell target after immunization with a multiple epitope cancer vaccine using peptide-pulsed dendritic cells.

Figure 49 shows the CTL response to a B16 cell target after immunization with tumor cell lysate in combination with encapsulated ODN 1 m or dendritic cells.

## DETAILED DESCRIPTION OF THE INVENTION

The humoral component of the immune response, characterized by the activation and proliferation of B cells that express and secrete immunoglobulins, is the principle protective response against bacteria and parasites and is an important component of a protective response against viruses. The cellular component of the immune response, characterized by the activation and proliferation of CTL and NK cells, is the principle protective response for combating internal pathologies such as cancer and chronic viral diseases. While both components of the immune response are important in helping the host respond to infectious pathogens, the humoral response is particularly significant given the role of antibodies and memory B cells in eliminating the invading pathogens and guarding against their return.

Pathogen vaccines and protocols capable of provoking strong, Th-1-biased humoral immune responses to microbial antigens are required for effective prophylactic protection against future infections and successful treatment of ongoing infections. The ability to enhance the humoral component of the immune response to pathogenic stimulation and improve maturation of the resulting antibody response are critical. As demonstrated herein, the vaccine compositions of the present invention stimulate strong, Th-1 biased immune responses to microbial antigens in vivo, and can significantly increase isotype switching and thereby improve maturation of the humoral response. Unlike the pathogen vaccines described in the prior art, the unique vaccine formulations provided herein enable simultaneous presentation of multiple antigenic determinants directly to professional APCs in vivo in conjunction with Th-1 biased immune stimulation.

The invention provides lipid-nucleic acid (LNA) formulations mixed or associated with at least one epitope of at least one microbial antigen of interest. Preferably, the LNA formulations are mixed or associated with a plurality of

epitopes from one or more microbial antigens to form polytope vaccines. Still more preferably, a plurality of such epitopes are associated with the LNA formulations. The nucleic acid comprises at least one CpG dinucleotide and preferably comprises an immunostimulatory sequence (ISS).

Abbreviations and Definitions

The following abbreviations are used herein: RBC, red blood cells; DDAB, N,N-distearyl-N,N-dimethylammonium bromide; DODAC, N,N-dioleyl-N,N-dimethylammonium chloride; DOPE, 1,2-sn-dioleoylphoshatidylethanolamine; DOSPA, 2,3-dioleyloxy-N-(2(sperminecarboxamido)ethyl)-N,N-dimethyl-1-propanaminiu m trifluoroacetate; DOTAP, 1,2-dioleoyloxy-3-(N,N,N-trimethylamino)propane chloride; DOTMA, 1,2-dioleyloxy-3-(N,N,N-trimethylamino)propanechloride; OSDAC, N-oleyl-N-stearyl-N,N-dimethytammonium chloride; RT, room temperature; HEPES, 4-(2-hydroxyethyl)-1-piperazineethanesulfonic acid; FBS, fetal bovine serum; DMEM, Dulbecco's modified Eagle's medium; PEG-Cer-C.sub.14, 1-O-(2'-(.omega.-methoxypolyethyleneglycol)succinoyl)-2-N-myristoyl-sphing osine; PEG-Cer-C.sub.20, 1-O-(2'-(.omega.-methoxypolyethyleneglycol)succinoyl)-2-N-arachidoyl-sphin gosine; PBS, phosphate-buffered saline; THF, tetrahydrofuran; EGTA, ethylenebis(oxyethylenenitrilo)-tetraacetic acid; SF-DMEM, serum-free DMEM; NP40, nonylphenoxypolyethoxyethanol, 1,2 dioleoyl-3 dimethylaminopropane (DODAP), palmitoyl oleoyl phsphatidylcholine (POPC) and dislearoylphosphatidylcholine (DSPC).

The technical and scientific terms used herein have the meanings commonty understood by one of ordinary skill in the art to which the present invention pertains, unless otherwise defined. Reference is made herein to various methodologies known to those of skill in the art. Standard reference works setting forth the general principles of recombinant DNA technology include Sambrook, J., et al., Molecular Cloning,: A Laboratory Manual, 2d Ed., Cold Spring Harbor Laboratory Press, Planview, N.Y. (1989); McPherson, M. J., Ed., Directed Mutagenesis: A Practical Approach, IRL Press, Oxford (1991); Jones, J., Amino Acid and Peptide Synthesis, Oxford Science Publications, Oxford (1992); Austen, B. M. and Westwood, O. M. R., Protein Targeting and Secretion, IRL Press, Oxford (1991). Any suitable materials and/or methods known to those of skill can be utilized in carrying out the present invention; however, preferred materials and/or methods are described. Materials, reagents and the like to which reference is made in the following description and examples are obtainable from commercial sources, unless otherwise noted. It is believed that one skilled in the art can, based on the description herein, utilize the present invention to its fullest extent.

The immunostimulatory compositions used in the present invention may generally be referred to as lipid-therapeutic agent ("LTA") formulations comprising at least one lipid component and at least one therapeutic agent, and having greater immunostimulatory activity than the therapeutic agent alone, *in vivo.* "Therapeutic agent" or "therapeutic compound" or "drug" as used herein can be used interchangeably and refer to any synthetic, recombinant, or naturally occurring molecule that provides a beneficial effect in medical treatment of a subject. Examples of therapeutic agents Include, but are not limited to nucleic acids, peptides, and chemicals.

In the present invention, the therapeutic agent comprises at least one oligonucleotide, preferably at least one oligodeoxynucleotide ("ODN"), comprising at least one CpG dinucleotide motif with a methylated cytosine. In a particularly preferred embodiment, the ODN comprises a methylated nucleic acid sequence that has immunostimulatory activity and is designated an immunostimulatory sequence ("ISS") in non-methylated form.

"Subject" or "host" as used herein refers to an organism, male or female, having an immune system, preferably an animal, more preferably a vertebrate, even more preferably a mammal, still even more preferably a rodent, and most preferably a human. Further examples of a subject include, but are not limited to, dogs, cats, cows, horses, pigs, sheep, goats, mice, rabbits, and rats. "Patient" as used herein refers to a subject in need of treatment for a medical condition (e.g., disease or disorder).

*"In vivo"* as used herein refers to an organism, preferably in a mammal, more preferably in a rodent, and most preferably in a human.

"Immunostimulatory," "immunostimulatory activity" or "stimulating an immune response," and grammatical equivalents thereof, as used herein refers to inducing, increasing, enhancing, or modulating an immune response, or otherwise providing a beneficial effect with respect to an immune response. As used herein "immune response" refers to both cellular and humoral immune responses, with humoral immune responses most preferred. The immunostimulatory activity of a given formulation and nucleic acid sequence may be readily determined using a suitable *in vivo* assay as described herein.

"A target antigen" as used herein refers to an antigen of interest to which a immune response can be directed or stimulated. The target antigen used in the compositions of the present invention for stimulating an immune response directed to that target antigen may be a synthetic, naturally-occurring or isolated molecule or a fragment thereof, and may comprise single or multiple epitopes. Thus, the compositions of the present invention may stimulate immune responses directed to single or multiple epitopes of an antigen. In preferred embodiments, the target antigen is associated with the lipid particles of the present invention. "In association with", "associated with", or grammatical equivalents thereof, as used herein with reference to an antigen (or target antigens), refers to antigens that are attached to or

encapsulated by another component. With reference to the lipid particles or liposomes of the present invention, the antigen may be, for example, encapsulated in the lumen or intralamellar spaces of the lipid particles; disposed or attached within or partially within the lipid membrane, or attached (*e.g.,* covalently or ionically) to the lipid particle. The antigen may be attached to the interior of the lipid particle or, more preferably, the antigen is attached to the exterior of the lipid particle. In preferred embodiments the antigen is encapsulated within the lipid particle.

Examples of antigens useful in the compositions and methods of the present invention include, but are not limited to, peptides or proteins, cells, cell extracts, polysaccharides, polysaccharide conjugates, lipids, glycolipids, glycopeptides, and carbohydrates. In one embodiment, the antigen is in the form of a peptide or protein antigen. In another embodiment, the antigen is a nucleic acid encoding a peptide or protein in a form suitable for expression in a subject and presentation to the immune system of that subject. In a preferred embodiment, the compositions used in the present invention comprise a peptide or protein target antigen that stimulates an immune response to that target antigen in a mammal. Preferably, the target antigen is a microbial antiogen ("target pathogen") capable of infecting a mammal including, for example, bacteria, viruses, fungi, yeast, parasites and other microorganisms capable of infecting mammalian species.

A "microbial antigen" as used herein is an antigen derived from a microorganism and includes but is not limited to, infectious virus, infectious bacteria, infectious parasites and infectious fungi. Microbial antigens may be intact microorganisms, and natural isolates, fragments, or derivatives thereof, as well as recombinant and synthetic compounds which are identical to or similar to naturally-occurring microbial antigens and which, preferably, induce an immune response specific for the corresponding microorganism (from which the naturally-occurring microbial antigen originated). In a preferred embodiment, a compound is-similar to a naturally-occurring microorganism antigen if it induces an immune response (humoral and/or cellular) to a naturally-occurring microorganism antigen. Compounds or antigens that are similar to a naturally-occurring microorganism antigen are well known to those of ordinary skill in the art. A non-limiting example of a compound that is similar to a naturally-occurring microorganism antigen is a peptide mimic of a polysaccharide antigen. More specific embodiments are provided herein.

The term "antigen" is further intended to encompass peptide or protein analogs of known or wild-type antigens such as those described above. The analogs may be more soluble or more stable than wild type antigen, and may also contain mutations or modifications rendering the antigen more immunologically active. Also useful in the compositions and methods of the present invention are peptides or proteins which have amino acid sequences homologous with a desired antigen's amino acid sequence, where the homologous antigen induces an immune response to the respective pathogen.

"Homologous" as used herein refers to the subunit sequence similarity between two polymeric molecules, e.g., between two nucleic acid molecules (e.g., two DNA molecules or two RNA molecules) or two polypeptide molecules. When a subunit position in both molecules is occupied by the same monomeric subunit, e.g., if a position in each of two DNA molecules is occupied by adenine, then they are homologous at that position. The homology between two sequences is a direct function of the number of matching or homologous positions, e.g., if half (e.g. five positions in a polymer ten subunits in length) of the positions in two compound sequences are homologous then the two sequences are 50% homologous, if 90% of the positions, e.g., 9 of 10, are matched or homologous, the two sequences share 90% homology. By way of example, the DNA sequences 5'-CCGTTA-3' and 5'-GCGTAT-3' share 50% homology. By the term "substantially homologous" as used herein, is meant DNA or RNA which is about 50% homologous, more preferably about 70% homologous, even more preferably about 80% homologous and most preferably about 90% homologous to the desired nucleic acid. Genes which are homologous to the desired antigen-encoding sequence should be construed to be included in the invention provided they encode a protein or polypeptide having a biological activity substantially similar to that of the desired antigen. Where in this text, protein and/or DNA sequences are defined by their percent homologies or identities to identified sequences, the algorithms used to calculate the percent homologies or percent identities include the following: the Smith-Waterman algorithm (J. F. Collins et al, Comput. Appl. Biosci., (1988) 4:67-72; J. F. Collins et al, Molecular Sequence Comparison and Alignment, (M. J. Bishop et al, eds.) In Practical Approach Series: Nucleic Acid and Protein Sequence Analysis XVIII, IRL Press: Oxford, England, UK (1987) 417), and the BLAST and FASTA programs (E. G. Shpaer et al, 1996, Genomics, 38:179-191).

Analogs of the antigens described herein can differ from naturally occurring proteins or peptides by conservative amino acid sequence differences or by modifications which do not affect sequence, or by both. For example, conservative amino acid changes may be made, which although they alter the primary sequence of the protein or peptide, do not normally alter its function. Modifications (which do not normally alter primary sequence) include *in vivo,* or *in vitro* chemical derivatization of polypeptides, e.g., acetoation, or carboxylation. Also contemplated as antigens are proteins modified by glycosylation, e.g., those made by modifying the glycosylation patterns of a polypeptide during its synthesis and processing or in further processing steps; e.g., by exposing the polypeptide to enzymes which affect glycosylation, e.g., mammalian glycosylating or deglycosylating enzymes. Also contemplated as antigens are amino acid sequences which have phosphorylated amino acid residues, e.g., phosphotyrosine, phosphoserine, or phosphothreonine. Also contemplated as antigens are polypeptides which have been modified using ordinary molecular biological techniques so as to

improve their resistance to proteolytic degradation or to optimize solubility properties. Analogs of such polypeptides include those containing residues other than naturally occurring L-amino acids, e.g., D-amino acids or non-naturally occurring synthetic amino acids.

The antigens useful in the present invention are not limited to products of any of the specific exemplary processes listed herein. In addition to substantially full length polypeptides, the antigens useful in the present invention include immunologically active fragments of the polypeptides. For example, the antigen may be a fragment of a complete antigen including at least one epitope. "Epitope" as used herein refers to any antigenic determinant on an antigen to which the paratope of an antibody can bind. Epitopic determinants usually consist of chemically active surface groupings of molecules such as, e.g., amino acids or sugar side chains and usually have specific three-dimensional structural characteristics. Particularly preferred embodiments of the compositions and uses of the present invention include combination antigens which include multiple epitopes from the same target antigen, or epitopes from two or more different target antigens (i.e., polytope vaccines). For example, the combination antigens can be the same or different type such as, e.g., a peptide-peptide antigen, glycolipid-peptide antigen, or glycolipid-glycolipid antigen.

A polypeptide or antigen is "immunologically active" if it induces an immune response to a target antigen or pathogen. "Vaccine" as used herein refers to a composition comprising a target antigen that stimulates a specific immune response to that target antigen. "Pathogen vaccine" as used herein refers to a composition comprising at least one epitope of at least one microbial antigen that stimulates a specific immune response to the antigen(s).

"Adjuvant" as used herein refers to any substance which can stimulate or enhance the stimulation of an immune responses. Some adjuvants can cause activation of a cell of the immune system, for example, an adjuvant can cause an immune cell to produce and secrete cytokines. Examples of adjuvants that can cause activation of a cell of the immune system include, but are not limited to, saponins purified from the bark of the Q. saponaria tree, such as QS21 (a glycolipid that elutes in the 21 st peak with HPLC fractionation; Aquila Biopharmaceuticals, Inc., Worcester, Mass.); poly(di(carboxylatophenoxy)phosphazene (PCPP polymer; Virus Research Institute, USA); derivatives of lipopolysaccharides such as monophosphoryl lipid A (MPL; Ribi ImmunoChem Research, Inc., Hamilton, Mont.), muramyl dipeptide (MDP; Ribi) and threonyl-muramyl dipeptide (t-MDP; Ribi); OM-174 (a glucosamine disaccharide related to lipid A; OM Pharma SA, Meyrin, Switzerland); and Leishmania elongation factor (a purified Leishmania protein; Corixa Corporation, Seattle, Wash.). Traditional adjuvants are well known in the art and include, for example, aluminum phosphate or hydroxide salts ("alum").

As compared to known adjuvants, the present invention provides improved adjuvants comprising combinations of lipids and nucleic acids that act synergistically to stimulate enhanced, Th-1 biased immune responses. Such compositions of the present invention comprise a nucleic acid component and a lipid component. The nucleic acid component comprises at least one oligonucleotide, more preferably at least one ODN, comprising at least one CpG motif, where the cytosine is methylated.

In preferred embodiments the immunostimulatory compositions used in the present invention comprise a lipid component comprising a lipid membrane that encapsulates a therapeutic agent. As used herein "liposomal particle," "liposome," "lipid vesicte," and "liposomal vesicle," or grammatical equivalents thereof, may be used interchangeably and refer to structures, particles, complexes, or formulations comprising lipid-containing membranes which enclose or encapsulate an aqueous interior. In preferred embodiments, the liposomes enclose or encapsulate therapeutic agents, i.e., nucleic acids. The liposomes may have one or more lipid membranes. Liposomes having one lipid-containing membrane are referred to herein as "unilamellar." Liposomes having multiple lipid-containing membranes are referred to herein as "multilamellar." "Lipid bilayer" as used herein refers to a lipid-containing membrane having two layers. In preferred embodiments, the liposomes are multilamellar.

### Nucleic Acids

Nucleic acids suitable for use in the compositions of the present invention include, for example, DNA or RNA. The nucleic acids are oligonucleotides, more preferably ODNs, and most preferably an ODN comprising an ISS ("ISS ODN") and at least one CpG dinucleotide.

"Nucleic acids" as used herein refer to multiple nucleotides (i.e., molecules comprising a sugar (e.g. ribose or deoxyribose) linked to a phosphate group and to an exchangeable organic base, which is either a substituted pyrimidine (e.g. cytosine (C), thymine (T) or uracil (U)) or a substituted purine (e.g. adenine (A) or guanine (G)). Nucleic acids may be, for example DNA or RNA. Preferably the nucleic acids are ofigoribonucleotides and more preferably ODNs. Nucleic acids may also be polynucleosides, i.e.. a polynucleotide minus the phosphate and any other organic base containing polymer. The immunostimulatory compositions of the present invention comprise a nucleic acid component. "Nucleic acid component" as used herein with reference to compositions of the present invention refers to a component comprising nucleic acids.

In a preferred embodiment, the oligonucleotides are single stranded and in the range of 5 - 50 nucleotides ("nt") in length. However, any oligonucleotides may be used including, for example, large double stranded plasmid DNA

in the range of 500- 50,000 base pairs ("bp").

Nucleic acids useful in the compositions and uses of the present invention can be obtained from known sources or isolated using methods well known in the art. The nucleic acids can also be prepared by recombinant or synthetic methods which are equally well known in the art. Such nucleic acids can then be encapsulated in lipid particles and the resulting compositions tested for immunostimulatory activity using the methods of the present invention as described herein.

For use *in vivo,* nucleic acids may be resistant to degradation (e.g., via endo-and exonucleases). Secondary structures, such as stem loops, can stabilize nucleic acids against degradation. Alternatively, nucleic acid stabilization can be accomplished via phosphate backbone modifications. A preferred stabilized nucleic acid has at least a partial phosphorothioate ("PS") modified backbone. Phosphorothioates may be synthesized using automated techniques employing either phosphoramidate or H-phosphonate chemistries. Aryl-and alkyl-phosphonates can be made, e.g., as described in U.S. Patent No. 4,469,863; and alkylphosphotriesters (in which the charged oxygen moiety is alkylated as described in U.S. Patent No. 5,023,243 and European Patent No. 092,574) can be prepared by automated solid phase synthesis using commercially available reagents. Methods for making other DNA backbone modifications and substitutions have been described (Uhlmann, E. and Peyman, A., Chem. Rev. 90:544, 1990; Goodchild, J., Bioconjugate Chem. 1:165, 1990). As described herein, however, the uses and compositions of the present invention alleviate the need to include such modifications to the subject nucleic acids.

Thus, oligonucleotides useful in the compositions and uses of the present invention may have a modified phosphate backbone such as, *e.g.,* phosphorothioate, methylphosphonate, methylphosphorothioate, phosphorodithioate, and combinations thereof with each other and/or with phosphodiester ("PO") oligonucleotide. In addition, other modified oligonucleotides include: nonionic DNA analogs, such as alkyl- and aryl-phosphates (in which the charged phosphonate oxygen is replaced by an alkyl or aryl group), phosphodiester and alkylphosphotriesters, In which the charged oxygen moiety is alkylated. As demonstrated herein, PO ODN may be preferred where cellular immune responses are desired, while modified ODN such as, *e.g.,* PS ODN may be preferred where humoral responses are desired.

Numerous other chemical modifications to the base, sugar or linkage moieties are also useful. Bases may be methylated or unmethylated. In the preferred embodiments, methyl or hydroxymethyl groups are attached to the carbon-4 position (4-mC) or carbon-5 position (5-mC) of at least one cytosine. The methylated cytosine is preferably located within a CpG motif in the nucleic acid sequence. Alternatively or additionally, the sugar moiety may be modified with a methyl group as described in the art.

Nucleic acid sequences useful in the compositions and uses of the present invention may be complementary to patient/subject mRNA, such as antisense oligonucleotides, or they may be foreign or non-complementary (*e.g.,* the nucleotide sequences do not specifically hybridize to the patient/subject genome). The nucleotide sequences may be expressed and the resulting expression products may be RNA and/or protein. In addition, such nucleotide sequences may be linked to appropriate promoters and expression elements, and may be contained in an expression vector. Nucleotide sequences useful in the composition and uses of the present invention may be ISS, such as certain palindromes leading to hairpin secondary structures (see Yamamoto S., et al. (1992) J. Immunol. 148: 4072-4076), or CpG motifs, or other known ISS features (such as multi-G domains, see WO 96/11266). The nucleotide sequence comprises at least one CpG motif having a methylated cytosine.

The nucleic acids useful in the present invention can be synthesized de novo using any of a number of procedures well known in the art. For example, the b-cyanoethyl phosphoramidite method (Beaucage, S. L., and Caruthers. M. H., Tet. Let 22:1859, 1981); nucleoside H-phosphonate method (Garegg et al., Tet. Let. 27:4051-4054, 1986; Froehler et al., Nucl. Acid. Res. 14:5399-5407, 1986, ; Garegg et al., Tet. Let. 27:4055-4058, 1986, Gaffney et al., Tet. Let. 29: 2619-2622,1988). These chemistries can be performed by a variety of automated oligonucleotide synthesizers available in the market. Also, CpG dinucleotides can be produced on a large scale in plasmids, (see Sambrook. T., et al., Molecular Cloning: A Laboratory Manual, Cold Spring Harbor laboratory Press, New York, 1989). Such plasmids may also encode other genes to be expressed such as an antigen-encoding gene in the case of a DNA vaccine. Oligonucleotides can be prepared from existing nucleic acid sequences (e.g., genomic or cDNA) using known techniques, such as those employing restriction enzymes, exonucleases or endonucleases.

For administration *in vivo,* compositions of the present invention, including components of the compositions, e.g., a lipid component or a nucleic acid component, may be associated with a molecule that results in higher affinity binding to target cell (e.g., B-cell, monocytic cell and natural killer (NK) cell) surfaces and/or increased cellular uptake by target cells. The compositions of the present invention, including components of the compositions, can be ionically or covalently associated with desired molecules using techniques which are well known in the art. A variety of coupling or cross-linking agents can be used, e.g., protein A, carbodiimide, and N-succinimidyl-3-(2-pyridyidithio) propionate (SPDP).

The immune stimulating activity of a nucleic acid sequence in an organism can be determined by simple experimentation, for example, by comparing the sequence in question with other immunostimulatory agents, e.g., other adjuvants, or ISS; or by detecting or measuring the immunostimulatory activity of the sequence in question, e.g., by

detecting or measuring the activation of host defense mechanisms or the activation of immune system components. Such assays are well known in the art. Also, one of skill in the art would know how to identify the optimal oligonucleotides useful for a particular mammalian species of interest using routine assays described herein and/or known in the art.

Specific nucleic acid sequences of ODNs suitable for use in the compositions and methods of the invention are described in U. S. Patent Appln. 60/379,343, U. S. Patent Appln. No. 09/649,527, Int. Publ. WO 02/069369, Int. Publ. No. WO 01/15726, U.S. Patent No. 6,406,705, and Raney et al., Journal of Pharmacology and Experimental Therapeutics, 298: 1185-1192 (2001). Exemplary sequences of the ODNs include, but are not limited to, those nucleic acid sequences shown in Table 1. ODNs used in the compositions and uses of the present invention have a phosphodiester ("PO") backbone or a phosphorothioate ("PS") backbone. As described herein, PS ODN are most preferred for their ability to enhance the humoral component of the immune response. The ODNs comprise at least one methylated cytosine residue in a CpG motif.

**Table 1**

| ODN NAME | ODN SEQ ID NO | ODN SEQUENCE (5'-3') |
|---|---|---|
| ODN 1 (INX-6295) human c-myc | SEQ ID NO: 2 | 5'-TAACGTTGAGGGGCAT-3 |
| * ODN 1m (INX-6303) | SEO ID NO: 4 | 5'-TAAZGTTGAGGGGCAT-3 |
| ODN 2 (INX-1826) | SEQ ID NO: 1 | 5'-TCCATGACGTTCCTGACGTT-3 |
| * ODN 2m (INX-1826m) | SEO ID NO: 31 | 5'-TCCATGAZGTTCCTGAZGTT-3 |
| ODN 3 (INX-6300) | SEQ ID NO: 3 | 5'-TAAGCATACGGGGTGT-3 |
| ODN 5 (INX-5001) | SEQ ID NO: 5 | 5'-AACGTT-3 |
| ODN 6 (INX-3002) | SEQ ID NO: 6 | 5'-GATGCTGTGTCGGGGTCTCCGGGC-3' |
| ODN 7 (INX-2006) | SEQ ID NO: 7 | 5'-TCGTCGTTTTGTCGTrTTGTCGTT-3' |
| ODN 7m (INX-2006m) | SEQ ID NO: 7 | 5'-TZGTZGTTTTGTZGTTTTGTZGTT-3' |
| ODN 8 (INX-1982) | SEO ID NO: 8 | 5'-TCCAGGACTTCTCTCAGGTT-3' |
| ODN 9 (INX-G3139) | SEQ ID NO: 9 | 5'-TCTCCCAGCGTGCGCCAT-3' |
| ODN 10 (PS-3082)<br>murine Intracellular Adhesion Molecule-1 | SEO ID NO: 10 | 5'-TGCATCCCCCAGGCCACCAT-3 |
| ODN 11 (PS-2302)<br>human Intracellular Adhesion Molecule-1 | SEO ID NO: 11 | 5'-GCCCAAGCTGGCATCCGTCA-3' |
| ODN 12 (PS-8997)<br>human Intracellular Adhesion Molecule-1 | SEO ID NO: 12 | 5'-GCCCAAGCTGGCATCCGTCA-3' |
| ODN 13 (US3)<br>human erb-B-2 | SEQ ID NO: 13 | 5'-GGT GCTCACTGC GGC-3' |
| ODN 14 (LR-3280)<br>human c-myc | SEQ ID NO: 14 | 5'-AACC GTT GAG GGG CAT-3' |
| ODN 15 (LR-3001)<br>human c-myc | SEO ID NO: 15 | 5'-TAT GCT GTG CCG GGG TCT TCG GGC-3' |
| ODN 16 (Inx-6298) | SEO ID NO: 16 | 5'-GTGCCG GGGTCTTCGGGC-3' |
| ODN 17 (hIGF-1 R)<br>human Insulin Growth Factor 1 - Receptor | SEQ ID NO: 17 | 5'-GGACCCTCCTCCGGAGCC-3' |
| ODN 18 (LR-52)<br>human Insulin Growth Factor 1 - Receptor | SEQ ID NO: 18 | 5'-TCC TCC GGA GCC AGA CTT-3' |
| ODN 19 (hEGFR)<br>human Epidermal Growth Factor-Receptor | SEO ID NO: 19 | 5'-AAC GTT GAG GGG CAT-3' |
| ODN 20 (EGFR) | SEO ID NO: 20 | 5'-CCGTGGTCA TGCTCC-3' |

(continued)

| ODN NAME | ODN SEQ ID NO | ODN SEQUENCE (5'-3') |
|---|---|---|
| Epidermal Growth Factor - Receptor | | |
| ODN 21 (hVEGF) human Vascular Endothelial Growth Factor | SEQ ID NO: 21 | 5'-CAG CCTGGCTCACCG CCTTGG-3' |
| ODN 22 (PS-4189) murine Phosphokinase C - alpha | SEQ ID NO: 22 | 5'-CAG CCA TGG TTC CCC CCA AC-3' |
| ODN 23 (PS-3521) | SEO ID NO: 23 | 5'-GTT CTC GCT GGT GAG TTT CA-3' |
| ODN 24 (hBcl-2) human Bcl-2 | SEQ ID NO: 24 | 5'-TCT CCCAGCGTGCGCCAT-3' |
| ODN 25 (hC-Raf-1) human C-Raf-s | SEQ ID NO: 25 | 5'-GTG CTC CAT TGA TGC-3' |
| ODN #26 (hVEGF-R1) human Vascular Endothelial Growth Factor Receptor-1 | SEO ID NO: 26 | 5'-GAGUUCUGAUGAGGCCGAAAGGCCG AAAGUCUG-3' |
| ODN #27 | SEO ID NO: 27 | 5'-RRCGYY-3' |
| ODN # 28 (INX-3280) | .SEO ID NO: 28 | 5'-AACGTTGAGGGGCAT-3- |
| ODN #29 (INX-6302) | SEO ID NO: 29 | 5'-CAACGTTATGGGGAGA-3' |
| ODN #30 (INX-6298) human c-myc | SEO ID NO: 30 | 5'-TAACGTTGAGGGGCAT-3' |
| "Z" represents a methylated cytosine residue. Note: ODN 14 is a 15-mer oligonucleotide and ODN 1 is the same oligonucleotide having a thymidine added onto the 5' end making ODN I into a 16-mer. No difference in biological activity between ODN 14 and ODN 1 has been detected and both exhibit similar immunostimulatory activity (Mui *et al.,* 2001) | | |

**Lipids and other Components**

Lipid formulations and methods of preparing liposomes as delivery vehicles are known in the art, and any of number of such formulations may find advantageous use herein, including those described in U.S. Patent No. 6,465,439, U.S. Patent No. 6,379,698, U.S. Patent No. 6,365.611, and U.S. Patent No. 6,093.816 . Preferred lipid formulations are the lipid particle formulations described herein and more fully described in, for example, U.S. Patent No. 5,785,992, U.S. Patent No. 6,287,591, U.S. Patent No. 6,287,591 B1, co-pending U.S. Patent Appln. Ser. No. 60/379,343, and co-pending U.S. Patent Appln. Ser. No. 09/649,527.

In one preferred embodiment, the preferred lipid formulation is DSPC, DODMA, Chol, and PEG-DMG having a ratio of 20:25:45:10 mol/mol. As used herein, the molar amount of each lipid in a lipid formulation is given in the same order that the lipid is listed (e.g., the ratio of DSPC to DODMA to Chol to PEG-DMG is 20 DSPC: 25 DODMA: 45 Chol; 10 PEG-DMG or "20:25:45:10"). In alternate embodiments the DSPC may be replaced with POPC, the DODMA replaced with DODAP and the PEG-DMG replaced with PEGCer14 or PEGCer20.

The term "lipid" refers to a group of organic compounds that are esters of fatty acids and are characterized by being insoluble in water but soluble in many organic solvents. They are usually divided in at least three classes: (1) "simple lipids" which include fats and oils as well as waxes; (2) "compound lipids" which include phospholipids and glycolipids; and (3) "derived lipids" such as steroids and compounds derived from lipid manipulations. A wide variety of lipids may be used with the invention, some of which are described below.

The term "charged lipid" refers to a lipid species having either a cationic charge or negative charge or which is a zwitterion which is not net neutrally charged, and generally requires reference to the pH of the solution in which the lipid is found.

Cationic charged lipids at physiological pH include, but are not limited to, N,N-dioleyl-N,N-dimethylammonium chloride ("DODAC"); N-(2,3-dioleyloxy)propyl)-N,N,N-trimethylammonium chloride ("DOTMA"); N,N-distearyl-N,N-dimethylammonium bromide ("DDAB"); N-(2,3-dioleyloxy)propyl)-N,N,N-trimethylammonium chloride ("DOTAP"); 3b-(N-(N',N'-dimethylaminoethane)-carbamoyl)cholesterol ("DC-Chol") and N-(1,2-dimyristyloxyprop-3-yl)-N,N-dimethyl-N-hydroxyethyl ammonium bromide ("DMRIE"). Additionally, a number of commercial preparations of catioinic lipids are available which can be used in the present invention. These include, for example, Lipofectin™ (commercially available cationic liposomes comprising DOTMA and 1,2-dioleoyl-sn-3-phosphoethanolamine ("DOPE"), from GIBCOBRL, Grand Island, New York, U.S.A); and Lipofectamine™ (commercially available cationic liposomes comprising N-(1-(2,3-dioleyloxy)propyl)-N-(2-(sperminecarboxamido)ethyl)-N,N-dimethylammonium trifluoroacetate ("DOSPA") and DOPE from GIBCO/BRL).

Some cationic charged lipids are titratable, that is to say they have a pKa at or near physiological pH, with the significant consequence for this invention that they are strongly cationic in mild acid conditions and weakly (or not) cationic at physiological pH. Such cationic charged lipids include, but are not limited to, N-(2,3-dioleyloxy)propyl)-N,N-dimethylammonium chloride ("DODMA") and 1,2-Dioleoyl-3-dimethylammonium-propane ("DODAP"). DMDMA is also a useful titratable cationic lipid.

Anionic charged lipids at physiological pH include, but are not limited to, phosphatidyl inositol, phosphatidyl serine, phosphatidyl glycerol, phosphatidic acid, diphosphatidyl glycerol, poly(ethylene glycol)-phosphatidyl ethanolamine, dimyristoylphosphatidyl glycerol, dioleoylphosphatidyl glycerol, dilauryloylphosphatidyl glycerol, dipalmitoylphosphatidyl glycerol, distearyloylphosphatidyl glycerol, dimyristoyl phosphatic acid, dipalrriitoyl phosphatic acid, dimyristoyl phosphatidyl serine, dipalmitoyl phosphatidyl serine, brain phosphatidyl serine, and the like.

Some anionic charged lipids may be titrateable, that is to say they would have a pKa at or near physiological pH, with the significant consequence for this invention that they are strongly anionic in mild base conditions and weakly (or not) anionic at physiological pH. Such anionic charged lipids can be identified by one skilled in the art based on the principles disclosed herein.

The term "neutral lipid" refers to any of a number of lipid species which exist either in an uncharged or neutral zwitterionic form at physiological pH. Such lipids include, for example, diacylphosphatidylcholine, diacylphosphatidylethanolamine, ceramide, sphingomyelin, cephalin, cholesterol, cerebrosides and diacylglycerols.

Certain preferred lipid formulations used in the invention include aggregation preventing compounds such as PEG-lipids or polyamide oligomer-lipids (such as an ATTA-lipid), and other steric-barrier or "stealth"-lipids, detergents, and the like. Such lipids are described in U.S. Patent No. 4,320,121, U.S. Patent No. 5,820,873, U.S. Patent No. 5,885,613, Int. Publ. No. WO 98/51278, and U.S. Patent Appln. Serial No. 09/218,988 relating to polyamide oligomers. These lipids and detergent compounds prevent precipitation and aggregation of formulations containing oppositely charged lipids and therapeutic agents. These lipids may also be employed to improve circulation lifetime *in vivo* (see Klibanov et al. (1990) FEBS Letters, 268 (1): 235-237), or they may be selected to rapidly exchange out of the formulation *in vivo* (see U.S. Patent No. 5.885,613).

A preferred embodiment of the invention employs exchangeable steric-barrier lipids (as described in U.S. Patent No. 5.820,873, U.S. Patent No. 5,885,613, and U.S. Patent Appln. Ser. No. 09/094540 and U.S. Patent No. 6,320,017). Exchangeable steric-barrier lipids such as PEG2000-CerC14 and ATTA8-CerC14 are steric-barrier lipids which rapidly exchange out of the outer monolayer of a lipid particle upon administration to a subject/patient. Each such lipid has a characteristic rate at which it will exchange out of a particle depending on a variety of factors Including acyl chain length, saturation, size of steric barrier moiety, membrane composition and serum composition, etc. Such lipids are useful in preventing aggregation during particle formation, and their accelerated departure from the particle upon administration provides benefits, such as programmable fusogenicity and particle destabilizing activity, as described in the above noted patent submissions.

Some lipid particle formulations may employ targeting moieties designed to encourage localization of liposomes at certain target cells or target tissues. Targeting moieties may be associated with the outer bilayer of the lipid particle (i.e., by direct conjugation, hydrophobic interaction or otherwise) during formulation or post-formulation. These methods are well known in the art. In addition, some lipid particle formulations may employ fusogenic polymers such as PEAA, hemagluttinin, other lipo-peptides (see U.S. Patent No. 6,417,326, and U.S. Patent Appln. Ser. No. 09/674,191 and other features useful for *in vivo* and/or intracellular delivery.

In another preferred embodiment, the lipid component lipid particles of the present invention comprises sphingomyelin and cholesterol ("sphingosomes"). In a preferred embodiment, the lipid particles used in the compositions and uses of the present invention are comprised of sphingomyelin and cholesterol and have an acidic intraliposomal pH. The lipid particles comprising sphingomyelin and cholesterol have several advantages when compared to , other formulations. The sphingomyelin/cholesterol combination produces liposomes which have extended circulation lifetimes, are much more stable to acid hydrolysis, have significantly better drug retention characteristics, have better loading characteristics into tumors and the like, and show significantly better anti-tumor efficacy than other liposomal formulations tested.

In a preferred embodiment, the lipid particles of the present invention comprise a cationic compound of Formula I and at least one neutral lipid as follows (and fully described in U.S. Pat. Serial No. 5,785,992). In a preferred embodiment, the LNA formulations of the present invention comprise a cationic compound of Formula I and at least one neutral lipid as follows (and fully described in U.S. Pat. Serial No. 5,785,992,

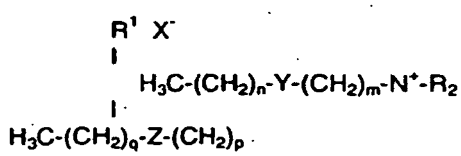

In Formula I, $R^1$ and $R^2$ are each independently $C_1$ to $C_3$; alkyl. Y and Z are akyl or alkenyl chains and are each independently: $-CH_2CH_2CH_2CH_2CH_2$--, --$CH=CHCH_2CH_2CH_2$--, $-CH_2$ $CH=CHCH_2CH_2$--, --$CH_2CH_2CH=CHCH_2$--, $-CH_2CH_2CH_2CH=CH$-. $-CH=CHCH=CHCH_2$--, --$CH=CHCH_2CH=CH$--, or --$CH_2CH=CH$-$CH=CH$--, with the proviso that Y and Z are not both $-CH_2CH_2CH_2CH_2CH_2$--. The letters n and q denote integers of from 3 to 7, while the letters m and p denote integers of from 4 to 9, with the proviso that the sums n+m and q+p are each integers of from 10 to 14. The symbol X' represents a pharmaceutically acceptable anion. In the above formula, the orientation of the double bond can be either cis or trans, however the cis isomers are generally preferred.

In another preferred embodiment, the cationic compounds are of Formula I, wherein $R^1$ and $R^2$ are methyl and Y and Z are each independently: --$CH=CHCH_2CH_2CH_2$--,-$CH_2CH=CHCH_2CH_2$--,- $CH_2CH_2CH=CHCH_2$-- or --$CH_2CH_2CH_2CH=CH$--. In preferred embodiments, $R^1$ and $R^2$ are methyl; Y and Z are each $-CH=CHCH_2H_2CH_2$--; n and q are both 7; and m and p are both 5. In another preferred embodiment, the cationic compound is DODAC (N,N-dioleyl-N,N-dimethylammonium chloride). DODAC is a known in the art and is a compound used extensively as an additive in detergents and shampoos. DODA is also used as a co-lipid in liposomal compositions with other detergents (see, Takahashi, et al., GB 2147243).

The neutral lipids in the LNA formulations of the present invention can be any of a variety of neutral lipids which are typically used in detergents, or for the formation of micelles or liposomes. Examples of neutral lipids which are useful in the present compositions are, but are not limited to, diacylphosphatidylcholine, diacylphosphatidylethanolamine, ceramide, sphingomyelin, cephalin, cardiolipin, and cerebrosides. In a preferred embodiment, the present compositions will include one or more neutral lipids which are diacylphosphatidylcholine, diacylphosphatidylethanolamine, ceramide or sphingomyelin. The acyl groups in these neutral lipids are preferably acyl groups derived from fatty acids having $C_{10}$-$C_{24}$ carbon chains. More preferably the acyl groups are lauroyl, myristoyl, palmitoyl, stearoyl or oleoyl. In particularly preferred embodiments, the neutral lipid will be 1,2-sn-dioleoylphosphatidylethanolamine.

The anion, X-, can similarly be any of a variety a pharmaceutically acceptable anions. These anions can be organic or inorganic, including for example, $Br^-$, $Cl^-$, $F^-$, $I^-$, sulfate, phosphate, acetate, nitrate, benzoate, citrate, glutamate, and lactate. In preferred embodiments, $X^-$ is $Cl^-$ or $AcO^-$.

In addition to the other components described herein, the compositions of the present invention may contain a pharmaceutically acceptable carrier. Pharmaceutically acceptable carriers are well-known in the art. The choice of carrier is determined in part by the particular composition to be administered as well as by the particular method used to administer the composition. Preferably, the pharmaceutical carrier is in solution, in water or saline.

In the compositions of the present invention, the ratio of cationic compound to neutral lipid is preferably within a range of from about 25:75 (cationic compound:neutral lipid), or preferably to 75:25 (cationic compound:neutral lipid), or preferably about 50:50.

The cationic compounds which are used in the compositions of the present invention can be prepared by methods known to those of skill in the art using standard synthetic reactions (see March, Advanced Organic Chemistry, 4th Ed., Wiley-Interscience, NY, N.Y. (1992) ). For example, the synthesis of OSDAC can be carried out by first treating oleylamine with formaldehyde and sodium cyanoborohydride under conditions which result in the reductive alklation of the amine. This approach provides dimethyl oleylamine, which can then be alkylated with stearyl bromide to form the corresponding ammonium salt. Anion exchange results in the formation of OSDAC. Dimethyloleylamine can also be synthesized by treatment of oleyl bromide with a large excess of dimethylamine, and further derivatized as described above.

For cationic compounds in which both fatty acid chains are unsaturated (i.e., DODAC), the following general procedure can be used. An unsaturated acid (i.e., oleic acid) can be converted to its corresponding acyl chloride with such reagents as oxalyl chloride, thionyl chloride, PCI3 or PCI5. The acyl chloride can be treated with an unsaturated amine (i.e., oleylamine) to provide the corresponding amide. Reduction of the amide with, for example, lithium aluminum hydride provides a secondary amine wherein both alkyl groups are unsaturated long chain alkyl groups. The secondary amine can then be treated with alkyl halides such as methyl iodide to provide a quaternary ammonium compound. Anion exchange can then be carried out to provide cationic compounds having the desired pharmaceutically acceptable anion. The alkylamine precursor can be synthesized in a similar manner. For example, treatment of an alkyl halide with a methanolic solution of ammonia in large excess will produce the required amine after purification. Alternatively, an acyl chloride, produced by treatment of the appropriate carboxylic acid with oxalyl chloride, can be reacted with ammonia to

produce an amide. Reduction of the amide with LiAlH4 will provide the required alkylamine.

In preferred embodiments, the pharmaceutical compositions of the present invention are formulated as micelles or liposomes. Micelles containing the cationic compounds and neutral lipids of the present invention can be prepared by methods well known in the art. In addition to the micellar formulations of the present compositions, the present invention also provides micellar formulations which include other species such as lysophosphatidylcholine, lysophosphatidylethanolamine, lysophosphatidyiserine, lysophosphatidylglycerol, phosphatidylethanolamine-polyoxyethylene conjugate, ceramide-polyoxyethylene conjugate or phosphatidic acid-polyoxyethylene conjugate.

The polyoxyethylene conjugates which are used in the compositions of the present invention can be prepared by combining the conjugating group (i.e. phosphatidic acid or phosphatidylethanolamine) with an appropriately functionalized polyoxyethylene derivative. For example, phosphatidylethanolamine can be combined with omega-methoxypolyethyleneglycol succinate to provide a phosphatidylethanolamine-polyoxyethylene conjugate (see, e.g., Parr, et al., Biochim. Biophys. Acta 1195:21-30 (1994).

The selection of neutral lipids for use in the compositions and uses of the present invention is generally guided by consideration of, e.g.. liposome size and stability of the liposomes in the bloodstream. As described above, the neutral lipid component in the liposomes is a lipid having two acyl groups, (i.e., diacylphosphatidylcholine and diacylphosphatidylethanolamine). Lipids having a variety of acyl chain groups of varying chain length and degree of saturation are available or may be isolated or synthesized by well-known techniques. In general, less saturated lipids are more easily sized, particularly when the liposomes must be sized below about 0.3 microns, for purposes of filter sterilization. In one group of embodiments, lipids containing saturated fatty acids with carbon chain lengths in the range of C14 to C22 are preferred. In another group of embodiments, lipids with mono or diunsaturated fatty acids with carbon chain lengths in the range of C14 to C22 are used. Additionally, lipids having mixtures of saturated and unsaturated fatty acid chains can be used.

Liposomes useful in the compositions and methods of the present invention may also be composed of sphingomyelin or phospholipids with other head groups, such as serine and lnositol. Still other liposomes useful in the present invention will include cholesterol, diglycerides, ceramides, phosphatidylethanolamine-polyoxyethylens conjugates, phosphatidic acid-polyoxyethylene conjugates, or polyethylene glycol-ceramide conjugates (*e.g.,* PEG-Cer-$C_{14}$ or PEG-Cer-C20). Methods used in sizing and fifter-sterilizing liposomes are discussed below.

A variety of methods are known in the art for preparing liposomes (see e.g., Szoka et al., Ann. Rev. Biophys. Bioeng. 9:467 (1980). U.S. Pat. Nos. 4,235,871, 4,501,728, 4,837,028, the text Liposomes, Marc J. Ostro, ed., Martel Dekker, Inc., New York, 1983, Chapter 1, and Hope, et al., Chem. Phys. Lip. 40:89 (1986). One known method produces multilamellar vesicles of heterogeneous sizes. In this method, the vesicle-forming lipids are dissolved in a suitable organic solvent or solvent system and dried under vacuum or an inert gas to form a thin lipid film. If desired, the film may be redissolved in a suitable solvent, such as tertiary butanol, and then lyophilized to form a more homogeneous lipid mixture which is in a more easily hydrated powder-like form. This film is covered with an aqueous buffered solution and allowed to hydrate, typically over a 15-60 minute period with agitation. The size distribution of the resulting multilamellar vesicles can be shifted toward smaller sizes by hydrating the lipids under more vigorous agitation conditions or by adding solubilizing detergents such as deoxycholate.

Following liposome preparation, the liposomes may be sized to achieve a desired size range and relatively narrow distribution of liposome sizes. A size range of about 0.2-0.4 microns allows the liposome suspension to be sterilized by filtration through a conventional filter, typically a 0.22 micron filter. The filter sterilization method can be carried out on a high through-put basis if the liposomes have been sized down to about 0.2-0.4 microns.

Several techniques are available for sizing liposomes to a desired size. One sizing method is described in U.S. Patent No. 4,737,323. Sonicating a liposome suspension either by bath or probe sonication produces a progressive size reduction down to small unilamellar vesicles less than about 0.05 microns in size. Homogenization is another method which relies on shearing energy to fragment large liposomes into smaller ones. In a typical homogenization procedure, multilamellar vesicles are recirculated through a standard emulsion homogenizer until selected liposome sizes, typically between about 0.1 and 0.5 microns, are observed. In both methods, the particle size distribution can be monitored by conventional laser-beam particle size discrimination.

Extrusion of liposomes through a small-pore polycarbonate membrane or an asymmetric ceramic membrane is also an effective method for reducing liposome sizes to a relatively well-defined size distribution. Typically, the suspension is cycled through the membrane one or more times until the desired liposome size distribution is achieved. The liposomes may be extruded through successively smaller-pore membranes, to achieve a gradual reduction in liposome size. For use in the present inventions, liposomes having a size of from about 0.05 microns to about 0.15 microns are preferred.

As further described below, the compositions of the present invention can be administered to a subject by any known route of administration. Once adsorbed by cells, the liposomes (including the complexes previously described) can be endocytosed by a portion of the cells, exchange lipids with cell membranes, or fuse with the cells. Transfer or incorporation of the polyanionic portion of the complex can take place via any one of these pathways. In particular, when fusion takes place, the liposomal membrane can be integrated into the cell membrane and the contents of the liposome

can combine with the intracellular fluid.

As described below in detail, additional components, which may also be therapeutic compounds, may be added to the lipid particles of the present invention to target them to specific cell types. For example, the liposomes can be conjugated to monoclonal antibodies or binding fragments thereof that bind to epitopes present only on specific cell types, such as cancer-related antigens, providing a means for targeting the liposomes following systemic administration. Alternatively, ligands that bind surface receptors of the target cell types may also be bound to the liposomes. Other means for targeting liposomes may also be employed in the present invention.

Following a separation step as may be necessary to remove free agent from the medium containing the liposome, the liposome suspension is brought to a desired concentration in a pharmaceutically acceptable carrier for administration to the patient or host cells. Many pharmaceutical acceptable carriers may be employed in the compositions and uses of the present invention. A variety of aqueous carriers may be used, e.g., water, buffered water, 0.4% saline, 0.3% glycine, and the like, and may include glycoproteins for enhanced stability, such as albumin, lipoprotein, globulin. Generally, normal buffered saline (135-150 mM NaCl) will be employed as the pharmaceutically acceptable carrier, but other suitable carriers will suffice. These compositions may be sterilized by conventional liposomal sterilization techniques, such as filtration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride. These compositions may be sterilized techniques referred to above or produced under sterile conditions. The resulting aqueous solutions may be packaged for use or filtered under aseptic conditions and lyophilized, the lyophilized preparation being combined with a sterile aqueous solution prior to administration.

The concentration of liposomes in the carrier may vary. In preferred embodiments, the concentration of liposomes is about 0.1-200 mg/ml. Persons of skill would know how to vary these concentrations to optimize treatment with different liposome components or for particular patients. For example, the concentration may be increased to lower the fluid load associated with treatment.

The cells of a subject are usually exposed to the compositions of the present invention by *in vivo* or *ex vivo* administration. In the preferred embodiments described herein, the compositions of the present invention are administered systemically, e.g., intravenously, with intramuscular, subcutaneous and topical administration also contemplated. Alternatively, intranasal or intratracheal administration may be used. Intratracheal administration may be provided as a liquid, preferably as an aerosol. For example, nebulizers may be used to create aerosols of droplets of between 70-100 μm in diameter. It will be understood that droplet size should generally be of greater size than the liposomes.

Multiple administrations to a patient are contemplated. The dosage schedule of the treatments will be determined by the disease and the patient's condition. Standard treatments with therapeutic compounds, including immunostimulatory compositions *(e.g.,* vaccines), that are well known in the art may serve as a guide to treatment with liposomes containing the therapeutic compounds. The duration and schedule of treatments may be varied by methods well known to those of skill, but the increased circulation time and decreased in liposome leakage will generally allow the dosages to be adjusted downward from those previously employed. The dose of liposomes of the present invention may vary depending on the clinical condition and size of the animal or patient receiving treatment. The standard dose of the therapeutic compound when not encapsulated may serve as a guide to the dose of the liposome-encapsulated compound. The dose will typically be constant over the course of treatment, although in some cases the dose may vary. Standard physiological parameters may be assessed during treatment that may be used to alter the dose of the liposomes of the invention.

### Antigens

The pathogen vaccines of the present invention further comprise at least one epitope of at least one antigen derived from a pathogen, either mixed with or more preferably associated with the LNA formulations described above. Pathogens which may be targeted by the subject vaccines include, but are not limited to infectious virus, infectious bacteria, infectious parasites and infectious fungi. Most preferably, polytope vaccines are provided comprising a plurality of epitopes from one or more such antigens. The microbial antigens finding use in the subject compositions and methods may be inherently immunogenic, or non-immunogenic, or slightly immunogenic. Exemplary antigens include, but are not limited to, synthetic, recombinant, foreign, or homologous antigens, and antigenic materials may include but are not limited to proteins, peptides, polypeptides, lipids, glycolipids, carbohydrates and DNA.

Exemplary viral pathogens include, but are not limited to, infectious virus that infect mammals, and more particularly humans. Examples of infectious virus include, but are not limited to: Retroviridae (e.g. human immunodeficiency viruses, such as HIV-1 (also referred to as HTLV-III, LAV or HTLV-III/LAV, or HIV-III; and other isolates, such as HIV-LP; Picomaviridae (e.g. polio viruses, hepatitis A virus; enteroviruses, human Coxsackie viruses, rhinoviruses, echoviruses); Calciviridae (e.g. strains that cause gastroenteritis); Togaviridae (e.g. equine encephalitis viruses, rubella viruses); Flaviridae (e.g. dengue viruses, encephalitis viruses, yellow fever viruses); Coronoviridae (e.g. coronaviruses);

Rhabdoviradae (e.g. vesicular stomatitis viruses, rabies viruses); Coronaviridae (e.g. coronaviruses); Rhabdoviridae (e.g. vesicular stomatitis viruses, rabies viruses); Filoviridae (e.g. ebola viruses); Paramyxoviridae (e.g. parainfluenza viruses, mumps virus, measles virus, respiratory syncytial virus); Orthomyxoviridae (e.g. influenza viruses); Bungaviridae (e.g. Hantaan viruses, bunga viruses, phleboviruses and Nairo viruses); Arena viridae (hemorrhagic fever viruses); Reoviridae (e.g. reoviruses, orbiviurses and rotaviruses); Bimaviridae; Hepadnaviridae (Hepatitis B virus); Parvovirida (parvoviruses); Papovaviridae (papilloma viruses, polyoma viruses); Adenoviridae (most adenoviruses); Herpesviridae herpes simplex virus (HSV) 1 and 2, varicella zoster virus, cytomegalovirus (CMV), herpes virus; Poxviridae (variola viruses, vaccinia viruses, pox viruses); and Iridoviridae (e.g. African swine fever virus); and unclassified viruses (e.g. the etiological agents of Spongiform encephalopathies, the agent of delta hepatitis (thought to be a defective satellite of hepatitis B virus), the agents of non-A, non-B hepatitis (class 1=intemally transmitted; class 2=parenterally transmitted (i.e. Hepatitis C); Norwalk and related viruses, and astroviruses).

Also, gram negative and gram positive bacteria may be targeted by the subject compositions and methods in vertebrate animals. Such gram positive bacteria include, but are not limited to Pasteurella species, Staphylococci species, and Streptococcus species. Gram negative bacteria include, but are not limited to, Escherichia coli, Pseudomonas species, and Salmonella species. Specific examples of infectious bacteria include but are not limited to: Helicobacterpyloris, Borelia burgdorferi, Legionella pneumophilia, Mycobacteria sps (e.g. M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes (Group A Streptococcus), Streptococcus agalactiae (Group B Streptococcus), Streptococcus (viridans group), Streptococcusfaecalis, Streptococcus bovis, Streptococcus (anaerobic sps.), Streptococcus pneumoniae, pathogenic Campylobacter sp., Enterococcus sp., Haemophilus infuenzae, Bacillus antracis, corynebacterium diphtheriae, corynebacterium sp., Erysipelothrix rhusiopathiae, Clostridium perfringers, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, Rickettsia, and Actinomyces israelli.

Polypeptides of bacterial pathogens which may find use as sources of microbial antigens in the subject compositions include but are not limited to an iron-regulated outer membrane protein, ("IROMP"), an outer membrane protein ("OMP"), and an A-protein of Aeromonis salmonicida which causes furunculosis, p57 protein of Renibacterium salmoninarum which causes bacterial kidney disease ("BKD"), major surface associated antigen ("msa"), a surface expressed cytotoxin ("mpr"), a surface expressed hemolysin ("ish"), and a flagellar antigen of Yersiniosis; an extracellular protein ("ECP"), an iron-regulated outer membrane protein ("IROMP"), and a structural protein of Pasteurellosis; an OMP and a flagellar protein of Vibrosis anguillarum and V. ordalii; a flagellar protein, an OMP protein,aroA, and purA of Edwardsiellosis ictaluri and E. tarda; and surface antigen of Ichthyophthirius; and a structural and regulatory protein of Cytophaga columnari; and a structural and regulatory protein of Rickettsia. Such antigens can be isolated or prepared recombinantly or by any other means known in the art.

Examples of pathogens further include, but are not limited to, infectious fungi that infect mammals, and more particularly humans. Examples of infectious fungi include, but are not limited to: Cryptococcus neoformans, Histoplasma capsulatum, Coccidioides immitis, Blastomyces dermatitidis, Chlamydia trachomatis, Candida albicans. Examples of infectious parasites include Plasmodium such as Plasmodium falciparum, Plasmodium malariae, Plasmodium ovale, and Plasmodium vivax. Other infectious organisms (i.e. protists) include Toxoplasma gondii. Polypeptides of a parasitic pathogen include but are not limited to the surface antigens of Ichthyophthirius.

Other medically relevant microorganisms that serve as antigens in mammals and more particularly humans are described extensively in the literature, e.g., see C. G. A Thomas, Medical Microbiology, Bailliere Tindall, Great Britain 1983 . In addition to the treatment of infectious human diseases and human pathogens, the compositions and methods of the present invention are useful for treating infections of nonhuman mammals. Many vaccines for the treatment of non-human mammals are disclosed in Bennett, K. Compendium of Veterinary Products, 3rd ed. North American Compendiums, Inc., 1995; *see also* WO 02/069369.

Exemplary non-human pathogens include, but are not limited to, mouse mammary tumor virus ("MMTV"), Rous sarcoma virus ("RSV"), avian leukemia virus ("ALV"), avian myeloblastosis virus ("AMV"), murine leukemia virus ("MLV"), feline leukemia virus ("FeLV"), murine sarcoma virus ("MSV"), gibbon ape leukemia virus ("GALV"), spleen necrosis virus ("SNV"), reticuloendotheliosis virus ("RV"), simian sarcoma virus ("SSV"), Mason-Pfizer monkey virus ("MPMV"), simian retrovirus type 1 ("SRV-1"), lentiviruses such as HIV-1, HIV-2, SIV, Visna virus, feline immunodeficiency virus ("FIV"), and equine infectious anemia virus ("EIAV"), T-cell leukemia viruses such as HTLV-1, HTLV-II, simian T-cell leukemia virus ("STLV"), and bovine leukemia virus ("BLV"), and foamy viruses such as human foamy virus ("HFV"), simian foamy virus ("SFV") and bovine foamy virus ("BFV").

In preferred embodiments, "treatment", "treat", "treating" as used herein with reference to infectious pathogens, refers to a prophylactic treatment which increases the resistance of a subject to infection with a pathogen or decreases the likelihood that the subject will become infected with the pathogen; and/or treatment after the subject has become infected in order to fight the infection, e.g., reduce or eliminate the infection or prevent it from becoming worse.

Microbial antigens can be prepared by methods well known in the art. For example, these antigens can be

prepared directly from viral and bacterial cells either by preparing crude extracts, by partially purifying the antigens, or alternatively by recombinant technology or by de novo synthesis of known antigens. The antigen may also be in the form of a nucleic acid encoding an antigenic peptide in a form suitable for expression in a subject and presentation to the immune system of the immunized subject. Further, the antigen may be a complete antigen, or it may be a fragment of a complete antigen comprising at least one epitope.

The antigen of the lipid formulation may be encapsulated, associated, or mixed with the liposome or lipid particle. In certain embodiments of the present invention, the antigen is encapsulated in the liposome or lipid particle. In other embodiments, the antigen is mixed with the liposome or lipid particle. In other embodiments, the antigen is associated with the liposome or lipid particle. In one aspect, the antigen is adsorbed to the liposome or lipid particle. In other aspects, the antigen is covalently attached to the liposome or lipid particle. Methods used to covalently attach the antigen to the liposome or lipid particle are those standard methods known to those of skill in the art.

**Other Drug Components**

Some preferred embodiments of the invention further comprise other therapeutic agents, e.g., drugs or bioactive agents. These additional components may provide direct additional therapeutic benefit or additional immune-stimulating benefits. A wide variety of therapeutic compounds may be delivered by the compositions and methods of the present invention. Examples of therapeutic compounds include, but are not limited to, nucleic acids, proteins, peptides, oncolytics, anti-infectives, anxiolytics, psychotropics, immunomodulators, ionotropes, toxins such as gelonin and inhibitors of eucaryotic protein synthesis, and the like. Preferred therapeutic compounds for entrapment in the liposomes of the present invention are those which are lipophilic cations. Among these are therapeutic agents of the class of lipophilic molecules which are able to partition into a lipid bilayer phase of a liposome, and which therefore are able to associate with the liposomes in a membrane form. Further examples of therapeutic compounds include, but are not limited to, prostaglandins, amphotericin B, methotrexate, cisplatin and derivatives, progesterone, testosterone, estradiol, doxorubicin, epirubicin, beclomethasone and esters, vitamin E, cortisone, dexamethasone and esters, betamethasone valerete and other steroids, the fluorinated quinolone antibacterial ciprofloxacin and its derivatives, and alkaloid compounds and their derivatives. Among the alkaloid derivatives are swainsonine and members of the vinca alkaloids and their semisynthetic derivatives, such as, e.g., vinblastine, vincristine, vindesin, etoposide, etoposide phosphate, and teniposide. Among this group, vinblastine and vincristine, and swainsonine are particularly preferred. Swainsonine (Creaven and Mihich, Semin. Oncol. 4:147 (1977) has the capacity to stimulate bone marrow proliferation (White and Olden, Cancer Commun. 3:83 (1991)). Swainsonine also stimulates the production of multiple cytokines including IL-1, IL-2, TNF, GM-CSF and interferons (Newton, Cancer Commun. 1:373 (1989); Olden, K., J. Natl. Cancer Inst., 83:1149 (1991)). Further Swainsonine reportedly induces B- and T-cell immunity, natural killer T-cell and macrophage-induced destruction of tumor cells *in vitro,* and when combined with interferon, has direct anti-tumor activity against colon cancer and melanoma cancers *in vivo* (Dennis, J., Cancer Res., 50:1867 (1990); Olden, K., Pharm. Ther. 44:85 (1989); White and Olden, Anticancer Res., 10:1515 (1990)). Other alkaloids useful in the compositions and methods of the present invention include, but are not limited to, paclitaxel (taxol) and synthetic derivatives thereof. Additional drug components, include but are not limited to, any bioactive agents known in the art which can be incorporated into lipid particles.

These additional drug components may be encapsulated or otherwise associated the lipid particles described herein. Alternatively, the compositions of the invention may include drugs or bioactive agents that are not associated with the lipid-nucleic acid particle. Such drugs or bioactive agents may be in separate lipid carriers or co-administered.

**Manufacturing of Compositions**

Manufacturing the compositions of the invention may be accomplished by any technique, but most preferred are the ethanol dialysis or detergent dialysis methods detailed in the following publications, patents, and applications: U.S. Pat. Ser. No. 5,705,385; U.S. Pat. No. 5,976,567; U.S. Pat. Appln. No. 09/140,476; U.S. Pat. No. 5,981,501; U.S. Pat. No. 6,287,591; Int. Publ. No. WO 96/40964; and Int. Publ. No. WO 98/51278. These manufacturing methods provide for small and large scale manufacturing of immunostimulatory compositions comprising therapeutic agents encapsulated in a lipid particle, preferably lipid-nucleic acid particles. The methods also generate such particles with excellent pharmaceutical characteristics.

Pathogen vaccines of the present invention may be prepared by adding one or more microbial antigens to which the immune response is desired. Means of incorporating antigens are well known in the art and include, for example: 1) passive encapsulation of the antigen during the formulation process (e.g., the antigen can be added to the solution containing the ODN); 2) addition of glycolipids and other antigenic lipids to an ethanol lipid mixture and formulated using the ethanol-based protocols described herein; 3) insertion into the lipid vesicle (e.g.. antigen-lipid can be added into formed lipid vesicles by incubating the vesicles with antigen-lipid micelles); and 4) the antigen can be added post-formulation (e.g., coupling in which a lipid with a linker moiety is included into formulated particle, and the linker is

activated post formulation to couple a desired antigen). Standard coupling and cross-linking methodologies are well known in the art. An alternative preparation incorporates the antigen into a lipid-particle which does not contain a nucleic acid, and these particles are mixed with lipid-nucleic acid particles prior to administration to the subject.

Characterization of Compositions Used in the Uses of the Present Invention

Preferred characteristics of the compositions used in the uses of the present invention are as follows.

The preferred lipid-nucleic acid particles of the invention comprise a lipid membrane (generally a phospholipid bilayer) exterior which fully encapsulates an interior space. These particles, also sometimes herein called lipid membrane vesicles, are small particles with mean diameter 50-200.nm, preferably 60-130 nm. Most preferred for intravenous administrations are particles of a relatively uniform size wherein 95% of particles are within 30 nm of the mean. The nucleic acid and other bioactive agents are contained in the interior space, or associated with an interior surface of the encapsulating membrane.

"Fully encapsulated" as used herein indicates that the nucleic acid in the particles is not significantly degraded after exposure to serum or a nuclease assay that would significantly degrade free DNA. In a fully encapsulated system, preferably less than 25% of particle nucleic acid is degraded in a treatment that would normally degrade 100% of free nuctetc acid, more preferably less than 10% and most preferably less than 5% of the particle nucleic acid is degraded. Alternatively, full encapsulation may be determined by an Oligreen™ assay. Fully encapsulated also suggests that the particles are serum stable, that is, that they do not rapidly decompose into their component parts upon *in vivo* administration.

These characteristics of the compositions of the present invention distinguish the preferred particles of the invention from lipid-nucleic acid aggregates (also known as cationic complexes or lipoplexes) such as DOTMA/DOPE (LIPOFECTIN™) formulations. These aggregates are generally much larger (>250 nm) diameter, they do not competently withstand nuclease digestion. They generally decompose upon *in vivo* administration. Lipid-nucleic acid formulations comprising cationic lipid-nucteic acid aggregates with weak antigens, as described above, may provide suitable vaccines for local and regional applications, such as intramuscular, intra-peritioneal and intrathecal administrations, and more preferably intranasal administration.

The liposomal particles of the invention can be formulated at a wide range of drug:lipid ratios. "Drug to lipid ratio" as used herein refers to the amount of therapeutic nucleic acid (*i.e.,* the amount of nucleic acid which is encapsulated and which will not be rapidly degraded upon exposure to the blood) in a defined volume of preparation divided by the amount of lipid in the same volume. This may be determined on a mole per mole basis or on a weight per weight basis, or on a weight per mole basis. Drug to lipid ratio may determine the lipid dose that is associated with a given dose of nucleic acid. In a preferred embodiment, the compositions of the present invention have a drug:lipid ratio in the range of about 0.01 to 0.25 (wt/wt).

Uses of the Compositions and Uses of the Present Invention

As demonstrated herein, the subject pathogen vaccines are capable of stimulating a strong, Th-1 biased immune response against microbial antigens, and can enhance the humoral component of the host immune response. Thus, the pathogen vaccines described herein find use in inducing Th-1 biased humoral immunity to microbial antigens, as well as improving the maturation of the humoral response and increasing antibody isotype switching in response to antigenic stimulation.

These immune responses can be measured in many ways including but not limited to activation, proliferation or differentiation of cells of the immune system (e.g., B cells, T cells, APCs, such as dendritic cells or macrophages, NK cells, NKT cells etc.); up-regulated or downregulated expression of markers; cytokine secretion; stimulation of or increase in IgA, IgM, or IgG titer, isotype class switching, and splenomegaly (including increased spleen cellularity). The presence of a Th-1 biased immune response in particular can be determined directly by the induction of Th-1 cytokines (e.g., IFN-γ, IL-12) and antigen-specific CD8+ CTL Thus, if Th-1 cytokines or CTL are induced. Th-1 biased immune responses are induced according to the invention. Similarly, enhanced humoral responses and improvements in the maturation of the humoral response are indicated by detecting the isotype of type-1 antigen-specific antibodies that are induced (*e.g.,* IgG2a, IgG1 in mice. IgG and IgA in humans), and determining if isotype switching has occurred, *e.g.,* IgM to IgG or IgA, as exemplified herein. If increased isotype switching has occurred in comparison with alternative adjuvants, enhanced humoral immune responses are induced according to the invention.

In a preferred embodiment, the uses of the present invention comprise stimulating a Th1-baised immune response against a pathogen in a subject by administering to the subject an effective amount of a pathogen vaccine comprising at least one microbial antigen. Preferably the vaccine comprises an LNA particle comprising an encapsulated ODN. More preferably the antigen is associated with the LNA particle, and most preferably a plurality of antigens are employed. In a particularly preferred embodiment, the ODN comprises a PS or other modified, non-phosphodiester

backbone. Alternative adjuvants that induce Th1 responses include but are not limited to MPL, MDP, ISCOMS, IL-12, IFN-γ, and SB-AS2.

Examples of such pathogens are, but not limited to, HIV, HPV, HSV-1, HSV-2, HBV, SARS, Neisseria gonorrhea, Chlamydia, and Treponema pallidum which can provide antigens or DNA sequences encoding antigens for use in the methods of this invention. Thus, additional antigens suitable for use in the present invention include, but are not limited to, the L1 protein of HPV, the L2 protein of HPV, the E6 protein of HPV, the E7 protein of HPV, the surface protein of HBV, the "e" antigen of HBV, the gp41 protein of HIV, the gag protein of HIV, the tet protein of HIV and the gp120 glycoprotein of HIV, among others. Still other pathogens for which such vaccines and vaccine protocols of the present invention are useful include, but are not limited to, the pathogens that cause trichomoniasis, candidiasis, hepatitis, scabies, and syphilis. Further, pathogens which invade via the mucosa also include, but are not limited to, those that cause respiratory syncytial virus, flu, other upper respiratory conditions, as well as agents which cause intestinal infections. The methods of the present invention are also useful in stimulating mucosal immunity to such pathogens. Accordingly, the Invention encompasses the expression of antigens derived from a wide range of human pathogens to which mucosal immunity is desired. Thus, the invention is not limited by the identity of a particular antigen.

Neonates (newborn) and infants (which include humans three months of age and referred to hereinafter as infants) bom in HBV endemic areas require particularly rapid induction of strong HBV-specific immunity owing to the high rate of chronicity resulting from infection at a young age. Without immunoprophylaxis, 70-90% of infants bom to mothers positive for both HBsAg and the "e" antigen (HBeAg) become infected and almost all of these become chronic carriers (Stevens *et al.,* 1987). Even when vaccinated with a four dose regime of the HBV subunit vaccine commencing on the day of birth, 20% of such infants became chronically infected and this was reduced to only 15% if they were also given HBV-specific immunoglobulin (Chen *et al.* 1996) HBV chronicity results in 10-15% of individuals infected as adolescents or adults, but 90-95% for those infected (either vertically or horizontally) as infants. The compositions of the present invention could be prepared with HBe antigen and used in the methods of the present invention further reduce such chronic infections owing to a more rapid appearance and higher titers of anti-HB antibodies and the induction of HBV-specific CTL, which could help clear virus from the liver of babies infected in utero, and which likely account for most of the failures with infant vaccination.

**Indications, Administration and Dosages**

The compositions and uses of the present invention are indicated for use in any patient or organism having a need for immune system stimulation. Such a need encompasses, but is not limited to, most medical fiefds, such as oncology, inflammation, arthritis & rheumatology, immuno-deficiency disorders. One skilled in the art can select appropriate indications to test for efficacy based on the disclosure herein. In a preferred embodiment, the compositions and uses of the invention are used prophylactically or therapeutically to target any pathogen of interest such as Hepatitis B virus exemplified in the Examples below.

Administration of the compositions of the invention to a subject may be by any method including *in vivo* or ex vivo methods. *In vivo* methods can include local, regional or systemic applications. In a preferred embodiment, the compositions are administered intravenously such that particles are accessible to B cells, macrophages or a splenocytes in a patient, and/or the particle can stimulate lymphocyte proliferation, resulting in secretion of IL-6, IL-12, IFNg and/or IgM in said patient.

Vaccine compositions of the present invention may be administered by any known route of administration. In one embodiment, the compositions of the present invention are administered via intravenous injection. In another embodiment, intramuscular or subcutaneous injection is employed and in this manner larger-sized (150-300 nm) lipid particles can be used. Consequently, the need for costly extrusion steps can be reduced or eliminated, and since the particles do not need to circulate, the selection of lipid components can be biased in favor of less expensive materials. For example, the amount of Chol can be reduced, DSPC can be replaced with something less rigid (e.g., POPC or DMPC), and PEG-lipids can be replaced with less expensive PEG-acyl chains. In a still further embodiment, the compositions of the present invention are administered via the respiratory tract, e.g., by intratracheal instillation or intranasal inhalation.

One skilled in the art would know how to identify possible toxicities of formulations, for example, complement activation, coagulation, renal toxicities, liver enzyme assays, etc. Such toxicities may differ between organisms.

Pharmaceutical preparations of compositions usually employ additional carriers to improve or assist the delivery modality. Typically, compositions of the invention will be administered in a physiologically-acceptable carrier such as normal saline or phosphate buffer selected in accordance with standard pharmaceutical practice. Other suitable carriers include water, 0.9% saline, 0.3% glycine, and the like, including glycoproteins for enhanced stability, such as albumin, lipoprotein, globulin, etc.

Dosages of lipid-nucleic acid formulations depend on the desired lipid dosage, the desired nucleic acid dosage, and the drug:lipid ratio of the composition. One skilled in the art can select proper dosages based on the information

provided herein.

Immunotherapy or vaccination protocols for priming, boosting, and maintenance of immunity are well known in the art and further described below. In particular, one skilled in the art would know how to calculate dosage amounts for a subject, particularly a mammal, and more particularly a human, based on the dosage amounts described herein. Specific conversion factors for converting dosage amounts from one animal to another (e.g., from mouse to human) are well known in the art and are fully described, *e.g.,* on the Food and Drug Administration Web site at: www.fda.gov/cder/cancer/animalframe.htm (in the oncology tools section). As compared to known immunostimulatory compositions having free nucleic acids, the immunostimulatory compositions and uses of the present invention may utilize reduced amounts of nucleic acids to stimulate enhanced immune responses in *vivo.*

The amount of nucleic acids in the formulations of the present invention will generally vary between about 0.001-60 mg/kg (mg nucleic acids per kg body weight of a mouse per dose). In preferred embodiments for Intravenous (i.v.) administration, the compositions and methods of the present invention utilize about 1-50 mg/kg, more preferably about 5-20 mg/kg. In preferred embodiments for subcutaneous (s.c.) administration, the compositions and methods of the present invention utilize about 1-10 mg/kg, and more preferably about 1-5 mg/kg. usually about about 3-5 mg/kg. The amount of antigen associated with the lipid particles of the present invention is preferably about 0.04-40 mg/kg, and more preferably about 0.04-4 mg/kg. As described above, one skilled in the art could readily determine suitable dosage amounts for other mammals given the dosage amounts described herein, based on the well-known conversion factors identified above and further empirical testing.

The formulations of the invention may be administered in pharmaceutically acceptable solutions, which may routinely contain pharmaceutically acceptable concentrations of salt, buffering agents, preservatives, compatible carriers, adjuvants, and optionally other therapeutic ingredients.

For use in therapy, an effective amount of the immunostimulatory compositions of the present invention can be administered to a subject by any mode allowing uptake by the appropriate target cells. "Administering" the immunostimutatory composition of the present invention may be accomplished by any means known to the skilled artisan. Preferred routes of administration include but are not limited to parenteral injection (*e.g.,* subcutaneous, intradermal, intravenous, parenteral, intraperitoneal, intrathecal, etc.), mucosal, intranasal, intratracheal, inhalation, and intrarectal, intravaginal; or oral, transdermai (e.g., via a patch). An injection may be in a bolus or a continuous Infusion.

For example, the iminunostimulatory compositions of the present invention can be administered by intramuscular or intradermal injection, or other parenteral means, or by biolistic "gene-gun" application to the epidermis. The immunostimulatory compositions of the present invention may also be administered, for example, by inhalation, topically, intravenously, orally, implantation, rectally, or vaginally. Suitable liquid or solid pharmaceutical preparation forms are, for example, aqueous or saline solutions for injection or inhalation, encochleated, coated onto microscopic gold particles, and nebulized. For a brief review of present methods for drug delivery, see Langer, Science 249:1527-1533, 1990.

The pharmaceutical compositions are preferably prepared and administered in dose units. Liquid dose units are vials or ampoules for injection or other parenteral administration. Solid dose units are tablets, capsules and suppositories. For treatment of a patient, depending on activity of the compound, manner of administration, purpose of the immunization (i.e., prophylactic or therapeutic), nature and severity of the disorder, age and body weight of the patient, different doses may be necessary. The administration of a given dose can be carried out both by single administration in the form of an individual dose unit or else several smaller dose units. Multiple administration of doses at specific intervals of weeks or months apart is usual for boosting the antigen-specific responses.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

In preferred embodiments, the immunostimulatory compositions of the present invention contain an effective amount of a combination of adjuvants and antigens optionally included in a pharmaceutically-acceptable carrier. "Pharmaceutically-acceptable carrier" as used herein refers to one or more compatible solid or liquid filler, dilutants or encapsulating substances which are suitable for administration to a human or other mammal. "Carrier" as used herein refers to an organic or inorganic ingredient, natural or synthetic, with which the active ingredient is combined to facilitate the application. The components of the immunostimulatory compositions of the present invention also are capable of being comingled with the compounds of the present invention, and with each other, in a manner such that there is no interaction which would substantially impair the desired pharmaceutical efficiency.

Compositions suitable for parenteral administration conveniently comprise sterile aqueous preparations, which can be isotonic with the blood of the recipient. Among the acceptable vehicles and solvents are water, Ringer’s solution, phosphate buffered saline and isotonic sodium chloride solution. In addition, sterile, fixed oils are conventionally employed as a solvent or suspending medium. For this purpose any bland fixed mineral or non-mineral oil may be employed including synthetic mono-ordi-glycerides. In addition, fatty acids such as oleic acid find use in the preparation of injectables. Carrier formulations suitable for subcutaneous, intramuscular, intraperitoneal, intravenous, etc. administrations may be found in Remington’s Pharmaceutical Sciences, Mack Publishing Company, Easton, Pa.

The adjuvants or antigens useful in the invention may be delivered in mixtures of more than two adjuvants or antigens. A mixture may consist of several adjuvants in addition to the LNA formulations described herein.

A variety of administration routes are available. The particular mode selected will depend, of course, upon the particular adjuvants or antigen selected, the age and general health status of the subject, the particular condition being treated and the dosage required for therapeutic efficacy. The methods of this invention, generally speaking, may be practiced using any mode of administration that is medically acceptable, meaning any mode that produces effective levels of an immune response without causing clinically unacceptable adverse effects. Preferred modes of administration are discussed above.

The compositions may conveniently be presented in unit dosage form and may be prepared by any of the methods well known in the art of pharmacy. All methods include the step of bringing the compounds into association with a carrier which constitutes one or more accessory ingredients. In general, the compositions are prepared by uniformly and intimately bringing the compounds into association with a liquid carrier, a finely divided solid carrier, or both, and then, if necessary, shaping the product.

Other delivery systems can include time-release, delayed release or sustained release delivery systems. Such systems can avoid repeated administrations of the compounds, increasing convenience to the subject and the physician. Many types of release delivery systems are available and known to those of ordinary skill in the art. They include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Patent No. 5,075,109. Delivery systems also include non-polymer systems that are: lipids including sterols such as cholesterol, cholesterol esters and fatty acids or neutral fats such as mono-di-and tri-glycerides; hydrogel release systems; sylastic systems; peptide based systems; wax coatings; compressed tablets using conventional binders and excipients; partially fused implants; and the like. Specific examples include, but are not limited to: (a) erosional systems in which an agent of the invention is contained In a form within amatrix such as those described in U.S. Patent Nos. 4,452,775, 4,675,189, and 5,736,152, and (b) diffusional systems in which an active component permeates at a controlled rate from a polymer such as described in U.S. Patent Nos. 3,854,480, 5,133,974 and 5,407,686. In addition, pump-based hardware delivery systems can be used, some of which are adapted for implantation.

Turning now to certain particular aspects of the present invention, one aspect arises from the recognition that encapsulated PS ODN are capable of stimulating enhanced Th-1 biased humoral immune responses to antigenic stimulation and improving the maturation of the humoral response in comparison with encapsulated PO ODN.

EXPERIMENTAL

**Experimental Details**

Mice. Female, Balb/c or C57/BL6 ("B6") mice (6-8 weeks) were purchased from Harlan-Sprague Dawley (Indianapolis, IN). All animals were quarantined for one week prior to use. All studies were conducted in accordance with the guidelines established by the Canadian Council on Animal Care (CCAC) and the Institutional Animal Care and User Committee (IACUC).

Peptides. Peptides were obtained from Commonwealth Biotechnologies and were >95% pure as determined by HPLC. QC analyses were obtained with each peptide.

Oligonucleotide formulations. Oligonucleotides were obtained from Avecia or Proligo or Trilink. All ODN were rehydrated in sterile water and diluted to the appropriate concentrations in sterile DPBS, pH 7.2.

LNA formulations. LNA formulations were prepared as follows. Initial ODN:lipid ratios were 0.25, w/w. For phosphodiester formulations, 20 mM citrate buffer was used in place of 300 mM citrate buffer to dissolve the ODN. Failure to do this results in considerably diminished encapsulation efficiency (i.e. - 3% final).

Vaccinations. B6 mice were vaccinated subcutaneously (SC) with 100 ml per injection. Dosing schedules were either q7dx2 or q4dx4 and are indicated in the individual figure legends. CFA mixtures were very viscous and had to be given with a larger gauge syringe. For humoral studies, a prime (day 0) and boost (day 14) strategy was used and blood samples were collected at various times by tail nicking.

Efficacy studies. E.G7-OVA or EL-4 thymoma cell lines were used throughout the study. These cells were cultured in vitro according to established methods. For tumor studies, 2.5 x 106 E.G7-OVA cells were injected subcutaneously in 50 ml of PBS containing 1% FCS. Tumor measurements were made by repeated perpendicular measurements of tumor dimensions and using the formula:

**Volume (mm3) = (L x W x H)p/6**

TRP-2 and gp100 studies were conducted using the B16/BL6 murine melanoma model. B16 cells (1.0x105) were injected IV in a volume of 200 ml. Typically, animals were vaccinated weekly for 2-3 injections and B16 cells were then administered 1-2 days after the final vaccination. Animals were terminated between days 14-18 post-B16 injection, lungs were removed, and metastases were counted using a stereomicroscope.

Flow cytometry analysis. Antigen specific T cells were determined in vaccinated mice at various times using either MHC tetramers (Beckman Coulter) or the Dimer X reagent (Pharmingen). Antibodies against CD8a, B220, and CD4 were used to identify cell populations of interest and gate out unwanted populations. Single cell suspensions of spleen and lymph nodes were prepared according to protocols outlined in Current Protocols in Immunology. Antibody stains were done on 1 x 106 cells in 96 well plates kept at 4˚C. Generally, 0.2 mg of each antibody was used per 1 x 106 cells.

## EXAMPLE 1

### *In vitro* vs *In vivo* Activation of Leukocytes in Whole Blood Cells by Exposure to Free or Encapsulated Oligonucleotides

In order to demonstrate the effectiveness of an in vitro assay for predicting immune stimulation in vivo a comparison of CD69 expression is shown in Figures 1 and 2. CD69 is a cell activation marker, which quantifies the activation of NK cells, B cells and monocytes. Expression of CD69 on NK cells indicates cell activation and production of IFN-g, which is important to inducing a Th-1 immune response. Free and encapsulated ODN1 and 2 were tested in vitro and in vivo for their ability to induce CD69 expression. A dose of 0.1 mg/ml of ODN2 and 10mg/ml ODN1 were used in vitro and 10mg/kg of ODN2 and 20mg/kg of ODN1 in vivo. Each oligonucleotide was encapsulated in a lipid particle composed of POPC:CHOL:DODMA:PEGDMG in a ratio of 20:45:25:10.

Figure 1 illustrates the *in vitro* stimulation of leukocytes bearing the activation marker CD69 from treating mouse whole blood with free oligonucleotides and encapsulated oligonucleoties, specifically ODN1 and 2. When mouse whole blood was treated *in vitro* with free oligonucleotides there was a dose responsive increase in the amount of CD69 positive B-cells, monocytes and to some extent, NK cells, according to the amount of free oligonucleotide used 15 hours after treatment. In this *in vitro* assay, free ODN2 caused much greater stimulation of CD69 than free ODN1. However, when these same oligonucleotides were encapsulated in a lipid vesicle, the *in vitro* stimulation of CD69 production on these same cell types was reduced or abolished altogether.

When the same oligonucleotides were tested *in vivo,* however, suprising results were obtained. Figure 2 illustrates that *in vivo* treatment of ICR mice by injection with encapsulated or free oligonucleotides produces results that are contrary to those obtained *in vitro.* This figure clearly demonstrates that *in vivo,* the lipid encapsulated oligonucleotides were more effective than the free oligonucleotides in stimulating the CD69 marker on the same cell types at 16 and 24 hours after injection. The results show that *in vitro* data is not sufficient for determining whether an oligonucleotide will be immunostimulatory *in vivo.* Moreover, Figures 1 and 2 suggest that lipid encapsulation is an important factor in determining whether an oligonucleotide would be effective *in vivo.* The *in vivo* results show that encapsulated ODN1 and ODN2 were both able to stimulate production of CD69 on NK cells, whereas the *in vitro* results indicated lipid encapsulation of ODN1 and 2 actually reduced the stimulation of CD69 on NK cells below the control level.

The foregoing *in vivo* results show that free oligonucleotides are not necessarily immunostimulatory unless they are encapsulated in a lipid vesicle as measured by stimulation of CD69 bearing cells *in vivo.* This is true even though stimulation of CD69 was observed *in vitro* by free ODN. Further, the results indicate that an encapsulated oligonucleotide may be effective *in vivo* even though it is not shown to be effective *in vitro.*

## EXAMPLE 2

### *In vivo* Dendritic Cell Activation with Methylated Oligonucleotides

As discussed in the Background section above, the prior art teaches that methylated CpG oligonucleotides are generally not effective, or less effective in comparison to unmethylated CpG oligonucleotides in stimulating immune responses whether measured *in vitro* or *in vivo.* United States Patent No. 6,429,199 discloses that methylated oligonucleotides did not enhance the expression of CD40 on NK cells or human B cells, nor did they show any improved survival of dendritic cells, which are the major antigen presenting cells involved in humoral and cellular immunity in a Th-1 response. Further, the methylated CpG oligonucleotides disclosed were inactive in improving survival, differentiation, activation or maturation of dendritic cells *in vitro.* Similarly, the *in vitro* PBMC results disclosed in WO 02/069369 did not demonstrate any activity of methylated oligonucleotides on dendritic cells.

In contrast, the present invention shows that methylated oligonucleotides are at least as effective and typically more effective at inducing proliferation of dendritic cells than unmethylated oligonucleotides. The counterpart of unmethylated ODN1 was made where the cytosine residue of the single CpG dinucleotide sequence was methylated and is referred to as ODN1m.

In order to demonstrate that methylated oligonucleotides are capable of stimulating dendritic cells each of ODN1 and ODN1m were encapsulated in a lipid vesicle comprising POPC:CHOL:DODMA:PEGDMG in a ratio of 20: 45:25:10. PBS was used as a control. The results of this experiment are show in Figure 3.

Figure 3 clearly illustrates the ability of encapsulated ODN1m to activate dendritic cells. Furthermore, when encapsulated in a lipid vesicle, methylated ODN1m was more active than its unmethylated counterpart ODN1 in stimulating activation of dendritic cells *in vivo.* Dendritic cell activation was measured by the percentage of IFN-γ secreting cells. These cells were labeled with an antibody to indicate the cell type and only those having the dendritic cell marker were included in this measurement.

In order to demonstrate the expansion of dendritic cells resulting from the administration of lipid-encapsulated unmethylated and methylated CpG oligonucleotides, cells from the blood, spleen and lymph nodes were analyzed for activation and expansion of dendritic cell populations. ICR mice were immunized with a single intravenous injection of encapsulated oligonucleotides at a dose of 20 mg/kg and the control ICR mouse was injected with PBS. Cells for each of the spleen, blood and lymph nodes were isolated at various time points, as shown in Figures 4 and 5, and the amount of dendritic cell expansion and activation was measured through the use of dendritic cell markers CD11c and DEC205. Each of these markers are specific to dendritic cells though they may represent different cell sub-populations. In each of Figures 4 and 5 the control was plotted as equivalent to 100% and the effect of ODN1m for each backbone configuration was plotted as a percentage of that control. Figure 4A illustrates in each of the panels that the methylated ODN1m stimulated the expansion of CD11c positive dendritic cells in spleen cells and whole blood cells but not in lymphoid tissue as measured against the control. Both the PO and PS backbones for ODN1m showed dendritic cell expansion. Figure 4B shows in each of the panels that methylated ODN1m also stimulated the expansion of DEC205 positive dendritic cells in spleen cells and lymphoid tissue but not in whole blood cells as measured against the control.

Figure 5 also demonstrates the activation of dendritic cells. On collection of the samples the cells were first analyzed by flow cytometry for the co-expression of CD86, which indicates cell activation, and the dendritic cell phenotype markers CD11c and DEC205. The percentage of activated dendritic cells was plotted against a PBS control equivalent to 100%. Figure 5, each of the panels show that the methylated ODN1m induced CD86 expression on CD11c positive dendritic cells when the oligonucleotide was lipid encapsulated. Similar results are shown when measuring DEC205 positive dendritic cells in Figure 5, each of the panels.

The data in Figures 4 and 5 therefore refutes the statements in United States Patent No. 6,429,199 which teaches that methylated CpG oligonucleotides are inactive in improving survival, differentiation, *activation* or maturation of dendritic cells.

## EXAMPLE 3

### The Effect of PS and PO Backbone Configurations on Plasma Cytokine Levels

As noted above, with non-lipid encapsulated oligonucleotides the backbone is traditionally modified so as to reduce molecular degradation by nucleases. However, on encapsulation, such a modification is no longer required to prevent degradation. To establish the effect of a different backbone on immune stimulation the cytokine stimulation induced by lipid encapsulation, mice were injected with oligonucleotides having both PO and PS backbones, and cytokine stimulation was measured over a series of points in time (as shown in Figure 6 and 7B) and over a sliding dosage scale as shown in Figure 7A. In this experiment ICR mice were injected i.v. with a 20mg ODN/kg dose of free PO ODN1, encapsulated PO-ODN1 and encapsulated PS-ODN1. Cytokines common to both Th1 and Th2 (IL-12, IL-6 and IFN-γ) and MCP-1 (a macrophage chemokine) were measured over a 24-hour time course following administration. Cytokine stimulation is generally indicative of a cellular immune response as is a chemokine response in MCP-1. Oligonucleotides were encapsulated in a lipid particle composed of DSPC:Chol:DODAP:PEG-CER14 in a ratio of 20:45:25:10.

Figure 6 shows that *in vivo* administration of free PO-ODN1 had no affect on stimulation of IL-6, IL-12 IFN-γ or MCP-1 indicating that the oligonucleotide was likely degraded by nucleases. It is well known in the art that a PS backbone is required when administering free oligonucleotide in order to avoid nuclease degradation. In contrast, *in vivo* administration of lipid encapsulated PO and PS ODN1 stimulated production of each of these cytokines and chemokine. However, Figure 6 indicates that the PO-ODN is more effective at inducing cytokine and chemokine production.

Figure 7A illustrates increased IL-12 induction by treatment of ICR mice with either encapsulated PO or PS ODN14 in comparison to free ODN14 measured over a sliding dosage scale. This figure supports the conclusions drawn from Figure 6 indicating that lipid encapsulation increases the effectiveness of cytokine stimulation evidenced by an increase In IL-12 induction and that a PO-ODN is more effective at inducing a cytokine response than a PS-ODN. Figure 7A also demonstrates that a lower dose of oligonucleotides is required to facilitate a cytokine response when encapsulated in comparison with free olignonucleotldes administered in the absence of the lipid particles.

In order to further elucidate the difference in PO-ODN and PS-ODN Figure 7B illustrates differences in IFN-γ cytokine stimulation over time specific to PS and PO backbone configurations. Treatment with encapsulated PO ODN14 stimulates a strong early induction of IFN-γamma while treatment with encapsulated PS ODN14 stimulates a smaller but still effective induction of IFN-γ. Moreover, Figure 7B shows that over a period of days, a second large IFN-γ peak occurred when stimulating with the PS-ODN which may indicate that the immune system was primed by treatment with the encapsulated PS-ODN to respond more effectively to IL-12 production, possibly through expansion of NK cells after treatment.

Figure 22 similarly demonstrates the late IFN-γ peak seen in Figure 7B for the PS-ODN in comparison with the PO version of the same oligonucleotides, as discussed further in Example 4.

An important feature of lipid encapsulation according to the present invention is the finding that oligonucleotides having natural PO backbones can be used to stimulate an immune response whereas in the prior art, PS backbones are required for effective *in vivo* activity. As shown in Example 7 below, encapsulated PO oligonucleotides may be more effective than PS oligonucleotides when evaluating anti-tumor efficacy, especially where the oligonucleotide is methylated.

## EXAMPLE 4

### Evaluation of immunostimulatory properties of CpG oligonucleotides having PS and PO backbones

When differentiating the levels of response particularly associated with ODN's having PO and PS backbones, a further aspect is the analysis of the type of response being evaluated, more specifically, whether the response is a humoral response or a cellular response. In order to assess the effect of the backbones, an experiment was conducted to look at the ability of PS-ODN and PO-ODN to initiate and induce maturation (i.e. facilitate isotype switching) of a humoral immune response. The magnitude and kinetics of a humoral immune response elicited by administration of encapsulated PO-ODN and PS-ODN was compared. Each of the PO-ODN and PS-ODN were administered subcutaneously at a dose of 100 μg/dose in a q14 x 2 prime-boost setting on Days 0 and 14 and assessed at 6 weeks on Day 35. The control mice were immunized at the same dose using OVA-PBS and OVA-Alum. Oligonucleotides were encapsulated in a lipid particle composed of DSPC:Chol:DODMA:PEG-DMG in a ratio of 20:45:25:10.

It can be concluded that although both PO and PS ODNs are able to induce a humoral immune response, the nature of the response is different. Figures 8 and 9 illustrate the magnitude of the IgM and IgG response after 6 weeks for the various oligonucleotides and control in terms of absorbance. As is clearly demonstrated in the Figure, each of PS-ODN1 and PS-ODN2 produced a weak IgM response whereas PO-ODN2 produced a strong IgM response. Conversely, the IgG response produced was consistently better for each of the PS-ODN tested in comparison with the same oligonucleotides having a PO backbone. This suggests that a PS-ODN produces a superior IgG response. These data indicate that while both PO and PS ODN are able to initiate a humoral immune response, the PO response does not mature as indicated by a lack of isotype switching and a preponderance of the IgM isotype. On the other hand, PS-ODN are able to initiate a humoral immune response as well as induce maturation of the response as indicated by isotype switching to a dominance of IgG isotype antibodies.

This phenomenon may be related to the cytokine profiles induced by PO vs. PS ODN. Figures 7B and 22 illustrates that encapsulated PS oligonucleotides ODN1 and ODN2 produced a strong IFN-y peak 6 days after treatment that is not produced by encapsulated PO oligonucleotides. It has been reported that cytokines such as IFN-γ result in preferential isotype switching to various IgG isotypes. Therefore, the large PS-ODN-induced late IFN-γ peak may induce isotype switching from IgM to IgG isotypes while the lack of such a peak in PO-ODN-treated mice may result in no isotype switching. The basis for this reduced late IFN-γ peak with PO-ODN is not clear, but results may suggest that treatment with encapsulated PO oligonucleotides but not PS oligonucleotides causes a prior induction of type I interferons that inhibit the expression of IL-12, which is needed to promote IFN-γ expression in NK or T cells.

Similarly, Figure 6 not only shows that encapsulation of the oligonucleotides is important for stimulating the production of cytokines that lead to a Th-1 response as previously discussed, but also shows that more cytokines are produced using encapsulated PO oligonucleotides than PS oligonucleotides. This contrasts with administration of free oligonucleotides as taught in the prior art, which generally shows that a PS backbone is preferred over PO oligonucleotides to prevent degradation of the oligonucleotide *in vivo.*

## EXAMPLE 5

### *In vivo* Immunological Responses to Treatment with Oligonucleotides as measured by Cytokine Induction, Tetramer Analysis and Cytotoxicity Assay (CTL)

To monitor immunological response to subcutaneous immunization, antigen specific cellular immune responses

were monitored using MHC Class I-tetramer analyses, cytotoxicity assays and cytokine release assays while humoral immune responses were monitored by measuring plasma antibody levels. Cellular and humoral responses were assessed in C57Bl/6 and Balb/C mice respectively (5 animals per group). For analysis of the cellular response, mice were immunized subcutaneously with 3 injections on a q7d x 3 dosing regimen on Days 0, 7 and 14 at a dose of 100 μg oligonucleotide in combination with 20μg of antigen. The spleen, liver, lymph node and blood tissues were collected on Day 21. Solid tissues were mechanically dissociated and cells were processed to collect mononuclear cells. For analysis of the humoral response, animals were immunized twice on a q14d x 2 on Days 0 and 14 and blood was collected on Day 35 for analysis of plasma for immunoglobulin levels. In this series of experiments oligonucleotides were encapsulated either in lipid particles composed of DSPC:Chol:DODMA:PEG-DMG or POPC:Chol:DODMA:PEG-DMG at a ratio of 20:45:25:10. All comparisons were done with like lipid particles.

MHC-tetramer analysis is designed to detect CD8+ve, cytotoxic T-lymphocytes that possess the appropriate T-cell receptor to allow recognition and lysis of target cells bearing the target antigen in the context of a MHC Class I complex. Isolated splenocytes from immunized animals were stained with PE-labeled MHC Class I tetramers ($H_2K_b$) complexed with the immunodominant OVA SIINFEKL peptide as well as FITC-labeled anti-CD8 and Cy-Chrome-labeled anti-TCR antibodies and subjected to flow cytometric analysis. CD8 +ve, TCR+ve T-lymphocytes were assessed for the number of cells possessing T-cell receptors capable of specifically recognizing and binding to OVA in the context of MHC Class I molecules.

For the cytotoxicity assay, the ability of splenocytes from immunized animals to specifically recognize and lyse target cells in an antigen specific manner was assessed using a 4 hour $^{51}$Chromium-release assay. Target cells were labeled with $^{51}$Chromium and the amount of cytotoxicity was determined by the amount of radionuclide released into the supernatant from targets lysed by immune effector cells. Isolated splenocytes from immunized animals were tested immediately or after 5 days of *in vitro* restimulation with OVA-pulsed, syngeneic antigen presenting cells, for their ability to specifically lyse EG.7, OVA expressing target cells compared to EL4, non OVA-expressing cells.

The aim of the cytokine release assay is to detect antigen-specific immune effector cells that are activated to produce and secrete cytokines, specifically IFN-γ, in response to stimulation with a specific antigen. Cells were isolated from the spleen, liver, blood and lymph nodes of immunized animals and analyzed using the Cytokine Secretion Assay (Miitenyi Biotec). Cells were stimulated overnight with OVA-pulsed, autologous antigen presenting cells and labeled with a catch reagent (a bispecific antibody recognizing the CD45 epitope on the surface of immune cells and IFN-γ). Any cells capable of recognizing and responding to the antigen stimulation, synthesized and secreted cytokines which were then captured by the cell-bound catch reagent, resulting in IFN-γ bound markers on their surface. Cells were then labeled with fluorescently labeled antibodies against IFN-γ and various phenotype markers and analyzed by flow cytometry to allow detection of specific cell types that were activated to secrete IFN-γ.

Analysis of humoral response was designed to determine the level of antigen-specific IgG in the plasma of immunized mice. Blood was collected by cardiac puncture and centrifuged to collect plasma. Antigen specific immunoglobulin production was measured using the End-point dilution ELISA method to measure titers of total IgM, IgG and the IgG1, IgG2a, subclasses. Samples of pooled plasma were serially diluted and plated into OVA coated plates to capture OVA specific antibodies in the diluted samples. OVA specific antibodies were then detected with horseradish peroxidase-conjugated rabbit anti-mouse IgM, IgG, IgG1, or IgG2a antibodies and TMB substrate. The absorbance of the colorimetric reaction was measured at 450nm on ELISA plate reader and end-point dilution titers were defined as highest dilution of plasma that resulted in absorbance value two times greater then that of naive animals, with a cut-off value of 0.05. This was used to evaluate seroconversion and magnitude of response as well as to evaluate the Th type of response.

Each of Figures 10 and 11 illustrate the normalization of ODN1m to that of its unmethylated counterpart ODN1. Each of the bars on these figures represents a direct comparison of one animal group (5 animals per group) treated with a methylated ODN and a second group treated with the unmethylated counterpart wherein each oligonucleotide is lipid encapsulated in identical lipid particles. The results for the unmethylated population were set equivalent to 100% for each group and the methylated group was measured against this 100% standard. On bars showing an equivalence to 200%, this was the cut off value and in actuality the 200% line represents a value of 200% or greater.

Figure 10 shows the results of the cytokine release assay described above. This figure illustrates that over a series of screenings, although both the methylated and unmethylated lipid encapsulated oligonucleotides each exhibited an immune response, on comparison of the methylated ODN to the unmethylated ODN, the methylated oligonucleotide was as good as, and often better than, the unmethylated ODN in stimulating proliferation of dendritic cells, NK cells, and $CD8^+$ T-cells as indicated by cytokine secretion in Figure 10A, B, and C respectively.

The results of the tetramer and CTL analyses are shown in Figures 11 A - C. These figures again illustrates the ability of both methylated and unmethylated ODN to stimulate an immune response. However, Figures 11A and B further demonstrate that over a series of screenings of animals treated with methylated or unmethylated encapsulated ODN, in each of the tetramer and CTL analyses, the methylated oligonucleotides were consistently better in stimulating proliferation of cytotoxic T lymphocytes and tetrameric lymphocytes cells than the unmethylated ODN. In addition, Figure

11C illustrates data from a representative tetramer study, wherein overall averages are shown in Figure 11B. Each of ODN5, ODN5m, ODN7 and ODN7m were tested as per the protocol described above. It is clearly shown in Figure 11C that lipid encapsulated ODN5m and ODN7m induce a higher number of antigen specific CD8 T-cells on comparison to their lipid encapsulated unmethylated counterparts.

From each of Figures 10 and 11 it is shown that immune stimulation resulting from immunization with methylated ODN1m is consistently at least equivalent to, and often better than, the same treatment with its unmethylated oligonucleotide counterpart. This is further demonstrated in the following example.

## EXAMPLE 6

### Anti-tumor Efficacy Comparison of Methylated and Unmethylated Oligonucleotides In an EG7-OVA Tumor Model

As mentioned elsewhere herein, one aspect of the present invention includes use of lipid-methylated nucleic acid formulations in conjunction with an associated antigen to stimulate an immune response to the antigen *in vivo*.

Anti-tumor efficacy induced by subcutaneous immunization was assessed in C57Bl/6 (5 animals per group) in a prophylactic immunization model. Mice were immunized subcutaneously with 3 injections on a q7d x 3 dosing regimen on Days 0, 7 and 14 at a dose of 100μg oligonucleotide and 20μg of OVA antigen dose. Animals were then challenged with a subcutaneous injection of 2.5 x $10^6$ EG.7 Ova expressing tumor cells on Day 21. Mice were monitored 3 times weekly to assess tumor growth and weight gain. Control mice were injected with one of PBS or HBS and 20μg of OVA antigen on the same schedule described above. Oligonucleotides were encapsulated in a lipid particle having a lipid composition of one of POPC: CHOL: DODAP:PEGCer14 or DSPC: CHOL: DODAP:PEGCer14 each in a ratio of 25: 45:20:10. All comparisons of methylated and unmethylated oligonucleotides were done using like lipid particles. Results from these efficacy experiments are detailed in Figures 12 - 15, 18 - 21, and 25 - 28. Day 0 on each of the Figures is the day each animal was challenged with the tumor.

Figure 12 illustrates the efficacy trend when animals are immunized with free ODN. The results shown are consistent with the prior art, namely that when an animal is administered free oligonucleotides, the methylated oligonucleotides have less therapeutic efficacy than the unmethylated oligonucleotides in reducing tumor growth. Specifically, free unmethylated ODN1 and ODN2, having PS backbones so as to avoid nuclease degradation, showed a greater reduction in tumor growth than their methylated counterparts, ODN1m and ODN2m. This was most especially true about 25 days after inoculation with the tumor when the tumor growth rate of the methylated oligonucleotides approached the rate of the control animal treated only with a PBS buffer.

Figures 13-15 illustrate that encapsulation of oligonucleotides provides equivalent or better therapeutic efficacy of methylated over unmethylated oligonucleotides particularly when the oligonucleotides contain a natural phosphodiester (PO) backbone. Figure 13 shows that after implantation with a tumor, treatment with the methylated encapsulated ODN1m having a PS backbone was equal in therapeutic efficacy in comparison to the unmethylated ODN1. In contrast, Figure 14 shows the effect with the corresponding encapsulated methylated ODN1 m and unmethylated ODN1 oligonucleotides having a PO backbone, where therapeutic efficacy was greatest with the methylated version 32 days after transplantation while the unmethylated version lost its efficacy. Figure 15 shows that unmethylated ODN2 and its methylated counterpart ODN2m had virtually identical efficacy in reducing tumor growth. Accordingly, in certain embodiments the methylated oligonucleotide is at least as efficacious as an unmethylated counterpart when configured with a PS backbone.

Each of Figures 18, 19, 20 and 21 further elaborate on the above efficacy data. Figure 18 shows that lipid encapsulation of methylated PS-ODN1 m provided a therapeutic benefit that was more effective than encapsulation of the PS-ODN1 in reducing tumor growth over a prolonged period of time. This effectiveness was further borne out by the superior survival rates of mice treated with encapsulated PS-ODN1 m in comparison to treatment with the PS-ODN1 in two different studies depicted in Figure 19. Figure 19A illustrates the percentage of animals that are tumor free at a series of time points and 19B, the number of animals remaining in the study at these same time points. As is clearly shown in Figure 19B, the number of animals remaining in the study treated with ODN1 and ODN1m was essentially identical throughout the study. However, Figure 19A clearly illustrates a greater percentage of tumor free animals when treated with the methylated ODN1m compared to those treated with unmethylated ODN1.

Similarly, Figure 20 illustrates the tumor volume in mice treated with the two oligonucleotides over time and Figure 21 the percentage of animals surviving over time. Figure 20 shows improved efficacy when animals were treated with the encapsulated methylated ODN1m in comparison to the encapsulated unmethylated counterpart, ODN1. Correspondingly, Figure 21 shows an increase in the survival rate of mice treated with the methylated ODN1m relative to treatment with unmethylated ODN1.

A further study efficacy study was conducted on the same tumor model using a different immunization protocol. In this study anti-tumor efficacy induced by subcutaneous immunization was assessed in C57Bl/6 (5 animals per group)

in a prophylactic immunization model. Mice were immunized subcutaneously with 2 injections on a q7d x 2 dosing regimen on Days 0 and 7 at a dose of 100μg oligonucleotide and 20μg of antigen. Animals were then challenged with a subcutaneous injection of 5 x $10^5$ EG.7 Ova expressing tumor cells on Day 21. Mice were monitored 3 times weekly to assess tumor growth and weight gain. Control mice were injected with PBS on the same schedule described above. Oligonucleotides in Figure 24(b) were encapsulated in a lipid particle having a lipid composition of DSPC: CHOL: DODAP: PEGCer14 each in a ratio of 25:45:20:10. All comparisons of methylated and unmethylated oligos were done using like lipid particles. Results from these efficacy experiments are detailed in Figure 24. Day 0 on the Figure is the day each animal was challenged with the tumor.

Figure 24 illustrates an example of treating the experimental tumor E-G7 using the lipid encapsulated PS-ODN1, PS-ODN2, each unmethylated, PS-ODN1m. methylated, in conjunction with an E-G7 OVA tumor antigen, which in this case was associated with the lipid particle by being attached to the surface thereof. Figure 24A shows that when the oligonucleotides were administered in the absence of the immunostimulatory lipid particle, the methylated PS-ODN1m had little effect on tumor growth. The corresponding unmethylated oligonucleotide PS-ODN1 was effective in reducing tumor volume while the unmethylated oligonucleotide PS-ODN2 was partially effective. Figure 24B shows that not only did encapsulation of the oligonucleotides in the lipid particle increase the effectiveness of the unmethylated PS-ODN2 to a level similar to ODN1, but also that the encapsulated methylated oligonucleotide PS-ODN1m was more effective than either of the encapsulated unmethylated oligonucleotides.

Figures 25-28 further illustrate that encapsulation of oligonucleotides provides equivalent or greater therapeutic efficacy for encapsulated methylated over unmethylated oligonucleotides. Figure 25 illustrates that lipid encapsulation of methylated PS-ODN5m provided a more effective therapeutic benefit than encapsulation of the equivalent unmethylated PS-ODN5 in reducing tumor growth over time. The effectiveness was further borne out by the superior survival rate of mice treated with encapsulated methylated PS-ODN5m in comparison to treatment with the unmethylated PS-ODN5 as shown in Figure 26. Figure 27 illustrates that while free unmethylated PS-ODN7 provides some anti-tumor benefit, free unmethylated PS-ODN 7 and PO-ODN7 as well as free methylated PS-ODN7 and PO-ODN7 were relatively ineffective in reducing tumor growth. However, lipid encapsulation of methylated PO-ODN7m provided effective therapeutic benefit in reducing tumor growth. Similarly, these trends were also illustrated in Figure 27 in the survival rate of mice treated with these same ODN.

## EXAMPLE 7

### Anti-tumor Efficacy Comparison of Methylated and Urimethylated Oligonucleotides In an B-16 Melanoma Tumor Model

Anti-tumor efficacy induced by intravenous tail immunization was assessed in C57Bl/6 (8 animals per group) in a therapeutic immunization model. Animals were challenged with a subcutaneous injection of 3.0 x $10^5$ EG.7 B16/BL6 murine melanoma expressing tumor cells on Day-0. Mice were then treated intravenously every other day starting on day 4 for 14 days at a dose of 20mg/kg ODN. Mice were monitored every other day to assess tumor growth and weight gain. Control mice were injected with HBS on the same schedule described above. Oligonucleotides were encapsulated in a lipid particle having a lipid composition of DSPC: CHOL: DODAP:PEGCer14 each in a ratio of 25:45:20:10. Results from this efficacy experiments are detailed In Figures 16 and 17. Day 0 on each of the Figures is the day each animal was challenged with the tumor.

Figure 16 illustrates therapeutic efficacy of administering the methylated PS-ODN1 m to an animal inoculated with a B16 melanoma tumor in comparison to its unmethylated counterpart PS-ODN1. Encapsulation of PS-ODN1 m in a lipid particle Increased its efficacy in reducing tumor volume to at least that of the encapsulated unmethylated PS-ODN1.

Figure 17 illustrates the average weight of the tumors in each mouse on Day 22. The average tumor size in mice treated with free methylated PS-ODN1 m was nearly the same as in mice treated with a buffer control, while mice treated with free unmethylated PS-ODN1 showed reduced tumor growth. In contrast, when mice were treated with the methylated PS-ODN1 encapsulated in a lipid particle, the amount of tumor reduction was near equivalent to that obtained with the lipid encapsulated unmethylated PS-ODN1. Accordingly, lipid encapsulation of methylated oligonucleotides can yield efficacy in treating a tumor *In vivo* even though the free methylated oligonucleotide has little or no efficacy.

## EXAMPLE 8

### Blood Clearance Levels when Treated with Encapsulated Oligonucleotides

An important aspect in effective immune stimulation is the ability of the immune system to raise an antibody response against specific antigens. One of the first demonstrations of the capacity of antigen associated with lipid

encapsulated oligonucleotides to initiate such a response is illustrated by the data shown in Figure 23.

Each of ODN1 and ODN1m were encapsulated in two different lipid particles; Lipid one (L1) being a DSPC: CHOL:DODAP:PEGCer20 and the Upid 2 (L2) being the same but having a PEGCer14 in the place of the PEGCer20. The half-life of the PEGCer 20 within the liposome is known to be much longer than that of the PEGCer14 and thus the PEGCer20 remains with the lipid particle for a longer time period. Mice were given a series of 4 i.v. tail injections, starting on Day 0, and were dosed once a week for 3 weeks. Blood was collected 1-hour post injection each week and were analyzed for the presence of the encapsulated ODN.

Figure 23 illustrates the effect on clearance from the blood in mice for the different lipid compositions, L1 and L2 each with different PEG-ceramide steric coatings (PEG-ceramide-C-20 and PEG-ceramide C-14 respectively) in combination with either methylated or unmethylated oligonucleotides, ODN1m and ODN1 respectively. After injection 1 the results show extended circulation/slow clearance for both of the encapsulated ODNs from the blood sample regardless of composition. However, for each of injections 2, 3 and 4 the results show that L1 liposomes (L1-ODN1 and L1-ODN1m) containing the long-lived PEGCer20 had shorter circulation/rapid clearance while L2-ODN1 and L2-ODN1 m containing the short-lived PEG-ceramide C-14 had longer circulation/slower clearance than those encapsulated with lipid particles containing PEGCer20 lipid.

The data depicted herein demonstrates two specific points: (1) the induction of antigen specific antibodies; and (2) the relative immunostimulatory capacity of unmethylated and methylated oligonucleotide. In terms of induction of antigen specific antibodies, the initial injection resulted in the induction of antibodies directed against the PEG moiety of the PEG-ceraminde steric barrier lipid. The presence of these antibodies in the plasma of injected animals resulted in the opsonization and subsequent rapid clearance from the circulation of liposomes containing PEG after injections 2, 3 and 4 as seen with L1 liposomes with PEGCer20. However, animals injected with liposomes without PEG, such as L2 liposomes with PEGCer14 from which the PEG dissociated very rapidly in circulation, were not oposonized and thus had relatively extended circulation times. In terms of the relative immunostimulatory potency of unmethylated vs. methylated oligonucleotide, the clearance of the liposomes containing either the unmethylated oligonucleotide or the corresponding methylated form were cleared at similar rates, thus indicating that both are able to induce antigen specific antibodies.

## EXAMPLE 9

### HBsAg In combination with LNA and Adjuvants

The following data demonstrate immunological efficacy of a vaccine comprised of the combination of commercial Hepatitis B vaccines (Recombivax-HB, Merck; and Engerix B, Glaxo Smith-Kline) and LNA/ODN 1. The vaccine demonstrated significant enhancement in immunogenicity and yielded higher levels of HBsAg specific antibodies compared to the commercial vaccine alone. Efficacy was noticeable after a single immunization and resulted in an antibody titer similar to that of animals immunized with Recombivax-HB or Engerix-B by prime-boost immunization regimen. The Th type of immune response was assessed based on ratio of Hepatitis B specific IgG2a/IgG1 antibodies. The vaccine is shown to produce a Th1 biased immune response, often indicative of cell-mediated immune responses. Moreover, LNA/ODN 1 was well tolerated. No signs of local inflammation or necrosis upon IM administration of LNA/ODN 1 was observed. In comparison, alum administration, in some instances, led to mild local inflammatory responses.

**Materials.** Commercial Hepatitis B vaccines Recombivax-HB (Merck) and Engerix-B (Glaxo Smith-Kline) were obtained in reconstituted form. The HBsAg concentrations in the commercial vaccines are as follows: 10 μg/ml in Recombivax-HB and 20 μg/ml in Engerix-B. Both commercial vaccines were in a 1ml volume with the adjuvant Alum at a concentration of 0.5 mg/ml.

Table 4 Indicates clinical and experimental composition and dosage for commercial Hepatitis B vaccines Recombivax-HB (Merck) and Engerix-B (Glaxo Smith-Kline) used in these studies.

Table 2

| Vaccine name | Dose in commercial vaccine (1ml dose) | | Conc. in expt'l vaccine (50 ☐l dose) | |
|---|---|---|---|---|
| | HBsAg (μg) | Alum (mg) | HBsAg (μg) | Alum (μg) |
| Recombivax HB (Merck) | 10 | 0.5 | 0.5 | 25 |
| Engerix (GSK) | 20 | 0.5 | 0.5 | 25 |

Recombinant Hepatitis B surface antigen was obtained from Aldevron. It was produced in yeast Saccharomyces cerevisae, containing the expression plasmid pCGA7, subtype "ayw", at a concentration of 0.6mg/ml in 0.05M phosphate, 0.2M NaCl buffer pH7.2.

DSPC and DODAP were purchased from Northern Lipids (Vancouver, BC) and Avanti Polar Lipids (Alabaster, AL), respectively, while PEG-Cer$C_{14}$ was synthesized by Dr. Zhao Wang (INEX Pharmaceuticals Corporation). Cholesterol was obtained from Sigma (St. Louis, MO). The ODNs, ODN 1,a 16-mer c-myc ODN complimentary to the initiation codon region of the human/mouse c-myc proto-oncogene mRNA and CpG ODN 2 were obtained from INEX's general supplies. The polyclonal anti-IgG1, IgG2a and IgG (H&L) antibodies used were purchased from Zymed, (San Francisco, CA). Substrate for HRP ,3,3', 5,5'-tetramethlbenzidine (TMB) liquid substrate system purchased from Sigma.

Mice. Female, BALB/c (6 - 8 weeks) were purchased from Harlan-Sprague Dawley (Indianapolis, IN). All animals were quarantined for 2 weeks prior to use. All studies were conducted in accordance with the guidelines established by the Canadian Council on Animal Care (CCAC) and the Institutional Animal Care and User Committee (IACUC).

**LNA/ODN 1 preparation.** LNA composed of /DSPC/cholesterol/ DODMA /PEG-CerC14 (:20:45: 25:10, molar ratio) and encapsulated ODN were prepared as previously described (Semple et al., 2000). LNA formulations containing ODN 1 had a final ODN to lipid ratio which was determined to be 0.14 (wt/wt). Lipid content was calculated based on a Bligh & Dyer extraction of the lipids followed by a phosphate assay for DSPC content while ODN was quantified by A260 using extinction coefficients of 31.7 g/ml for ODN 1. The preparations were filtered through a 0.22μm filter before use.

**Immunization.** In prime-boost immunization model HBsAg protein was administered intramuscularly together with either free CpG ODN or with LNA/CpG ODN (investigational vaccine). Accordingly to the requirements of the Center for Biologics and Research (CBER) for evaluation of adjuvant, these studies included a control groups that received the antigen alone or the antigen adjuvanted with an aluminum compound to provide evidence that the investigational adjuvant augments the immune response to the antigen. Antigen-specific immunostimulatory properties of LNA/CpG ODN adjuvants were assessed based on specific immunoglobulin production and evaluation of Th type of immune response compared with immunostimulation induced with Alum (conventional vaccine).

For recombinant protein, each immunization dose consisted of 2 μg of purified HBsAg protein with or without 100 μg of free or encapsulated ODN and/or 25 μg $Al^{3+}$ (Alum). For the commercial HB vaccine, 0.5 μg of antigen with 25 μg of Alum was administered with or without 100 μg of free or encapsulated ODN. Immunizations were administered in a prime-boost regimen on a q14d x 2 schedule with the exception of a group receiving only a single IM injection. The study conditions are set forth below in Table 5. The Th type of immune response was effected by particular type of adjuvant. Co-administration of HBsAg with Alum resulted in Th2 type of immune response with prevalence of IgG1 antibodies. Addition of free or encapsulated CpG ODNs shifted the immune response towards a Th1 type with synthesis of IgG2a antibody.

Antibody titres were determined 6 weeks post-prime (4 weeks post-boost in those animals receiving the prime-boost, q14d x 2, dosing regimen).

**Table 3**

| Cage | # of Mice | Treatment | Injection Volume (μl) | Injection Type (μg) | Dosing Schedule | Ag Dose (μg) | ODN Dose (μg) | Alum Dose (μg) |
|---|---|---|---|---|---|---|---|---|
| 1 | 5 | PBS | 50 | IM | q14dx2 | -- | -- | -- |
| 2 | 5 | HBsAg alone | 50 | IM | q14dx2 | 2 | -- | -- |
| 3 | 5 | Free PS-ODN 2 + HB | 50 | IM | q14dx2 | 2 | 100 | -- |
| 4 | 5 | Free PS-ODN 1 + HB | 50 | IM | q14dx2 | 2 | 100 | -- |
| 5 | 5 | + HB Encaps. PS-ODN-1 | 50 | IM | q14dx2 | 2 | 100 | -- |
| 6 | 5 | Alum | 50 | IM | q14dx2 | -- | -- | 25 |
| 7 | 5 | Free ODN-2 PS + Alum + HBsAg | 50 | IM | q14dx2 | 2 | 100 | 25 |

(continued)

| Cage | # of Mice | Treatment | Injection Volume (μl) | Injection Type (μg) | Dosing Schedule | Ag Dose (μg) | ODN Dose (μg) | Alum Dose (μg) |
|---|---|---|---|---|---|---|---|---|
| 8 | 5 | Free PS-ODN 1 + Alum + HBsAg | 50 | IM | q14dx2 | 2 | 100 | 25 |
| 9 | 5 | Encaps. PS-ODN 1 + Alum + HBsAg | 50 | IM | q14dx2 | 2 | 100 | 25 |
| 10 | 5 | Recombivax | 100 | IM | q14dx2 | 0.5 | -- | 25 |
| 11 | 5 | Free PS-ODN 1 + Recombivax | 100 | IM | q14dx2 | 0.5 | 100 | 25 |
| 12 | 5 | Encaps. PS-ODN 1+ Recombivax | 100 | IM | q14dx2 | 0.5 | 100 | 25 |
| 13 | 5 | Encaps. PS-ODN 1 + Recombivax | 100 | IM | SINGLE | 0.5 | 100 | 25 |
| 14 | 5 | Engerix | 100 | IM | q14dx2 | 0.5 | -- | 25 |
| 15 | 5 | Free ODN 1 + Engerix | 100 | IM | q14dx2 | 0.5 | 100 | 25 |
| 16 | 5 | Encaps. PS-ODN 1+ Engerix | 100 | IM | q14dx2 | 0.5 | 100 | 25 |
| 17 | 4 | Alum + HBsAg | 50 | IM | q14dx2 | 2 | -- | 25 |

Gross morphology and histopathology of the injection site. Visual grading was applied to determine degree of local tissue reaction upon immunization.

Humoral immunogenicity studies. Negative control serum was obtained by tail nicking. The blood (50 μl), collected using a Gilson pipet, was placed into EDTA tubes and subsequently centrifuged (4˚C, 1,1400 rpm, 30 min) to obtain plasma. Blood was collected again by the tail-nicking process at weeks 4 and 6. Upon termination of mice at week 8, blood was collected by cardiac puncture and processed as above. Plasma was pooled for each group of five mice and assayed in duplicate. All results were represented as a mean value and standard deviation calculated for each group.

Antigen specific immunoglobulin production measured using End-point dilutions ELISA method described previously. This assay was developed to effectively measure the end point titers of IgG class or IgG1, IgG2a, subclasses of HBsAg-specific mouse IgG.

The test offers the ability to capture antibody specifically using a solid-phase coating with HBsAg. Abs specific to HBsAg were detected and quantified by end-point dilution ELISA assay (in duplicate) on samples of plasma pooled together from individual animals Briefly, a solid phase of HBsAg (100 μl of 10 μg/ml solution coated per well of a 96 well flat-bottomed plate, overnight at 4˚C), blocked with 1% BSA-PBS (1h, 37˚C) was used to capture HBsAg specific antibody from the serially diluted (with 1% BSA-PBS) plasma samples (1 h at 37˚C). Bound HBsAg specific antibodies were then detected with horseradish peroxidase (HRP)-conjugated rabbit anti-mouse IgG, IgG1, or IgG2a (1:4000 in PBS-1% BSA: 100 μl/well), followed by incubation with TMB liquid substrate system (100 μl/well, 30 min at room temperature, light protected). Reaction was stopped by addition of 100 μl/well of 2M sulfuric acid and absorbance measured at 450nm on ELISA plate reader. End-point dilution titers were defined as highest dilution of HBsAg-specific in which antibodies were detected in plasma that resulted in an absorbance value (OD450) of two times greater then

that of naïve animal (with cut-off value of 0.05). This method applied for evaluation of seroconversion and magnitude of Ab response as well as for evaluation of Th type of immune response initiated by immunization.

**Seroconversion and Magnitude of Antibody Response 6 weeks postprime.**

Antibody titers were evaluated after immunization with Recombivax-HB with and without LNA containing ODN 1 (Figure 29). Total IgG titers were measured 6 weeks after prime by an end-point dilution ELISA method. Seroconversion was complete in all investigated groups 6 weeks post-prime. Adjuvant activity of LNA/ODN 1 + Alum appeared to be significantly higher than that of Alum alone. Single administration of RecombiVax-HB together with LNA/ODN 1 produced immune response similar to that of prime-boost immunization with Recombivax-HB alone. Co-administration of LNA/ODN 1 with Recombivax-HB (Figure 29) in a prime boost regimen resulted in specific anti hepatitis B antibody titers at least 10 times higher than after immunization with of Recombivax-HB alone in an identical regimen.

To confirm this result, mice were immunized with 2μg/dose of recombinant HBsAg with Alum or LNA/ODN 1. A 10-fold increase in specific IgG titer was observed for LNA/ODN 1 compared to Alum (Figure 30).

These results are supported in Figure 31 which shows that a single immunization of RecombiVax-HB vaccine coadministered with LNA/ODN 1 induced similar anti-HBsAg antibody titers as the Hepatitis B vaccine Engerix-B, another alum-based vaccine, administered in a prime-boost, q14d x 2 dosing regimen. In addition, antibody titers from RecombiVax-HB given in an identical prime-boost regimen is also shown to be equivalent. Plasma obtained 6 weeks following the IM single dose immunization was compared to plasma obtained from mice 6 weeks following the prime (4 weeks following the boost) with either Engerix B or RecombiVax HB. Anti-HBsAg IgG levels were measured by end-point dilution ELISA.

Furthermore, data shown in Figure 32 confirms that coadministration of LNA/ODN 1 with alum-based vaccines such as RecombiVax-HB or Engerix-B results in enhanced antibody titers compared to administration of the vaccine alone. Injection of these vaccines at an antigen dose of 0.5 μg/immunization with 100 μg of oligonucleotide/immunization resulted in at least a 10 fold increase in anti-HBsAg antibody titers compared to vaccine alone (at 0.5 μg HBsAg/dose). Plasma was obtained 6 weeks following the prime (4 weeks following the boost) with either Engerix B or RecombiVax HB. Anti-HBsAg IgG levels were measured by End-point dilution ELISA.

These conclusions, based on studies combining LNA/ODN 1 with alum-based vaccines, were further confirmed in studies with purified, recombinant HBsAg (Figure 33). These data show the synergistic effect of LNA/ODN 1 and Alum adjuvants on HBsAg specific IgG production. The addition of an Alum adjuvant to HBsAg enhances antibody production compared to antigen alone. However, LNA/IODN 1 is a more effective adjuvant, resulting in a 10 fold higher titer and the combination of both Alum and LNA/ODN 1 results in a further increase in antibody titer. Animals were immunized in prime-boost regimen. Plasma was obtained 6 weeks following the prime (4 weeks following the boost) with either Engerix B or RecombiVax HB. Anti-HBsAg IgG levels were measured by End-point dilution ELISA.

It was believed that LNA encapsulation of CpG ODN would improve immunostimulatory properties of CpG ODN as immune adjuvants due to increased in circulation time and/or the bioavailability to immune cells. This was confirmed by data obtained from comparative studies of antigen specific IgG titers induced upon immunization with free ODN 1 compared to LNA/ODN 1 .

Evaluation of the antibody titers obtained by immunization with Recombivax-HB or Engerix- B vaccines alone or in combination with LNA/ODN 1 demonstrate: Single dose immunization with Recombivax-HB vaccine, co-administered with LNA/ODN 1 induced antibody titers as high as a prime-boost immunization with conventional vaccines. Co-administration of LNA/ODN 1 with Recombivax -HB or Engerix-B resulted in specific anti hepatitis B antibody titers being significantly higher than after immunization with Recombivax -HB-HB or Engerix-B alone. LNA/ODN 1 demonstrated adjuvant properties superior to those of Alum in mice immunized with 2μg/dose of HbsAg and LNA/ODN 1 acted synergistically with the Alum.

The Th type of immune response was effected by particular type of adjuvant. Co-administration of HBsAg with Alum resulted in Th2 type of immune response with prevalence of IgG1 antibodies. Addition of free or encapsulated CpG ODNs shifted the immune response towards a Th1 type with predominant preferable synthesis of IgG2a antibody. The type of immune response obtained are shown in Table 7.

Table 4

| Serum Titers | 1 E+02 | 1E+03 | 1 E+04 | 1E+05 | 1E+06 | 1E+07 | THtype |
|---|---|---|---|---|---|---|---|
| Alum+ HBsAg 2ug IgG1 | 3.536 | 3.535 | 3.399 | 2.484 | 1.770 | 1.557 | TH2 |
| Alum+ HbsAg 2ug IgG2a | 2.530 | 0.676 | 0.124 | 0.027 | 0.029 | 0.002 | |

(continued)

| Serum Titers | 1 E+02 | 1E+03 | 1 E+04 | 1E+05 | 1E+06 | 1E+07 | THtype |
|---|---|---|---|---|---|---|---|
| Recombivax IgG1 | 3.506 | 3.422 | 2.728 | 1.010 | 0.669 | 0.429 | **TH2** |
| Recombivax IgG2a | 0.535 | 0.081 | 0.036 | 0.025 | 0.019 | 0.000 | |
| Free PS-ODN 2 + Alum + 2ug HBsAg IgG1 | 3.474 | 3.406 | 2.626 | 0.841 | 0.525 | 0.232 | **TH0/TH1** |
| Free PS-ODN-2 + Alum + HBsAg 2ug IgG2a | 3.333 | 3.157 | 2.250 | 0.854 | 0.520 | 0.286 | |
| Encaps. ODN-1+Recombivax IgG1 | 3.483 | 3.014 | 0.941 | 0.265 | 0.204 | 0.127 | **TH0/TH1** |
| Encaps. ODN-1+Recombivax IgG2a | 3.130 | 2.038 | 0.664 | 0.162 | 0.178 | 0.139 | |
| Engerix IgG1 | 3.426 | 2.292 | 0.262 | 0.066 | 0.044 | 0.048 | **TH2** |
| Engerix IgG2a | 0.766 | 0.046 | 0.000 | 0.000 | 0.000 | 0.000 | |
| Encaps. PS-ODN 1 1PS+ Engerix IgG1 | 3.125 | 0.744 | 0.074 | 0.022 | 0.015 | 0.014 | **TH1** |
| Encaps. PS-ODN 1+ Engerix IgG2a | 3.172 | 2.188 | 0.395 | 0.030 | 0.000 | 0.000 | |

**Evaluation of TH type of immunity induced upon immunization with altered vaccine vs. conventional.**

Differential induction of Th1 type or Th2 type responses is required for protective immunity to certain infectious diseases. The type of response induced by vaccine may be crucial to its efficacy. Th1 type responses may be essential for control of Hepatitis B viral infection. The type of adjuvant used can direct the type of Th response generated to an administered antigen. Alum, a strong Th2 adjuvant, is a component of all current Hepatitis B vaccines. The research evaluated the ability of LNA/CpG ODN adjuvants to promote a Th1 response. The ratio of IgG2a/IgG1 was used as major indicator of either a predominantly Th1 (IgG2a>>IgG1), predominantly Th2 (IgG1<<IgG2a) or mixed Th1/Th2 (IgG1≅ IgG2a) response (ratio from 0.5 to 2.0). It has been shown that co-administering of CpG ODN with HBsAg not only significantly increased antigen-specific IgG production, but also shifted immune responses in favor of a Th1 response. This effect was augmented further In LNA formulations.

This feature of CpG ODN/LNA adjuvants is even more remarkable taking to the account that Balb/c mice, our model animals, are genetically predisposed to express a Th2 response.

In terms of gross morphology and histopathology of the injection site, LNA/ODN 1 is well tolerated. No signs of local inflammation or necrosis upon intramuscular administration of LNA/ODN 1 was noticed. In comparison, Alum administration, in some instances, led to mild local inflammatory responses and in some cases development of granulomas.

## EXAMPLE 10

**This example illustrates the humoral Immune response.**

**1. Humoral response to LNA and protein antigen**

The cytokine profile induced by LNA alone following a single intravenous administration indicates a Th1 bias, which is characterized by the stimulation of murine antibody isotype IgG2a. This is observed when LNA is co-mixed with a protein antigen such as bovine serum albumin (BSA) and administered subcutaneously to mice. If the DNA core does not contain a CpG motif a strong IgG1 response is observed but CpG-containing particles direct the immune system to produce higher titers of the IgG2a isotype (Figure 34).

**2. Coupling antigen to the LNA platform enhances the humoral response**

An advantage of the LNA platform over other particulate adjuvants is that disease associated antigens can be directly linked to the preformed particles using hydrophobic anchors. Significant enhancements in the nature of an immune response can be realized when antigen and adjuvant are coupled so that they are simultaneously delivered to APCs. For example, when LNA (containing an ISS optimized to activate the murine immune system) is coupled with ovalbumin (OVA) protein it induces titers of total OVA-specific IgG in mice which are 100-fold greater than levels observed

when ODN 2 and OVA are simply mixed or compared to standard benchmark adjuvants (Figure 36). Currently, the most active adjuvant combination commercially available for non-human applications is ImmunoEasy (alum mixed with CpG oligo), which is noted for its production of high IgG titers and early seroconversion. However, our data indicate that OligoVax-OVA particles significantly outperforms ImmunoEasy as well as a number of other adjuvants in the levels of OVA-specific IgG generated two weeks after a single injection (Figure 36).

**3. LNA Induction of mucosal immune responses**

While most vaccines are designed to induce systemic immunity, it is mucosal surfaces that represent the primary sites for entry and transmission of infectious diseases. Therefore, vaccination strategies that induce strong mucosal immune responses are expected to exhibit the most prophylactic benefit. However, induction of IgA antibodies (the dominant mucosal immunoglobulin) has proven to be difficult. The most potent mucosal adjuvant known is cholera toxin (CT), but it is too toxic for human applications. Notably, following three, weekly intranasal administrations, LNA-OVA particles induce an IgA response that is much greater than that obtained using CT mixed with OVA (Figure 35). Enhanced IgA titers were found in serum as well as lung and vaginal washes.

**EXAMPLE 11**

**Stimulation of an Antigen-Specific Mucosal Immune Response Using LNA formulations**

This example illustrates the stimulation of IgA and IgG immune responses to lipid-nucleic acid (LNA) formulations, including LNA formulations comprising a target antigen, chicken ovalbumin ("OVA").

Oligonucleotides

The oligodeoxyonucleotides ("ODNs") used in this study were synthesized with a phosphorothioate ("PS") backbone. The sequences of each ODN are as follows:

ODN #2 PS SEQ ID NO: 1 5'-TCCATGACGTTCCTGACGTT-3'

ODN #1 PS SEQ ID NO: 2 5'-TAACGTTGAGGGGCAT-3'

ODN #3 PS SEQ ID NO: 3 5'-TAAGCATACGGGGTGT-3'

**Preparation of LNA formulations**

LNA formulations were prepared using ODN #1 PS, ODN #2 PS, or ODN #3 PS and OVA as a target antigen. Specifically, LNA formulations were prepared by formulating the ODNs with the lipid mixture:DSPC:CH: DODAP: PEG-C14 (:20:45: 25:10 mol %), using the ethanol-based procedure described in U.S. Pat. Appln. Ser. No. 6,287,591 incorporated herein by reference. Thereby, liposomes encapsulating ODN #1 PS or ODN #2 PS, or ODN #3 PS were prepared. The particle size of the resulting liposomes was 100-140 nm.

The oligonucletides ODN #1 PS, ODN #2 PS, and ODN #3 PS were each encapsulated in lipid particles using an ethanol dialysis procedure and an ionizable aminolipid previously described (see, for example, Semple et al., Methods Enzymol. (2000) 313:322-341; Semple et al., Biochem. Biophys. Acta. (2001) 1510(1-2):152-166). The ODNs were then hydrated in 300 mM citrate buffer (pH 4.0) and prewarmed to 80˚C for 5 min (minutes) before formulation to ensure the presence of monomer ODNs. The lipid formulations consisted of DSPC/CH/DODAP/PEG-CerC14 at 20/45/25/10 molar ratios. Each ODN was encapsulated separately by slowly adding the lipid mixture dissolved in ethanol to the citrate solution of ODN to give a final ethanol concentration of 40 % (vol/vol). The initial ODN to lipid ratio (wt ODN to wt total lipid) was 0.25. The ODN-lipid mixtures were passed 10 times through two stacked 100 nm polycarbonate filters (Osmonics, Livermore, CA) using a thermobarrel extruder (Lipex Biomembranes, Vancouver, BC Canada) maintained at approximately 65 ˚C. Non-encapsulated ODN was then removed from the formulation by an initial 1 hr (hour) dialysis against 300 mM citrate buffer, pH 4.0, before an overnight dialysis against HBS (10 mM Hepes, 145 mM NaCl, pH7.5) followed by DEAE-sepharose CL-6B anion exchange chromatography. The ODN concentration of the formulations was determined by analysis at 260 nm in a spectrophotometer. The mean diameter and size distribution of the LNA particles were determined using a NICOMP Model 370 submicron particle sizer and was typically 110 +/- 30 nm.

**Immunization and Sample Isolation**

C57BL/6 mice (6 weeks old) were immunized with 20 μl of the following test formulations by intranasal administration on day 0 (initial immunization), and days 7, and 14 after the initial immunization.
For Figures 37-39:

OVA alone

OVA co-administered with 10 mg CT ("OVA + CT")

OVA co-administered with ODN #2 ("OVA + ODN #2")

OVA co-administered with ODN #1 ("OVA + ODN #1")

OVA co-administered with ODN #3 ("OVA + ODN #3")

OVA co-administered with LNA containing ODN #2 ("OVA + LNA-ODN #2")

OVA co-administered with LNA containing ODN #1 ("OVA + LNA-ODN #1")

OVA co-administered* with LNA containing ODN #3 ("OVA + LNA-ODN #3")

LNA containing ODN #1 ("LNA-ODN #1")

Mice received OVA protein at a dose of 75 μg per immunization. Free or encapsulated ODN were administered at doses of 1, 10 and 100 μg.
For Figures 42 and 43:

PBS alone

OVA co-administered with 10 mg CT ("OVA + CT")

LNA containing ODN #1 ("LNA-ODN #1")

OVA co-administered with ODN #2 ("OVA + ODN #2")

OVA co-administered with LNA containing ODN #2 ("OVA + LNA-ODN #2")

OVA co-administered with ODN #1 ("OVA + ODN #1") OVA co-administered with LNA containing ODN #1 ("OVA + LNA-ODN #1") OVA co-administered with ODN #3 ("OVA + ODN #3")

OVA co-administered* with LNA containing ODN #3 ("OVA + LNA-ODN #3")

Mice received OVA protein at a dose of 75 μg per immunization. Free or encapsulated ODN were administered at a dose of 100 μg.
Each treatment group (n=5) was anesthetized with halothane before droplets of the vaccine were applied to the esternal nares for complete inhalation. On day 28 after the initial immunization, plasma was collected from anesthetized mice by cardiac puncture and placed into serum tubes. Vaginal washes were obtained by pipetting 50 μl of PBS (Phosphate Buffer Saline) into and out of the vagina of each mouse. This procedure was repeated three times so that a total of 150 μl of washes were collected. The mice were subsequently terminated by cervical dislocation and a lung wash was performed by inserting tubing into the trachea and then pipetting 1 mL of PBS into and out of the lungs. Volume recovery for this precedure was generally70-80 %. Serum tubes were left at room temperature for 30 min to allow for clotting before centrifuging at 10,000 rpm (revolutions per minute) at 4˚C for 5 min, and the resulting aliquots of supematent collected and stored at -20˚C until analysis. The serum aliquots were also stored at -20 ˚C until analysis.
**ELISA Evaluation of the Immune Response**
OVA-specific IgG and IgA antibodies in serum, lung washes, and vaginal washes were measured by ELISA (enzyme-linked immunosorbent assay). Microtiter plates (96 wells) were coated overnight at 4 ˚C with 5 μl/ml of OVA diluted in PBS (50 μl). The microtiter plates were then washed with PBS containing 0.5 % Tween 20 (PBST) and blocked

with 200 μl of 1 % bovine serum albumin (BSA) in PBST for 1 hr at 37 °C with 50 μl of HRP (horse radish peroxidase)-conjugated goat anti-mouse IgG (1:4000) or HRP-conjugated sheep anti-mouse IgA (1:10) diluted with BSA-PBST. Plates were developed in a 30 min room temperature incubation with TMB (3,3',5,5'-Tetramethylbenzidine) (100 μL) before stopping the reaction with 50 μl of 0.5 M $H^2SO^3$. Optical densities were read at 450-570 nm with an ELISA plate reader.

**Results**

Results are shown in Figures 37-39 illustrating antibody titers in serum, lung washes, and vaginal washes.

Figures 37-39 (b) show that, in the test formulations using OVA co-administered with the LNA formulations, anti-OVA-specific IgA levels were increased at both local and distant mucosal sites by several orders of magnitude relative to OVA co-administered with ODN, OVA co-administered with CT, OVA alone, or LNA-ODN #1 alone. Figures 37-39 (a) show that, in the test formulations using OVA co-administered with the LNA formulations, anti-OVA-specific IgG levels were increased at both local and distant mucosal sites by several orders of magnitude relative to OVA co-administered with ODN, OVA co-administered with CT, OVA alone, or LNA-ODN #1 alone. Figures 37 and 38 (c) show that liposome encapsulation of the ODNs in the LNA formulations of the present invention increased anti-OVA IgM titers by several orders of magnitude relative to OVA co-administered with ODN, OVA co-administered with CT, OVA alone, or LNA-ODN #1. This response was dose dependent.

Figures 42 and 43 illustrate antibody titers in vaginal washes and lung washes.

These figures show that, in the test formulations using OVA coadministered with the LNA formulations containing ODN #1, ODN #2 and ODN #3, the anti-OVA-specific IgA levels were increased at both local and distant mucosal sites by several orders of magnitude relative to OVA co-administered ODN #1, ODN#2 and ODN #3, LNA-ODN #1 alone, OVA co-administered with CT and PBS alone.

**<u>EXAMPLE 12</u>**

**Stimulation of an Antigen-Specific Mucosal Immune Response Using OVA Coupled LNA formulations**

This example illustrates the stimulation of a mucosal immune response to a target antigen using lipid-nucleic acid ("LNA") formulations containing synthetic oligodeoxynucleotides having immunostimulatory CpG motifs ("ISS ODNs") and co-administered with ovalbumin ("OVA") as the target antigen.

**Oligonucleotides**

ISS ODN having 1 CpG motif, ODN #1 and ODN #2, were used in this Example and were synthesized with a phosphorothioate ("PS") backbone ("ODN #2 PS" and "ODN #1 PS" respectively). The sequence of each ODN is provided above in Example 11.

**Preparation of LNA formulations**

LNA formulations comprising ODN #1 PS or ODN #2 PS were prepared by formulating the ODNs with the lipid mixtureDSPC:CH: DODAP :PEG-C14 (20:45: 25:10 molar ratio), using the ethanol-based procedure fully described in U.S. Pat. Ser. No. 6,287,591 and incorporated herein by reference. Thereby, liposomes encapsulating ODN #1 ("LNA-ODN #1 PS") or ODN #2 ("LNA-ODN #2 PS") were prepared. Two different amounts of each ODN, 10 μg and100 μg, were used to prepare the LNA formulations. The particle size of the resulting liposomes was 100-140 nm. OVA coadminstered with CT was used as a control.

**Preparation of OVA coupled LNA formuation**

Two methods were used to prepare the formulation. Both methods rely on the OVA protein being activated by a thiolation procedure. The activated protein was chemically coupled directly to an active lipid species, for example DSPE-PEG-MPB with standard sulfahydryl chemistry (see, for example, Harasym et al., Bioconjugate Chemistry (1995) 6:187; Hermanson et al., Bioconjugate Techniques, Academic Press (1996) 230-232; Ansell et al., Antibody conjugation methods for active targeting of liposomes pages 51-68 in Drug targeting: strategies, principles and applications, Methods in Molecular Medicine, Vol 25, Edited by Francis, GE. and Delgado C., Human Press Inc., Totwa, New Jersey).

There are two ways of inserting the reactive lipid into the LNA formulation.

1) The reactive lipid is added when all the other lipid components of the LNA formuation are combined during the ethanol procedure described above. OVA is then coupled to the lipid after the lipid is in the formulation. This is called

active coupling and is described in detail below.

2) The second method requires the lipid to first be combined with the OVA protein. This combined structure is then inserted into a preformed LNA formulation. This is called passive couples and again is described in detail below.

**Thiolation of OVA protein with SPDP**

OVA (40 mg) dissolved in HBS (1 mol; 25 mM hepes, 150 mM NaCl, pH 7.4). A stock solution of SPDP (3-(2-pyridyldithio)propionic acid N-hydroxysuccinimide ester) was prepared in ethanol (28.8:1 EtOH/mg of SPDP) and an aliquot (40:1) added with vortexing to the OVA solution. The solution was stirred at room temperature for 30 minutes and passed down a Sephadex G-50 column (15 mil, SAS (sodium acetate saline) pH 4.4). Fractions were collected after 16 drops had fallen and analyzed at in a spectrophotometer at 280 nm, and fractions that were > 1.0 at A280 (absorbance at 280 nm) were combined. Typically 2.5-3 ml of protected thiolated protein is produced using this method. DTT (dithothreitol, 3.8 mg/ml solution) was then added directly as a solid and the solution stirred for 15 minutes. The solution was then passed down a sephadex G-50 column (50 ml in HBS, pH 7.4) collecting 20-24 drop fractions. The fractions were analyzed in a spectrophotometer at 280 nm, and those fractions that were > 1.0 at A280 were combined. An aliquot of the combined fraction was then diluted 10 fold in HBS (Hepes Buffered Saline) and analyzed at 280 nm using HBS as a control sample. The protein content was determined by applying a factor of 1.8 to convert the absorbance into concentration (mg/ml).

**Preparation of LNA-protein Conjugates Using Active Coupling**

Active coupling techniques refer to protocols in which an activated protein is chemically coupled directly to a reactive lipid incorporated into the lipid particle.

A solution of lipid comprised of DSPC/chol/MePEGS-2000-DMG/DODAP/DSPE-ATTA2-MPA (32:45:2:20:1 mol/mol) in ethanol (1.2 ml) was warmed to 60 oC and slowly added to a solution of ODN #1 (12 mg in 1.8 ml 300 mM citrate at PH 4.0), which had previously been warmed to 60 oC as well. The solution was vigorously agitated during addition. This crude LNA was then passed 10 times through two 100 nm filters using an extruder device set at 65 oC. The resulting sized and crude LNA was then passed down a Sephadex G-50 column (50 ml; HBS) and used immediately. The approximate lipid concentration was estimated assuming that most of the lipid had been recovered from the column.

The thiolated OVA was then added to the activated LNA particles at an initial protein to lipid ratio of 150 g/mol and stirred at room temperature for 16 hours. The resulting LNA-protein (or LNA-OVA) conjugates were then separated from unreacted protein using Sepharose Cl-4B columns (25 ml; HBS, -1 ml sample per column).

**Preparation of LNA-Protein Conjugates Using Passlve Coupling**

Passive coupling techniques refer to protocols in which an activated protein is coupled to a reactive lipid remotely from the final lipid particle, and is then incorporated into the particle in some way, either by exchange into a preformed particle or by incorporation during the formation phase of the particle.

LNA particles were prepared as described above. A micellar solution of DSPE-ATTA2-MPA/DSPE-ATTA4-NBOC (1:4) was prepared by dissolving the lipid in a minimum of ethanol and slowly adding HBS, with a final lipid concentration at 10 mM. An aliquot of this solution was then added to the thiolated OVA described above in a ratio of 3000 g OVA/mol lipid and allowed to stir at room temperature overnight. An aliquot of this solution, corresponding to 150 g OVA/mol of lipid in the LNA, was added to a sample of the LNA and incubated in a water bath at 60 oC for an hour. This solution was then passed down a Sepharose CL-4B column (25 ml; HBS; ~1ml sample per column).

**Immunization and Sample Isolation**

C57BU6 mice (6 weeks old) were immunized with 20 μl of the following test formulations by intranasal administration on day 0 (initial immunization), and days 7, and 14 after the initial immunization.

For Figures 40 and 41:

OVA co-administered with ODN #1 PS ("OVA + ODN #1 PS") at a dose of 10 μg.

OVA co-administered with ODN #1 PS ("OVA + ODN #1 PS") at a dose of 100 μg.

OVA co-administered with LNA containing ODN #1 PS ("OVA+LNA-ODN #1 PS") at a dose of 10 μg.

OVA co-administered with LNA containing ODN #1 PS ("OVA+LNA #1 PS") at a dose of 100 μg.

OVA coupled to LNA containing ODN #1 ("OVA/LNA-ODN #1 PS") at a dose of 10 μg

OVA coupled to LNA containing ODN #1 ("OVA/LNA-ODN #1 PS") at a dose of 100 μg.

OVA co-administered with 10μg CT ("OVA + CT").

OVA co-administered with LNA containing ODN #2 ("OVAILNA-ODN #2 PS") at a dose of 10 μg.

Mice received OVA protein at a dose of 75 μg per immunization.

For Figures.44-46:

OVA coupled to LNA containing ODN #1 ("OVA/LNA-ODN #1 PS") at a dose of 100 μg.

OVA coupled to LNA containing ODN #1 ("OVA/LNA-ODN #1 PS") at a dose of 10 μg.

OVA co-administered with LNA containing ODN #1 PS ("OVA+LNA #1 PS") at a dose of 100 μg.

OVA co-administered with LNA containing ODN #1 PS ("OVA+LNA #1 PS") at a dose of 10 μg.

OVA co-administered with ODN #1 PS ("OVA + ODN #1 PS") at a dose of 100 μg.

OVA co-administered with ODN #1 PS ("OVA + ODN #1 PS") at a dose of 10 μg.

OVA co-administered with 10 μg of CT ("OVA + CT").

PBS alone

Mice received OVA protein at a dose of 75 μg per immunization.

Each treatment group (n=5) was anesthetized with halothane before droplets of the vaccine were applied to the external nares for complete inhalation. On day 28 after the initial immunization, plasma was collected from anesthetized mice by cardiac puncture and placed into serum tubes. Vaginal washes were obtained by pipetting 50μl of PBS (Phosphate Buffer Saline) into and out of the vagina of each mouse. This procedure was repeated three times so that a total of 150 μl of washes were collected. The mice were subsequently terminated by cervical dislocation and a lung wash was performed by inserting tubing into the trachea and then pipetting 1 ml of PBS into and out of the lungs. Volume recovery for this procedure was generally 70-80 %. Serum tubes were left at room temperature for 30 min to allow for clotting before centrifuging at 10,000 rpm (revolutions per minute) at 4 ˚C for 5 min, and the resulting aliquots of serum collected and stored at -20 ˚C until analysis.

**EUSA Evaluation of the Immune Response**

OVA-specific IgG and IgA antibodies in serum, lung washes, and vaginal washes were measured by ELISA (enzyme-linked immunosorbent assay). Microtiter plates (96 wells) were coated overnight at 4 oC with 5 μl/ml of OVA diluted in PBS (50 μl). The microtiter plates were then washed with PBS containing 0.5 % Tween 20 (PBST) and blocked with 200 μl of 1 % bovine serum albumin (BSA) in PBST for 1 hr at 37 oC with 50 μl of HRP (horse radish peroxidase)-conjugated goat anti-mouse IgG (1:4000) or HRP-conjugated sheep anti-mouse IgA (1:10) diluted with BSA-PBST. Plates were developed in a 30 min room temperature incubation with TMB. (100 μL) before stopping the reaction with 50 μl of 0.5 M H2SO3. Optical densities were read at 450-570 nm with an ELISA plate reader.

**Results**

Results are shown in Figures 40 and 41 illustrating antibody titers in serum, lung washes, and vaginal washes, Figures 44 and 45 illustrating antibody titers in vaginal washes and lung washes and Figure 46 illustrating antibody titers in serum.

Figure 41 shows that, in the test formulations using OVA coupled to the LNA formulations containing ODN #1, the anti-OVA-specific IgA levels were increased at both local and distant mucosal sites by several orders of magnitude relative to OVA co-administered with the LNA formulations containing ODN #2 or #1, OVA co-administered with ODN #1 and OVA co-administered with CT.

Figure 40 shows that, in the test formulations using OVA coupled to the LNA formulations containing ODN #1,

the, anti-OVA-specific IgG levels were increased at both local and distant mucosal sites by several orders of magnitude relative to OVA co-administered with the LNA formulations containing ODN #2 or #1, OVA co-administered with ODN #1and OVA co-administered with CT.

Figure 44 shows that, in the test formulations using OVA coupled to the LNA formulations containing ODN #1, the anti-OVA-specific IgA levels were increased at both local and distant mucosal sites by several orders of magnitude relative to OVA co-administered with the LNA formulations containing ODN #1, OVA co-administered with ODN #1, OVA co-administered with CT, PBS alone.

Figures 45 and 46 show that, In the test formulations using OVA coupled to the LNA formulations containing ODN #1, anti-OVA-specific IgG levels were increased at both local and distant mucosal sites by several orders of magnitude relative to OVA co-administered with the LNA formulations containing ODN #1, OVA co-administered with ODN #1, OVA co-administered with CT, or PBS alone.

In summary, this data demonstrates that IgA and IgG responses are greatly enhanced when the OVA is coupled to the LNA formulations. For example, mice immunized with OVA coupled to the LNA formulations containing ODN #1 of the present invention exhibited greater IgA titers in all fluids analyzed when compared to mice immunized with OVA mixed with free or encapsulated ODN #1 (see Figures 41 and 44). A dose response was also observed with the OVA coupled to the LNA formulations as a greater amount of ODN administered to the mice resulted in a larger production of IgA antibodies. This data demonstrate that coupling of OVA to LNA formulations can increase the ability of the LNA particles to generate IgA antibodies which has important implications for mucosal immunity. Further, mice immunized with OVA coupled to the LNA formulations containing ODN #1 of the present invention exhibited greater IgG titers in all fluids analyzed when compared to mice immunized with OVA mixed with free or encapsulated ODN #1 (see Figures 40, 45 and 46). A dose response was also observed with the OVA coupled to the LNA formulations as a greater amount of ODN administered to the mice resulted in a larger production of IgG antibodies. This data demonstrate that coupling of OVA to LNA formulations can increase the ability of the LNA particles to generate IgG antibodies.

## EXAMPLE 13

### Induction of CTL Response Using A Polytope Approach with Multiple Tumor-Associated Antigens

Single epitope-based approaches have the disadvantage that an MHC-restricted CTL response is raised to only one antigen. In addition many cancer antigens are non-mutated differentiation antigens, such as TRP-2 exemplified above, and thus self-reactive T cells in the host are predominantly deleted during thymic education. A polytope approach would allow multiple antigens to be simultaneously targeted and should increase the spectrum of anti-tumor CTL responses against such self-antigens. CTL responses specific for multiple antigens and restricted by multiple MHC alleles would clearly be desirable for broader immune reactivity, given the variable expression of tumor antigens and MHC alleles in different malignancies. Targeting multiple antigens associated with a particular malignancy would minimize the chances of tumor escape by antigen downregulation or epitope mutation.

Targeting multiple antigens and MHC alleles might be achieved by using multiple recombinant antigen mixtures of synthetic peptide epitopes. To improve the immune response against these multi-epitope antigens several adjuvants are under assay. This experiment employs encapsulated PS-ODN 1m in POPC: CHL: DODMA: DMG (25:45:20:10 molar ratio)] in combination with two murine melanoma antigens TRP2 (H2Kb, VYDFFVWL) and Gp100 (H2Db, EGSRNQDWL). C57BL/6 mice were injected 3 times with either each antigen, or both, in the presence of encapsulated PS-ODN 1m. As positive control we used dendritic cells known to be potent in inducing CTL responses. B16 lysate containing multiple epitopes was also assayed either with ODN 1m or DC for the ability to induce multi-epitope immunity. Immune response was assessed by the ability of CD8+ T cells to mediate cytotoxicity against B16 tumor cells in an antigen-specific manner.

Results: When injected together with encapsulated ODN 1m, a CTL response was raised against both antigens (Figure 47). PS-ODN 1m was as good as DC in generating CTL response against both antigens delivered together (Figure 48). Injection of B16 lysate with PS-ODN 1m was more potent in inducing CTL than injection of DC incubated with B16 lysate (Figure 49).

The Examples provided illustrate certain embodiments of the invention. In a more general sense, however, the invention encompasses compositions and methods for providing therapeutic benefits to mammalian subjects (including humans) utilizing such compositions. Certain compositions of the invention are in the form of a lipid membrane vesicle; and a nucleic acid fully encapsulated within said vesicle. Where stimulation of a response to a particular antigen is desired, the composition may also associate the antigen with the vesicle, for example via chemical coupling, hydrophobic bonding or ionic bonding to an external surface of the vesicle, or encapsulation within the vesicle.

Preferred compositions are those in which the nucleic acid comprises greater than 4% by weight of the composition.

The nucleic acid in the compositions of the invention may suitably be nucleic acids which are not complementary

to the genome of the treated mammal, and which provide immunostimulation through a mechanism which does not depend on a complementary base-pairing interaction with nucleic acids of the mammal. Such nucleic acids will frequently contain an immunostimulating sequence, such as a CpG motif or an immune stimulating palindrome.

The nucleic acids used in the compositions of the invention may be nucleic acids which do not induce an immune response when administered in free form to a naive mammal, or which suppress an immune response to an immune stimulating sequence of nucleotides when administered in free form to a naive mammal.

The nucleic acids may have exclusively phosphodiester internucleotide linkages or may be modified in which a way that they a plurality of phosphodiester internucleotide linkages in combination with modified intemucteotide linkages. The nucleic acids may also contain exclusively modified linkages, or a plurality of modified linkages. For example, the nucleic acid may contain exclusively phosphorothioate internucleotide linkages or a plurality of phosphorothioate internucleotide linkages.

The cationic lipid which is used in formulating the composition suitably is selected from DODAP, DODMA, DMDMA, DOTAP, DC-Chol, DDAB, DODAC, DMRIE, and DOSPA. In addition, the lipid formulation preferably includes an aggregation preventing compound, such as a PEG-lipid. a PAO-lipid or a ganglioside.

In addition to or instead of an antigen, the compositions of the invention can include a co-encapsulated cytotoxic agent such as doxorubicin. The lipid membrane vesicle fully encapsulates both the nucleic acid and the cytotoxic agent. Compositions of this type can be prepared by a method which is a further aspect if the invention. In this method, a therapeutic composition is prepared preparing lipid in ethanol; mixing lipid with ofigonucleotide In aqueous buffer to form oligonucleotide loaded lipid vesicles; and exposing the oligonucleotide loaded lipid vesicles to a cytotoxic agent such that the cytotoxic agent actively accumulates in the interior space of said vesicle.

The compositions of the invention can be used in various methods to provide therapeutic benefits to mammals, including humans, through the use of a lipid-nucleic acid particle comprising a nucleic acid which is fully encapsulated in a lipid formulation comprising a cationic lipid in the manufacture of a medicament. Thus, the compositions can be used to induce an immune response in a mammal, to activate CTL or B cells in a mammal or to treat neoplasia in a mammal having a neoplasia by a method comprising the steps of preparing a lipid-nucleic acid particle comprising a nucleic acid which is fully encapsulated in a lipid formulation, which lipid formulation comprises a cationic lipid; and administering the lipid-nucleic acid particle to the mammal.

When an antigen is included in the composition, a method of inducing an immune response to the antigen may comprise preparing a particle comprising a lipid membrane vesicle comprising a nucleic acid fully encapsulated within said vesicle and an antigen to which an immune response is desired associated with an external surface of said vesicle, and administering the particles to the mammalian subject to be treated.

As demonstrated in the examples above, the utilization of a lipid carrier in the compositions in accordance with the invention allows a substantial reduction in the amount of oligonucleotide needed to achieve the desired stimulation of the immune system. In some cases, this is reflected in the fact that an oligonucleotide which had no apparent activity in the free form is useful for stimulating an immune response when provided in lipid-encapsulated form. In other cases, this is reflected in the fact that the amount of ODN necessary to achieve the same level of response with a lower dosage of ODN. Thus, in practicing a method employing an effective amount of oligonucleotide to stimulate an immune response in a mammal, the present invention provides the improvement comprising fully-encapsulating the oligonucleotide in a lipid vesicle and administering less than 20% of said effective amount of oligonucleotide to a mammalian subject, thereby obtaining a desired immune response in said mammalian subject.

While the data depicted herein demonstrates immunostimulatory activity *in vivo* and therapeutic efficacy using certain exemplary embodiments of the invention, which are provided for completeness and consistency, it is understood that the invention is not limited to these exemplary embodiments. One of ordinary skill in the art will be readily able to make and use other specific embodiments of the invention consistent with the teachings provided herein within the scope of the following claims.

## Claims

**1.** A pathogen vaccine comprising a lipid-nucleic acid (LNA) formulation in combination with at least one microbial antigen, wherein said at least one microbial antigen is mixed with or associated with said LNA formulation, said LNA formulation comprising:

a) a lipid component comprising at least one cationic lipid; and
b) a nucleic acid component comprising at least one oligonucleotide having at least one CpG dinucleotide with a methylated cytosine;

wherein said vaccine is capable of stimulating a Th-1 biased immune response *in vivo* to said at least one microbial

antigen.

2. The vaccine according to Claim 1, wherein said at least one microbial antigen comprises a single epitope.

3. The vaccine according to Claim 2, wherein said at least one microbial antigen comprises HBsAg.

4. The vaccine according to Claim 1, wherein said at least one microbial antigen comprises a plurality of epitopes from the same antigen.

5. The vaccine according to Claim 1, wherein said at least one microbial antigen comprises a plurality of epitopes from different antigens.

6. A polytope pathogen vaccine comprising a lipid-nucleic acid (LNA) formulation in combination with a plurality of microbial antigens, wherein said plurality of microbial antigens are associated with said LNA formulation, said formulation comprising:

a) a lipid component comprising at least one cationic lipid; and
b) a nucleic acid component comprising at least one oligonucleotide having at least one CpG dinucleotide with a methylated cytosine,

wherein said vaccine is capable of simultaneously delivering said plurality of antigens to antigen presenting cells in conjunction with adjuvant immune stimulation by said CpG dinucleotide to induce a Th-1 biased immune response.

7. The vaccine according to Claim 7, wherein said oligonucleotide comprises a phosphodiester backbone.

8. The vaccine according to any one Claims 1 to 6, wherein said oligonucleotide comprises a modified phosphate backbone.

9. The vaccine according to Claim 8, wherein said modified phosphate backbone is phosphorothioate.

10. The use of a vaccine according to any one of Claims 1-9 in the manufacture of a medicament for stimulating an enhanced host immune response to a microbial antigen.

11. The use of a lipid-nucleic acid (LNA) formulation associated with a target antigen, said LNA formulation comprising:

a) a lipid component comprising at least one cationic lipid; and
b) a nucleic acid component comprising at least one oligonucleotide having at least one CpG dinucleotide with a methylated cytosine;

in the manufacture of a medicament for simultaneously delivering antigenic and adjuvant immune stimulation to antigen presenting cells to enhance Th-1 biased immune response.

12. The use of an immunostimulatory composition comprising an encapsulated oligonucleotide having a modified phosphate backbone and at least one CpG dinucleotide with a methylated cytosine in the manufacture of a medicament for enhancing the humoral component of a host immune response to antigenic stimulation.

13. The use according to Claim 12, wherein said immunostimulatory composition is associated with at least one target antigen.

14. The use according to Claim 11, wherein the target antigen is associated with the LNA by at least one of chemical coupling, hydrophobic bonding or ionic bonding to a surface of the LNA.

15. The use of an immunostimulatory composition comprising an encapsulated oligonucleotide having a modified phosphate backbone and at least one CpG dinucleotide with a methylated cytosine in the manufacture of a medicament for improving the maturation of the humoral component of a host immune response to antigenic stimulation or for increasing antigen-specific antibody isotype switching in response to antigenic stimulation .

16. The use of an immunostimulatory composition comprising an encapsulated oligonucleotide having a modified phosphate backbone and at least one CpG dinucleotide with a methylated cytosine in the manufacture of a medicament for inducing increased Th-1 type cytokine secretion in a host in response to antigenic stimulation.

**17.** The use according to any one of Claims 12-16, wherein said modified phosphate backbone is phosphorothioate.

**18.** An improved vaccine for stimulating a host immune response against a hepatitis B virus, comprising a lipid-nucleic acid (LNA) formulation in combination with at least one hepatitis B antigen, wherein said at least one hepatitis B antigen is mixed with or associated with said LNA formulation, said LNA formulation comprising:

a) a lipid component comprising at least one cationic lipid; and
b) a nucleic acid component comprising at least one oligonucleotide having at least one CpG dinucleotide with a methylated cytosine;

wherein said vaccine is effective in inducing increased Th-1 type antibody titers *in vivo.*

**19.** The vaccine according to Claim 18, wherein said at least one hepatitis B antigen comprises at least one epitope of hepatitis B surface antigen (HBsAg).

**20.** The vaccine according to Claim 19, wherein said hepatitis B surface antigen is recombinantly produced.

## Patentansprüche

**1.** Ein pathogener Impfstoff, der eine Lipid-Nukleinsäure-(LNA)-Formulierung zusammen mit mindestens einem mikrobiellen Antigen umfasst, wobei das mindestens eine mikrobielle Antigen mit der LNA Formulierung gemischt wird oder damit verbunden ist, wobei die LNA Formulierung umfasst:

a) eine Lipidkomponente, die mindestens ein kationisches Lipid umfasst; und
b) eine Nukleinsäurekomponente, die mindestens ein Oligonukleotid mit mindestens einem CpG Dinukleotid mit einem methylierten Cytosin umfasst;

wobei der Impfstoff eine Th-1 beeinflusste Immunantwort *in vivo* gegenüber dem mindestens einen mikrobiellen Antigen stimulieren kann.

**2.** Der Impfstoff gemäß Anspruch 1, wobei das mindestens eine mikrobielle Antigen ein einzelnes Epitop umfasst.

**3.** Der Impfstoff gemäß Anspruch 2, wobei das mindestens eine mikrobielle Antigen HBsAg umfasst.

**4.** Der Impfstoff gemäß Anspruch 1, wobei das mindestens eine mikrobielle Antigen eine Vielzahl von Epitopen desselben Antigens umfasst.

**5.** Der Impfstoff gemäß Anspruch 1, wobei das mindestens eine mikrobielle Antigen eine Vielzahl von Epitopen von verschiedenen Antigenen umfasst.

**6.** Ein polytoper, pathogener Impfstoff, der eine Lipid-Nukleinsäure-(LNA)-Formulierung zusammen mit einer Vielzahl mikrobieller Antigene umfasst, wobei die Vielzahl mikrobieller Antigene mit der LNA Formulierung verbunden ist, wobei die Formulierung umfasst:

a) eine Lipidkomponente, die mindestens ein kationisches Lipid umfasst, und
b) eine Nukleinsäurekomponente, die mindestens ein Oligonukleotid mit mindestens einem CpG Dinukleotid mit einem methylierten Cytosin umfasst;

wobei der Impfstoff gleichzeitig die Vielzahl von Antigenen zu Antigen-präsentierenden Zellen zusammen mit Hilfsimmunstimulierung durch das CpG Dinukleotid abgeben kann, um eine Th-1 beeinflusste Immunantwort zu induzieren.

**7.** Der Impfstoff gemäß Anspruch 7, wobei das Oligonukleotid ein Phosphodiesterrückgrat umfasst.

8. Der Impfstoff gemäß einem der Anspruch 1 bis 6, wobei das Oligonukleotid ein modifiziertes Phosphatrückgrat umfasst.

9. Der Impfstoff gemäß Anspruch 8, wobei das modifizierte Phosphatrückgrat Phosphorothionat ist.

10. Die Verwendung eines Impfstoffs nach einem der Ansprüche 1-9 bei der Herstellung eines Medikaments zur Stimulierung einer gesteigerten Wirtimmunantwort gegenüber einem mikrobiellen Antigen.

11. Die Verwendung einer Lipid-Nukleinsäure-(LNA)-Formulierung, die mit einem Target-Antigen verbunden ist, wobei die LNA Formulierung umfasst:

a) eine Lipidkomponente, die mindestens ein kationisches Lipid umfasst, und
b) eine Nukleinsäurekomponente, die mindestens ein Oligonukleotid mit mindestens einem CpG Dinukleotid mit einem methylierten Cytosin umfasst;

bei der Herstellung eines Medikaments zur gleichzeitigen Abgabe von antigener und Hilfsimmunstimulierung gegenüber Antigen-präsentierenden Zellen, um eine Th-1 beeinflusste Immunantwort zu verstärken.

12. Die Verwendung einer immunostimulierenden Zusammensetzung, die ein eingekapseltes Oligonukleotid mit einem modifizierten Phosphatrückgrat und mindestens ein CpG Dinukleotid mit einem methylierten Cytosin umfasst, bei der Herstellung eines Medikaments zur Verstärkung der humoralen Komponente einer Wirtimmunantwort gegenüber einer antigenen Stimulierung.

13. Die Verwendung gemäß Anspruch 12, wobei die immunostimulierende Zusammensetzung mit mindestens einem Target-Antigen verbunden ist.

14. Die Verwendung gemäß Anspruch 11, wobei das Target-Antigen mit der LNA durch mindestens eines von chemischer Kopplung, hydrophober Bindung oder ionischer Bindung auf die Oberfläche der LNA verbunden ist.

15. Die Verwendung einer immunostimulierenden Zusammensetzung, die ein eingekapseltes Oligonukleotid mit einem modifizierten Phosphatrückgrat und mindestens einem CpG Dinukleotid mit einem methylierten Cytosin umfasst, bei der Herstellung eines Medikaments zur Verbesserung der Maturation der humoralen Komponente einer Wirtimmunantwort gegenüber einer antigenen Stimulierung oder zur Steigerung des Antigen-spezifischen Antikörper Klassenwechsels in Antwort auf antigene Stimulierung.

16. Die Verwendung einer immunostimulierenden Zusammensetzung, die ein eingekapseltes Oligonukleotid mit einem modifizierten Phosphatrückgrat und mindestens ein CpG Dinukleotid mit einem methylierten Cytosin umfasst, bei der Herstellung eines Medikaments zur Induzierung gesteigerter Th-1 Typ Cytokinsekretion in einem Wirt in Antwort auf antigene Stimulierung.

17. Die Verwendung nach einem der Ansprüche 12-16, wobei das modifizierte Phosphatrückgrat Phosphorothionat ist.

18. Ein verbesserter Impfstoff zur Stimulierung einer Wirtimmunantwort gegenüber einem Hepatitis B Virus, der eine Lipid-Nukleinsäure-(LNA)-Formulierung zusammen mit mindestens einem Hepatitis B Antigen umfasst, wobei das mindestens eine Hepatitis B Antigen mit der LNA Formulierung gemischt wird oder damit verbunden ist, wobei die LNA Formulierung umfasst:

a) eine Lipidkomponente, die mindestens ein kationisches Lipid umfasst, und
b) eine Nukleinsäurekomponente, die mindestens ein Oligonukleotid mit mindestens einem CpG Dinukleotid mit einem methylierten Cytosin umfasst;

wobei der Impfstoff bei der Induzierung erhöhter Th-1 Typ Antikörpertiter *in vivo* wirksam ist.

19. Der Impfstoff gemäß Anspruch 18, wobei das mindestens eine Hepatitis B Antigen mindestens ein Epitop eines Hepatitis B Oberflächenantigens (HBsAg) umfasst.

20. Der Impfstoff gemäß Anspruch 19, wobei das Hepatitis B Oberflächenantigen rekombinant hergestellt wird.

**Revendications**

1. Un vaccin contre un pathogène comportant une formulation lipide - acide nucléique (LNA) combinée avec au moins un antigène microbien, dans lequel ledit au moins un antigène microbien est mélangé ou associé avec la formulation LNA, ladite formation LNA comprenant :

   a) une composante lipide comportant au moins un lipide cationique ; et
   b) une composante acide nucléique comportant au moins un oligonucléotide ayant au moins un dinucléotide CpG avec une cytosine méthylée ;

   dans lequel ledit vaccin a la propriété de stimuler la réponse immune liée au Th-1 *in vivo* dirigée vers ledit antigène microbien.

2. Le vaccin selon la revendication 1, dans lequel ledit au moins un antigène microbien comporte un épitope unique.

3. Un vaccin selon la revendication 2, dans lequel ledit au moins un antigène microbien comporte de l'HbsAg.

4. Le vaccin selon la revendication 1, dans lequel ledit au moins un antigène microbien comprend une pluralité d'épitope à partir du même antigène.

5. Un vaccin selon la revendication 1, dans lequel ledit au moins un antigène microbien comprend une pluralité d'épitope à partir d'antigènes différents.

6. Un vaccin contre un pathogène poly-épitopique comportant une formulation lipide - acide nucléique (LNA) combinée avec une pluralité d'antigènes microbiens, dans lequel ladite pluralité d'antigènes microbiens est associée avec la formulation LNA, laquelle formulation comprenant :

   a) une composante lipide comportant au moins un lipide cationique ; et
   b) une composante acide nucléique comportant au moins un oligonucléotide ayant au moins un dinucléotide CpG avec une cytosine méthylée,

   lequel vaccin cité ici a la propriété de délivrer simultanément ladite pluralité d'antigènes aux cellules présentatrices d'antigènes en conjonction avec un adjuvant stimulant de la réponse immune par ledit dinucléotide CpG pour induire une réponse immune Th-1.

7. Un vaccin selon la revendication 1, dans lequel ledit oligonucléotide comporte un squelette phosphodiester.

8. Un vaccin selon l'une quelconque des revendications de 1 à 6, dans lequel ledit oligonucléotide comporte un squelette phosphodiester modifié.

9. Un vaccin selon la revendication 8, dans lequel ledit squelette phosphate modifié est un phosphoriothioate.

10. L'utilisation d'un vaccin selon l'une quelconque des revendications 1 - 9 dans la fabrication d'un médicament dont l'action est de stimuler la réponse immune de l'hôte envers un antigène microbien.

11. L'utilisation d'une formulation lipide - acide nucléique (LNA) associée avec un antigène cible, ladite formulation LNA comportant :

    a) une composante lipide comportant au moins un lipide cationique ; et
    b) une composante acide nucléique comportant au moins un oligonucléotide ayant au moins un dinucléotide CpG avec une cytosine méthylée ;

    dans la fabrication d'un médicament pour l'administration simultanée à des cellules présentatrices d'antigène d'une stimulation immune antigénique et d'un adjuvant afin d'améliorer la réponse immune Th-1.

12. L'utilisation d'une composition immunostimulante comportant un oligonucléotide encapsulé ayant un squelette phosphate modifié et au moins un dinucléotide comprenant une cytosine méthylée afin de fabriquer un médicament dont la propriété est d'améliorer la composante humorale de la réponse immune de hôte à une stimulation antigénique.

**13.** L'utilisation selon la revendication 12, dans laquelle ladite composition immunostimulante est associée avec au moins un antigène cible.

**14.** L'utilisation selon la revendication 11, dans laquelle l'antigène cible est associé avec du LNA avec au moins un lien chimique, liaison hydrophobe ou ionique à la surface du LNA.

**15.** L'utilisation d'une composition immunostimulante comportant un oligonucléotide encapsulé ayant un squelette phosphate modifié et au moins un dinucléotide CpG avec une cytosine méthylée dans la fabrication d'un médicament pour l'amélioration de la maturation de la composante humorale de la réponse immune de l'hôte à une stimulation antigénique ou pour l'augmentation du changement isotopique au niveau de l'anticorps spécifique de l'antigène en réponse à la stimulation antigénique.

**16.** L'utilisation d'une composition immunostimulante comportant un oligonucléotide encapsulé ayant un squelette phosphate modifié et au moins un dinucléotide avec une cytosine méthylée dans la fabrication d'un médicament capable d'induire une augmentation de la sécrétion de cytokine type Th-1 de l'hôte en réponse à la stimulation antigénique.

**17.** l'utilisation selon l'une quelconque des Revendications 12 - 16, dans laquelle ledit squelette phosphate modifié est un phosphorothioate.

**18.** Un vaccin amélioré pour stimuler la réponse immune de l'hôte contre le virus de l'hépatite B, comportant une formulation lipide - acide nucléique en combinaison avec au moins un antigène de l'hépatite B, dans lequel ledit au moins un antigène de l'hépatite B est mélangé ou associé avec la formulation LNA, ladite formulation LNA comprenant :

a) une composante lipide comportant au moins un lipide cationique ; et
b) une composante acide nucléique comportant au moins un oligonucléotide ayant au moins un dinucléotide CpG avec une cytosine méthylée ;

dans lequel ledit vaccin a la propriété d'induire une augmentation du titre d'anticorps type Th-1 *in vivo.*

**19.** Le vaccin selon la revendication 18, dans lequel ledit au moins un antigène de l'hépatite B comporte au moins un épitope de l'antigène de surface de l'hépatite B (HBsAg).

**20.** Le vaccin selon la revendication 19, dans lequel ledit antigène de surface de l'hépatite B est produit par technique de recombinaison.

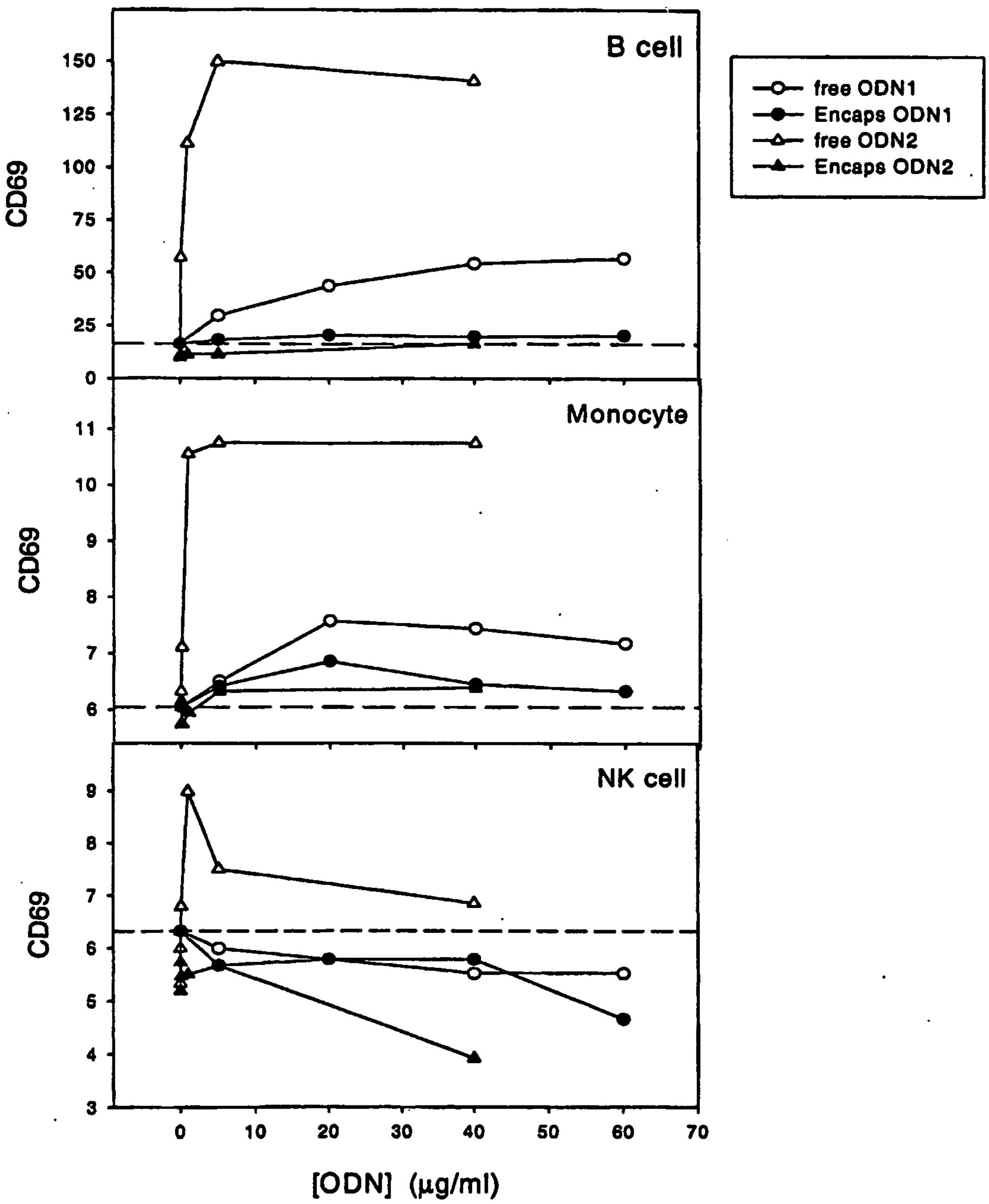
B cell
Monocyte
NK cell
CD69
CD69
CD69
150
125
100
75
50
25
0
11
10
9
8
7
6
9
8
7
6
5
4
3
0
10
20
30
40
50
60
70
[ODN] (μg/ml)
free ODN1
Encaps ODN1
free ODN2
Encaps ODN2

Figure 1

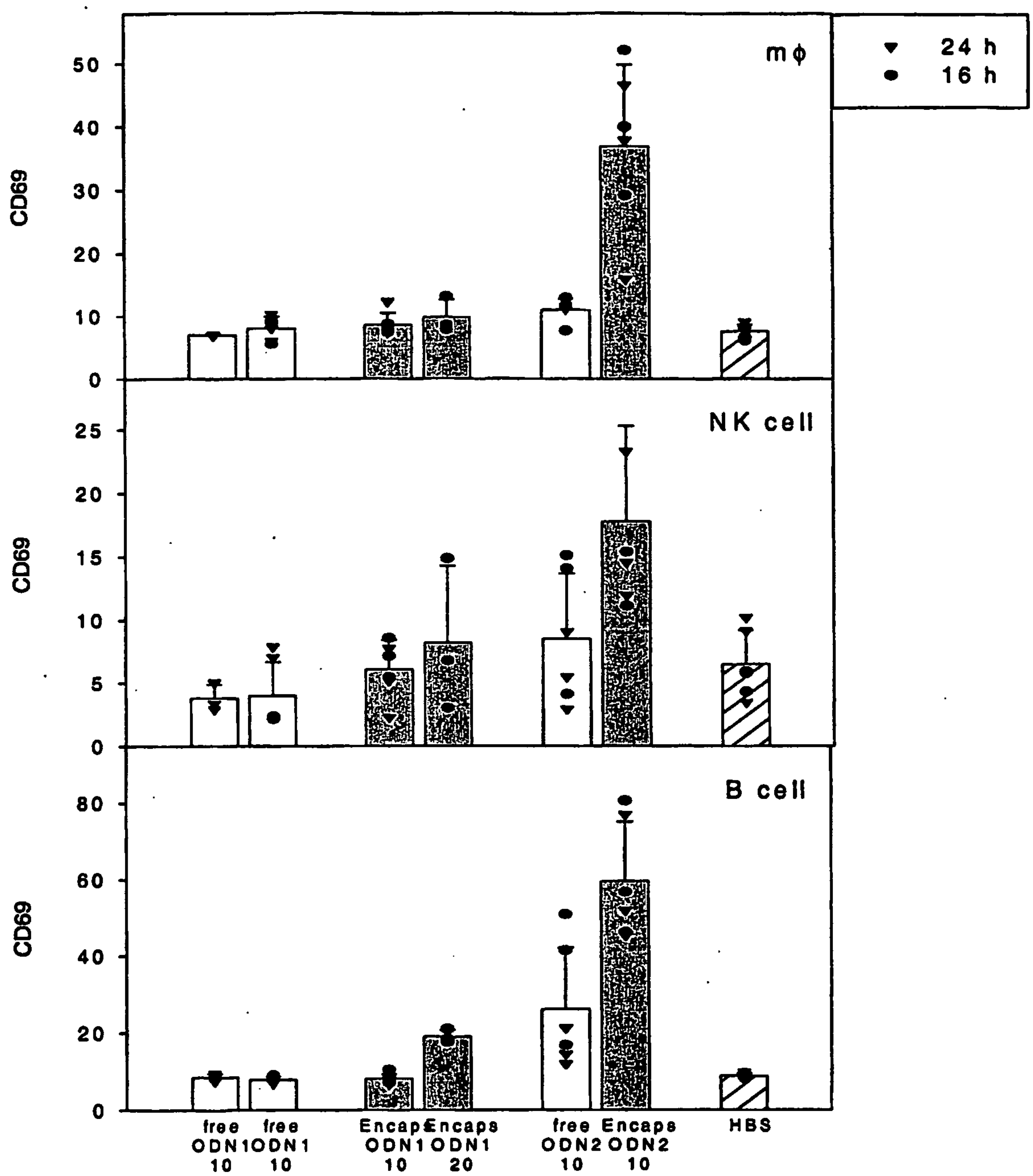
24 h
16 h
mϕ
NK cell
B cell
CD69
CD69
CD69
0
10
20
30
40
50
0
5
10
15
20
25
0
20
40
60
80
free ODN1 10
free ODN1 10
Encaps ODN1 10
Encaps ODN1 20
free ODN2 10
Encaps ODN2 10
HBS

Figure 2

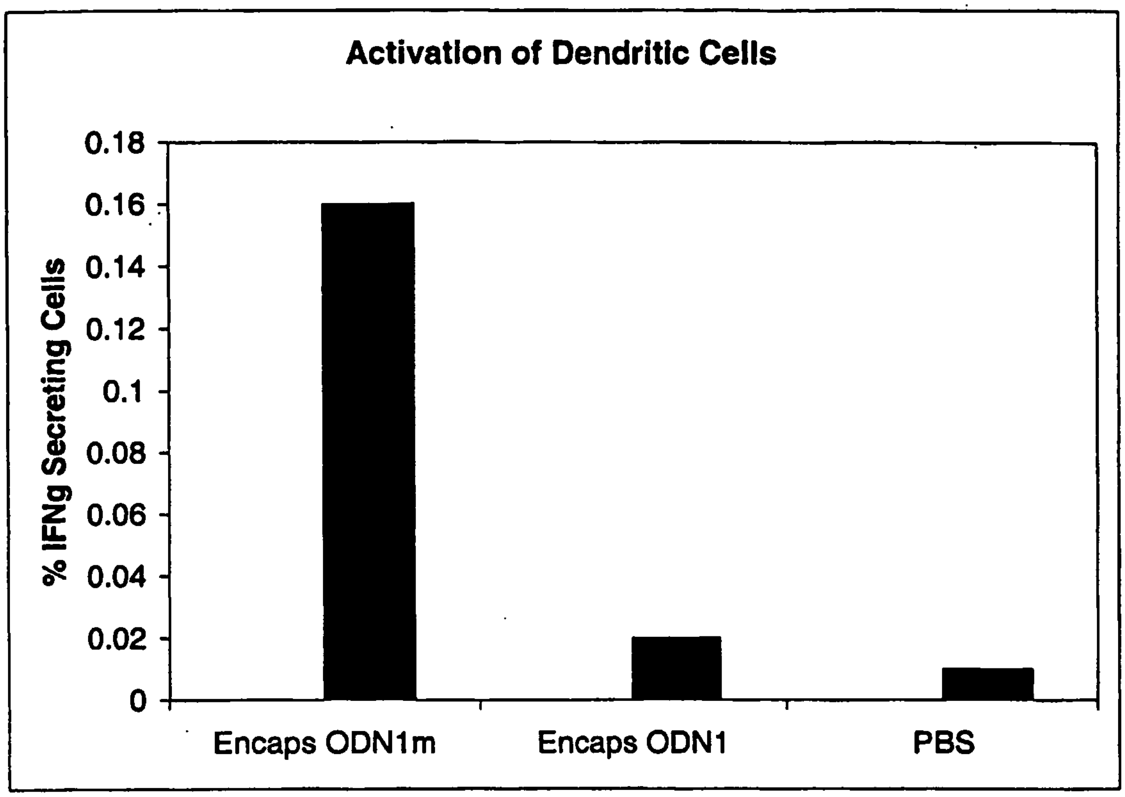
Activation of Dendritic Cells
% IFNg Secreting Cells
0.18
0.16
0.14
0.12
0.1
0.08
0.06
0.04
0.02
0
Encaps ODN1m
Encaps ODN1
PBS

Figure 3

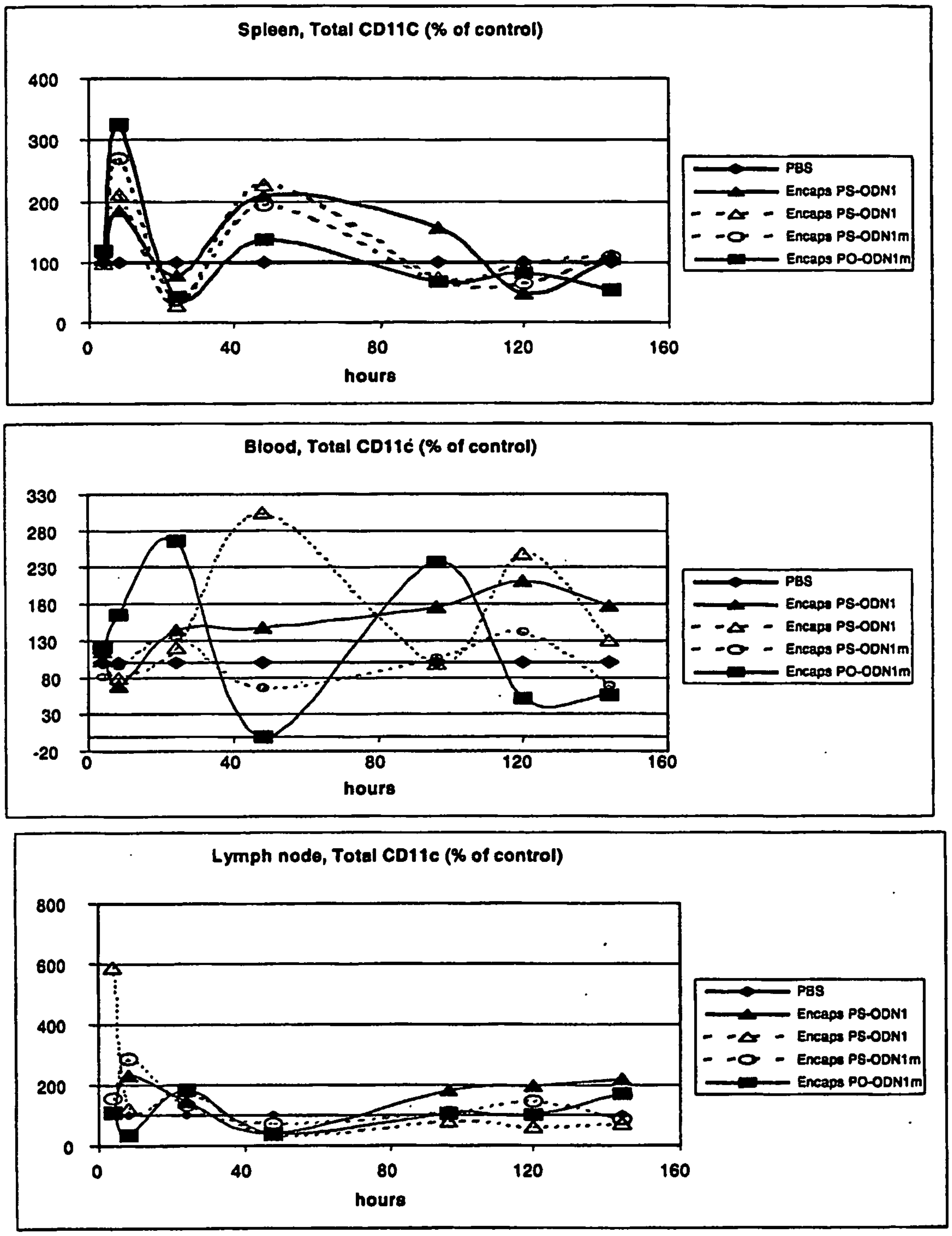
Spleen, Total CD11C (% of control)
400
300
200
100
0
0
40
80
120
160
hours
PBS
Encaps PS-ODN1
Encaps PS-ODN1
Encaps PS-ODN1m
Encaps PO-ODN1m
Blood, Total CD11c (% of control)
330
280
230
180
130
80
30
-20
0
40
80
120
160
hours
PBS
Encaps PS-ODN1
Encaps PS-ODN1
Encaps PS-ODN1m
Encaps PO-ODN1m
Lymph node, Total CD11c (% of control)
800
600
400
200
0
0
40
80
120
160
hours
PBS
Encaps PS-ODN1
Encaps PS-ODN1
Encaps PS-ODN1m
Encaps PO-ODN1m

Figure 4A

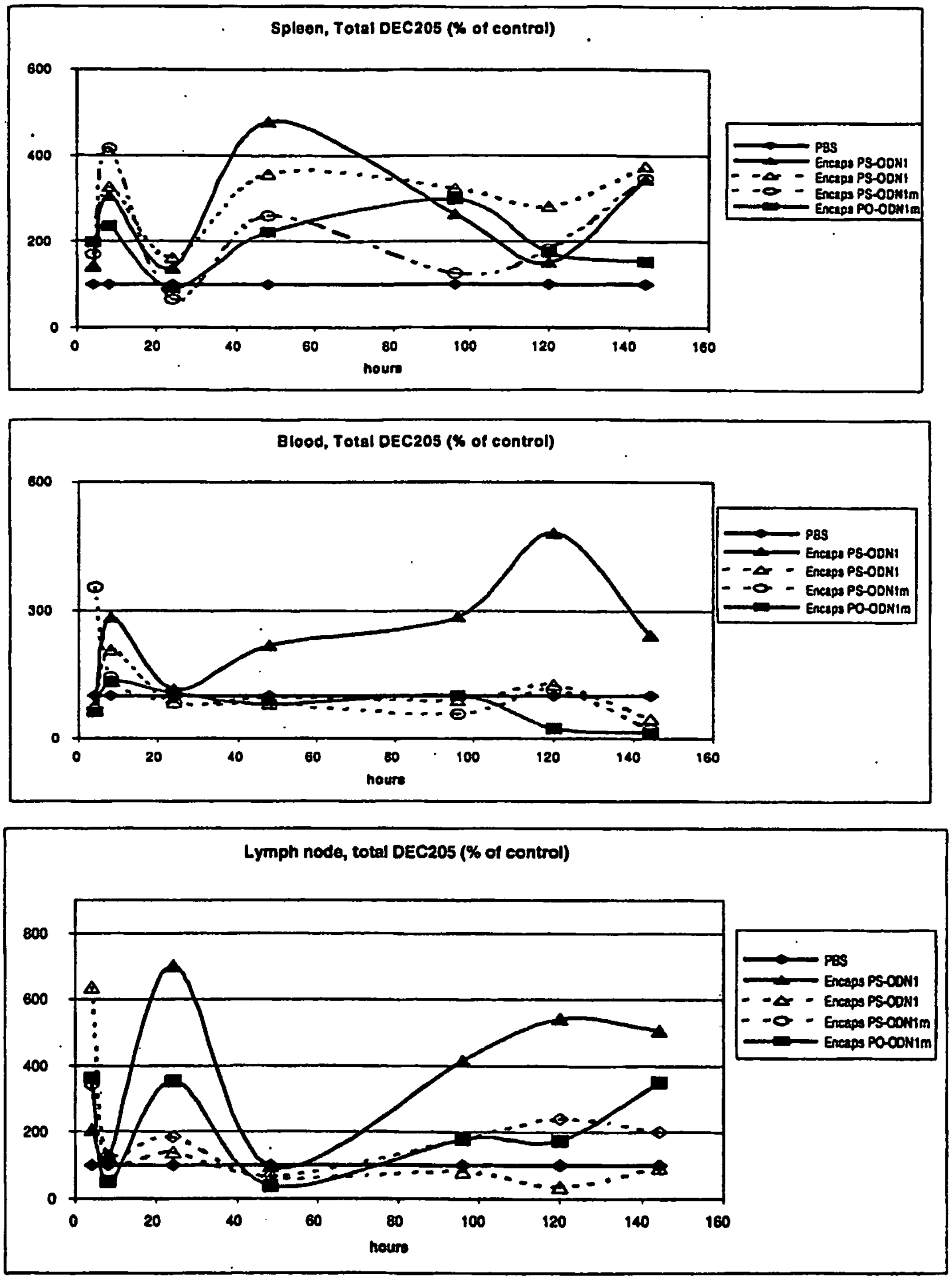
Spleen, Total DEC205 (% of control)
600
400
200
0
0 20 40 60 80 100 120 140 160
hours
PBS
Encaps PS-ODN1
Encaps PS-ODN1
Encaps PS-ODN1m
Encaps PO-ODN1m
Blood, Total DEC205 (% of control)
600
300
0
0 20 40 60 80 100 120 140 160
hours
PBS
Encaps PS-ODN1
Encaps PS-ODN1
Encaps PS-ODN1m
Encaps PO-ODN1m
Lymph node, total DEC205 (% of control)
800
600
400
200
0
0 20 40 60 80 100 120 140 160
hours
PBS
Encaps PS-ODN1
Encaps PS-ODN1
Encaps PS-ODN1m
Encaps PO-ODN1m

Figure 4B

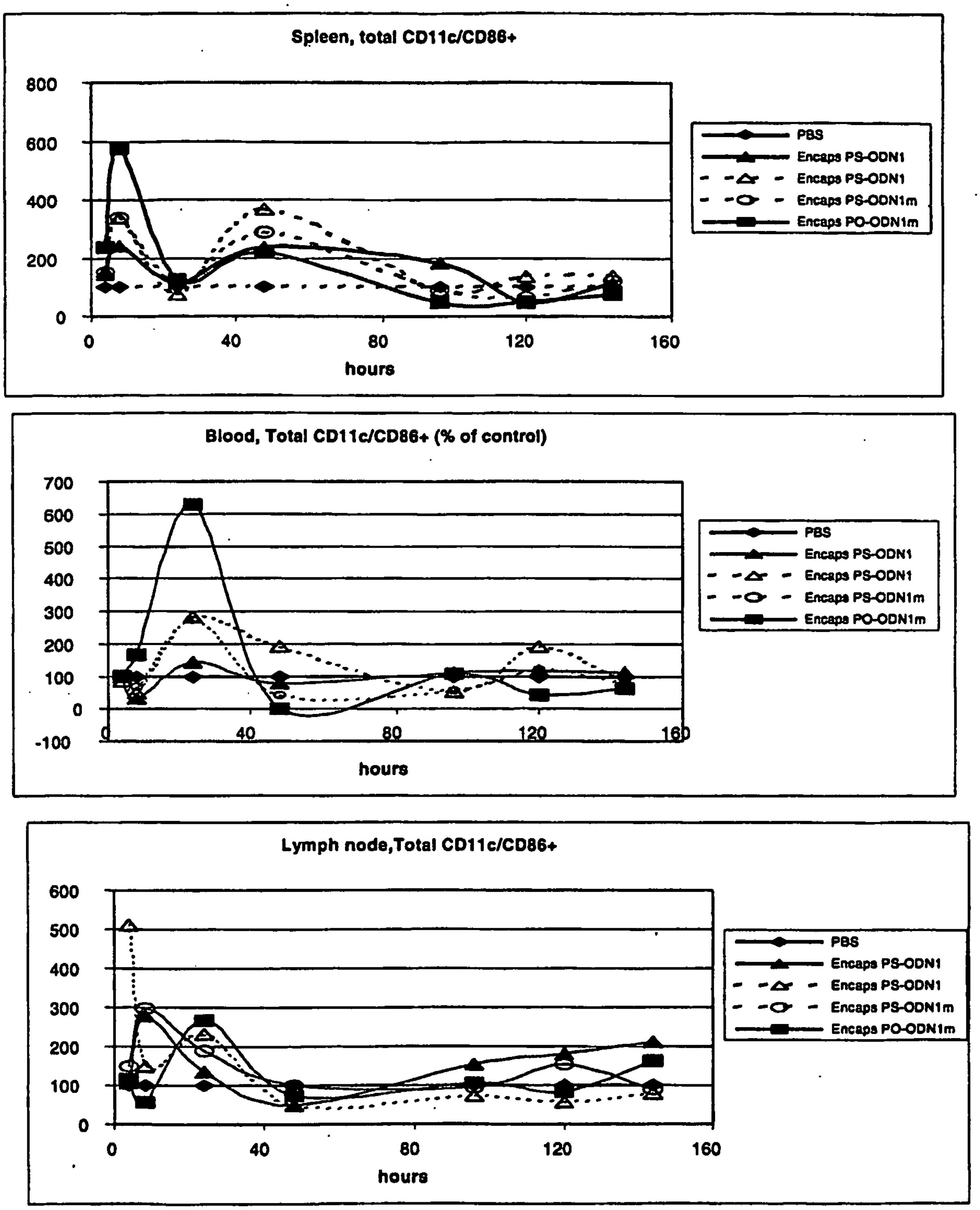
Spleen, total CD11c/CD86+
800
600
400
200
0
0
40
80
120
160
hours
PBS
Encaps PS-ODN1
Encaps PS-ODN1
Encaps PS-ODN1m
Encaps PO-ODN1m
Blood, Total CD11c/CD86+ (% of control)
700
600
500
400
300
200
100
0
-100
0
40
80
120
160
hours
PBS
Encaps PS-ODN1
Encaps PS-ODN1
Encaps PS-ODN1m
Encaps PO-ODN1m
Lymph node,Total CD11c/CD86+
600
500
400
300
200
100
0
0
40
80
120
160
hours
PBS
Encaps PS-ODN1
Encaps PS-ODN1
Encaps PS-ODN1m
Encaps PO-ODN1m

Figure 5A

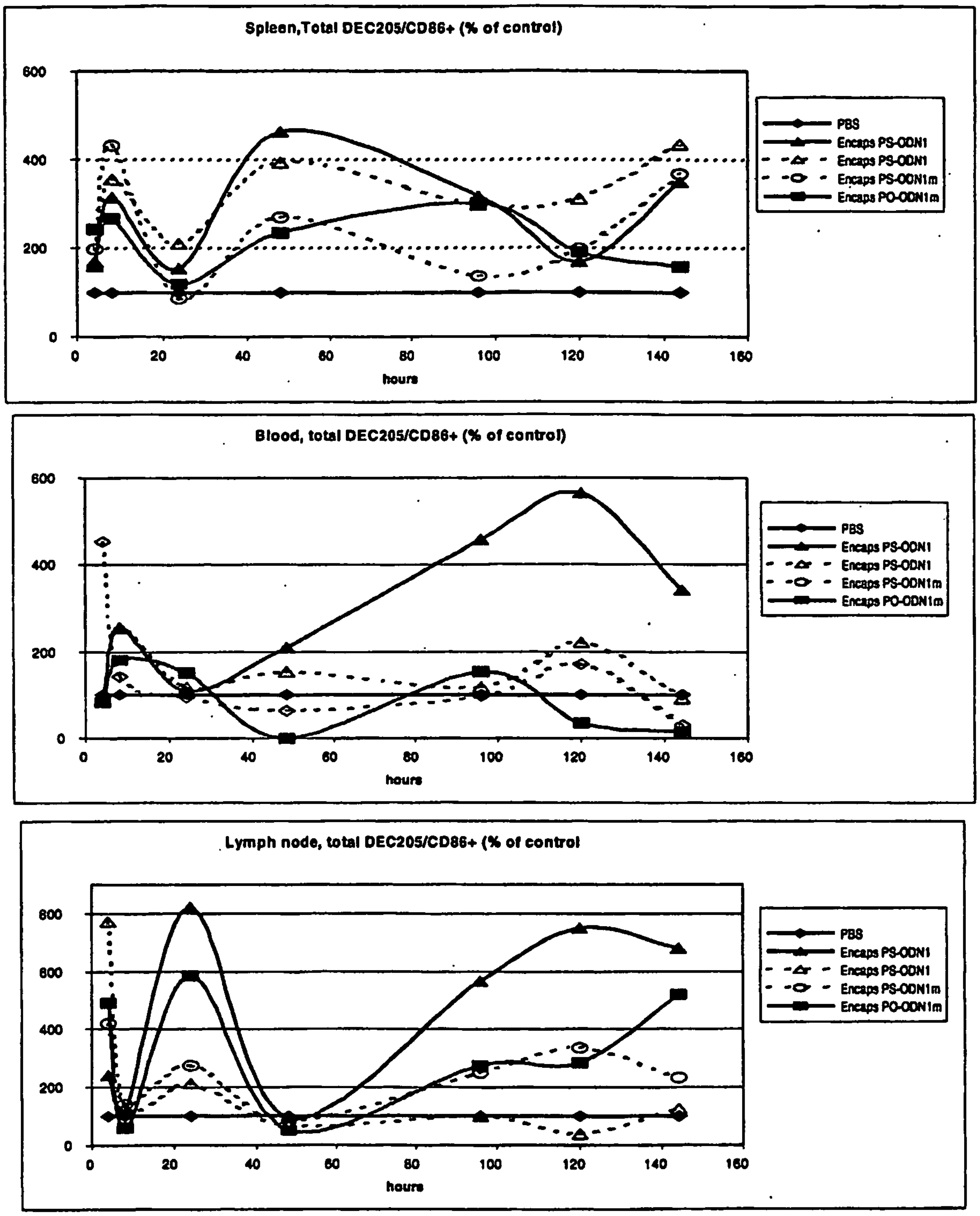
Spleen,Total DEC205/CD86+ (% of control)
0
200
400
600
0
20
40
60
80
100
120
140
160
hours
PBS
Encaps PS-ODN1
Encaps PS-ODN1
Encaps PS-ODN1m
Encaps PO-ODN1m
Blood, total DEC205/CD86+ (% of control)
0
200
400
600
0
20
40
60
80
100
120
140
160
hours
PBS
Encaps PS-ODN1
Encaps PS-ODN1
Encaps PS-ODN1m
Encaps PO-ODN1m
Lymph node, total DEC205/CD86+ (% of control
0
200
400
600
800
0
20
40
60
80
100
120
140
160
hours
PBS
Encaps PS-ODN1
Encaps PS-ODN1
Encaps PS-ODN1m
Encaps PO-ODN1m

Figure 5B

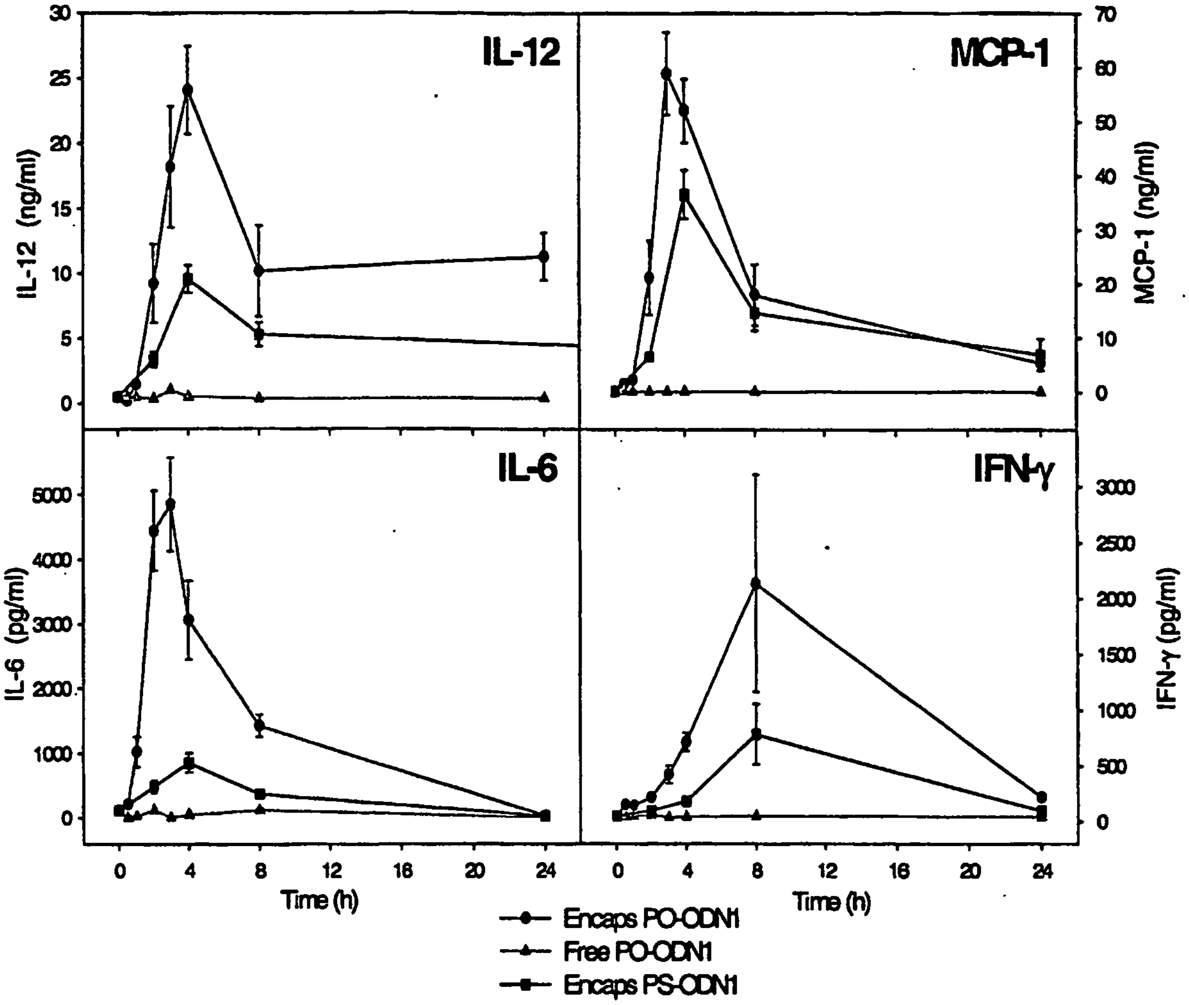
IL-12
MCP-1
IL-6
IFN-γ
IL-12 (ng/ml)
MCP-1 (ng/ml)
IL-6 (pg/ml)
IFN-γ (pg/ml)
Time (h)
Time (h)
Encaps PO-ODN1
Free PO-ODN1
Encaps PS-ODN1

Figure 6

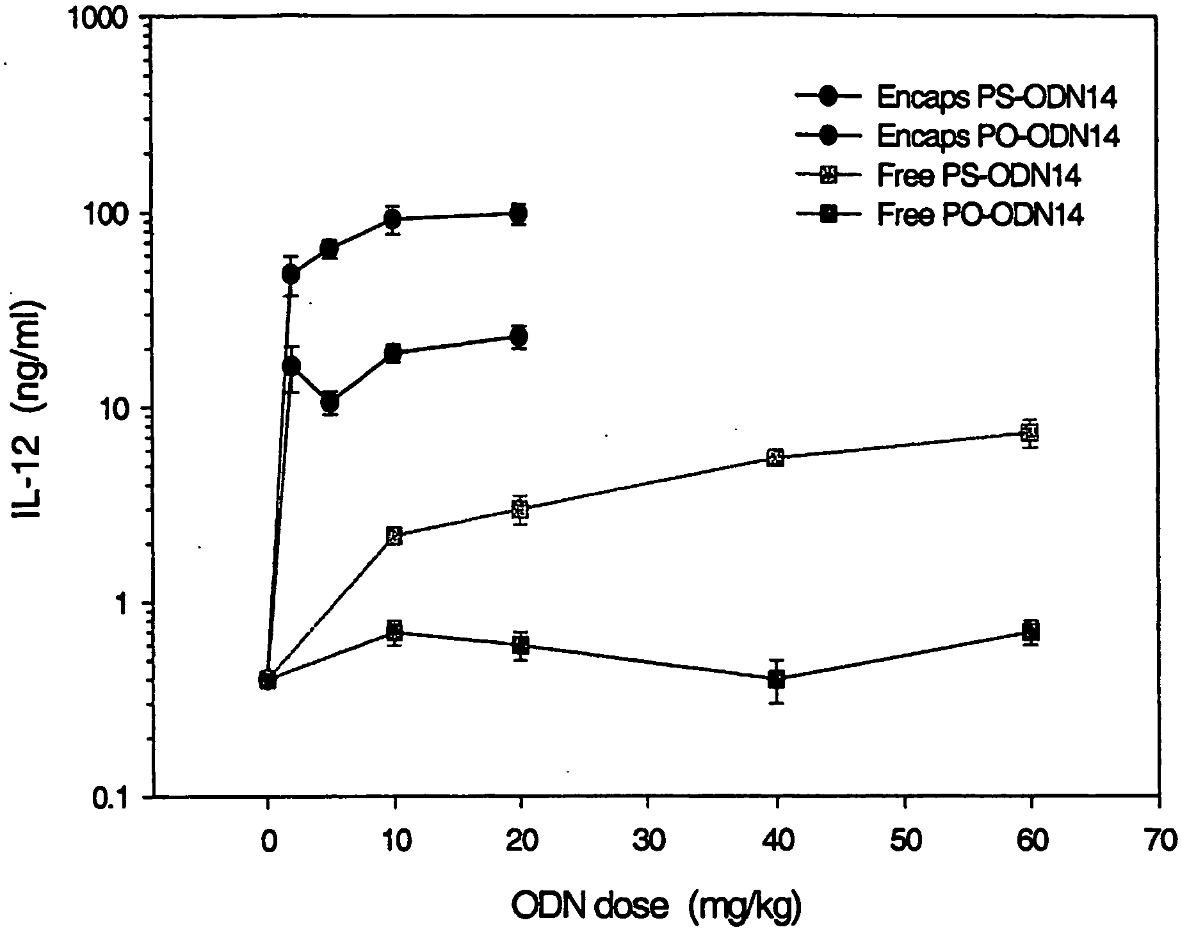
1000
100
10
1
0.1
IL-12 (ng/ml)
Encaps PS-ODN14
Encaps PO-ODN14
Free PS-ODN14
Free PO-ODN14
0
10
20
30
40
50
60
70
ODN dose (mg/kg)

Figure 7A

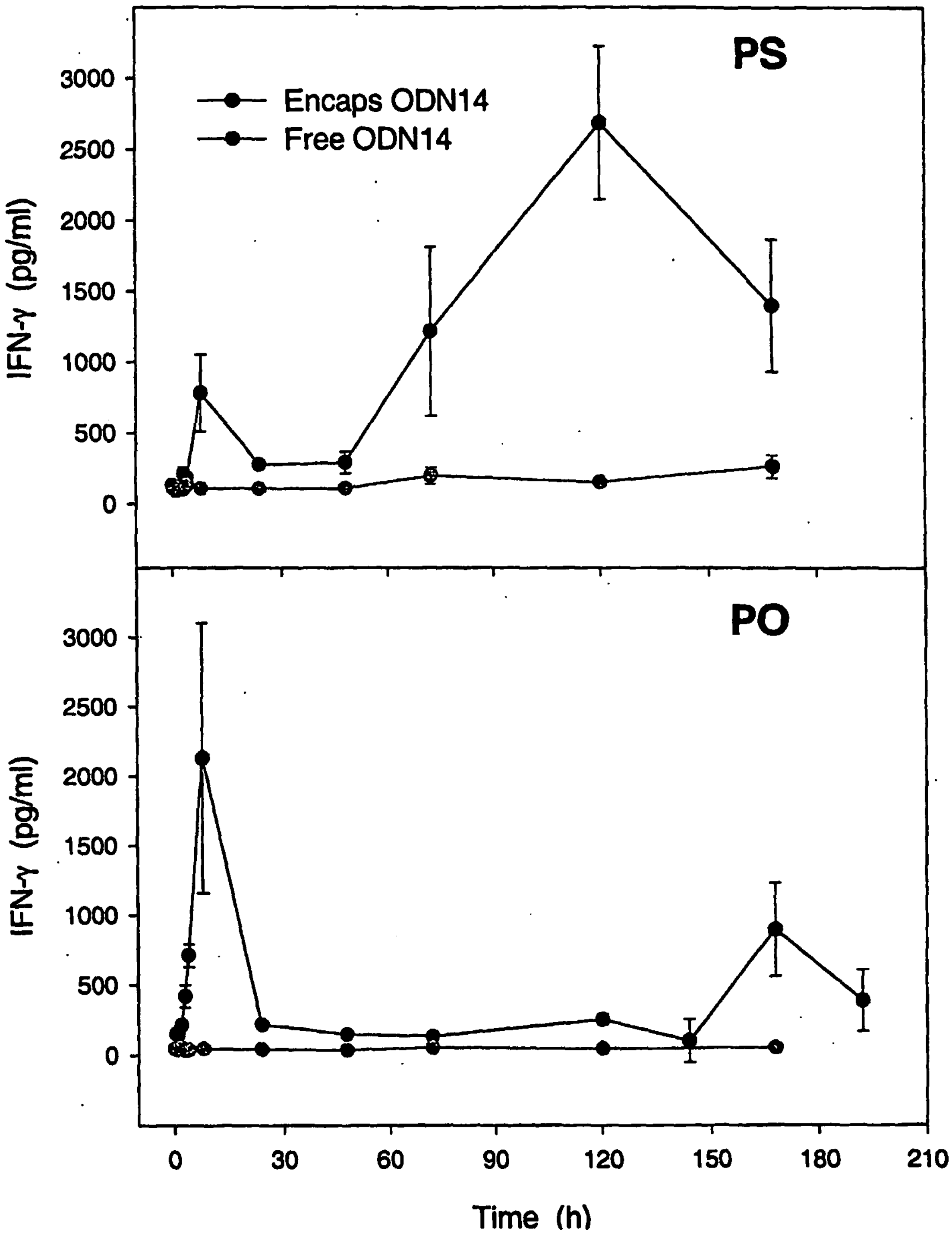
PS
Encaps ODN14
Free ODN14
IFN-γ (pg/ml)
3000
2500
2000
1500
1000
500
0
PO
IFN-γ (pg/ml)
3000
2500
2000
1500
1000
500
0
0
30
60
90
120
150
180
210
Time (h)

Figure 7B

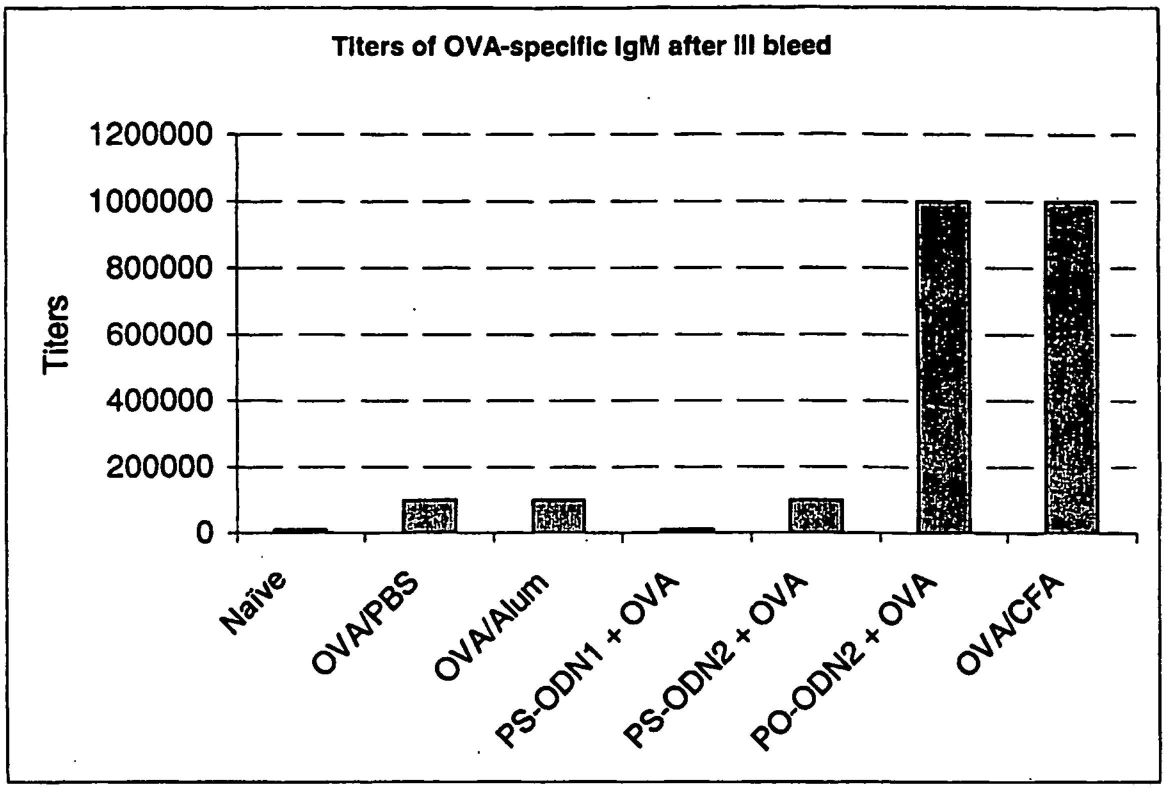
Titers of OVA-specific IgM after III bleed
1200000
1000000
800000
600000
400000
200000
0
Titers
Naïve
OVA/PBS
OVA/Alum
PS-ODN1 + OVA
PS-ODN2 + OVA
PO-ODN2 + OVA
OVA/CFA

Figure 8

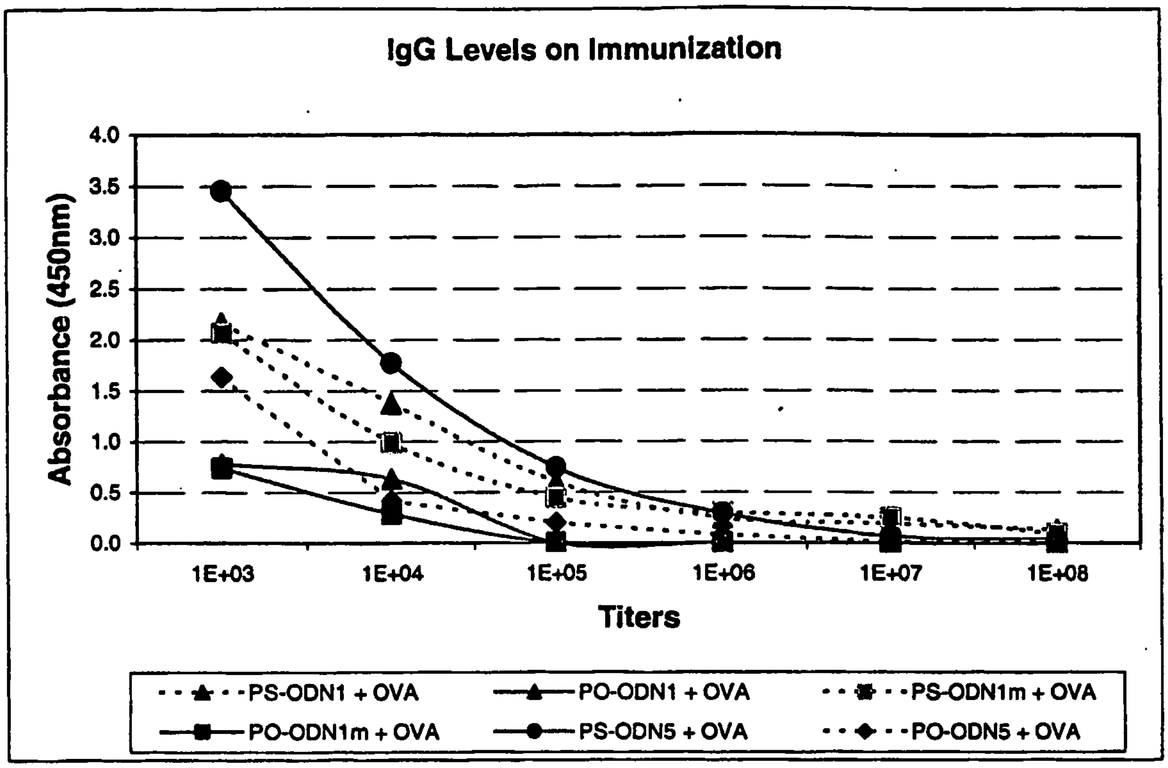
IgG Levels on Immunization
Absorbance (450nm)
4.0
3.5
3.0
2.5
2.0
1.5
1.0
0.5
0.0
1E+03
1E+04
1E+05
1E+06
1E+07
1E+08
Titers
PS-ODN1 + OVA
PO-ODN1 + OVA
PS-ODN1m + OVA
PO-ODN1m + OVA
PS-ODN5 + OVA
PO-ODN5 + OVA

Figure 9

(a) (b) (c)

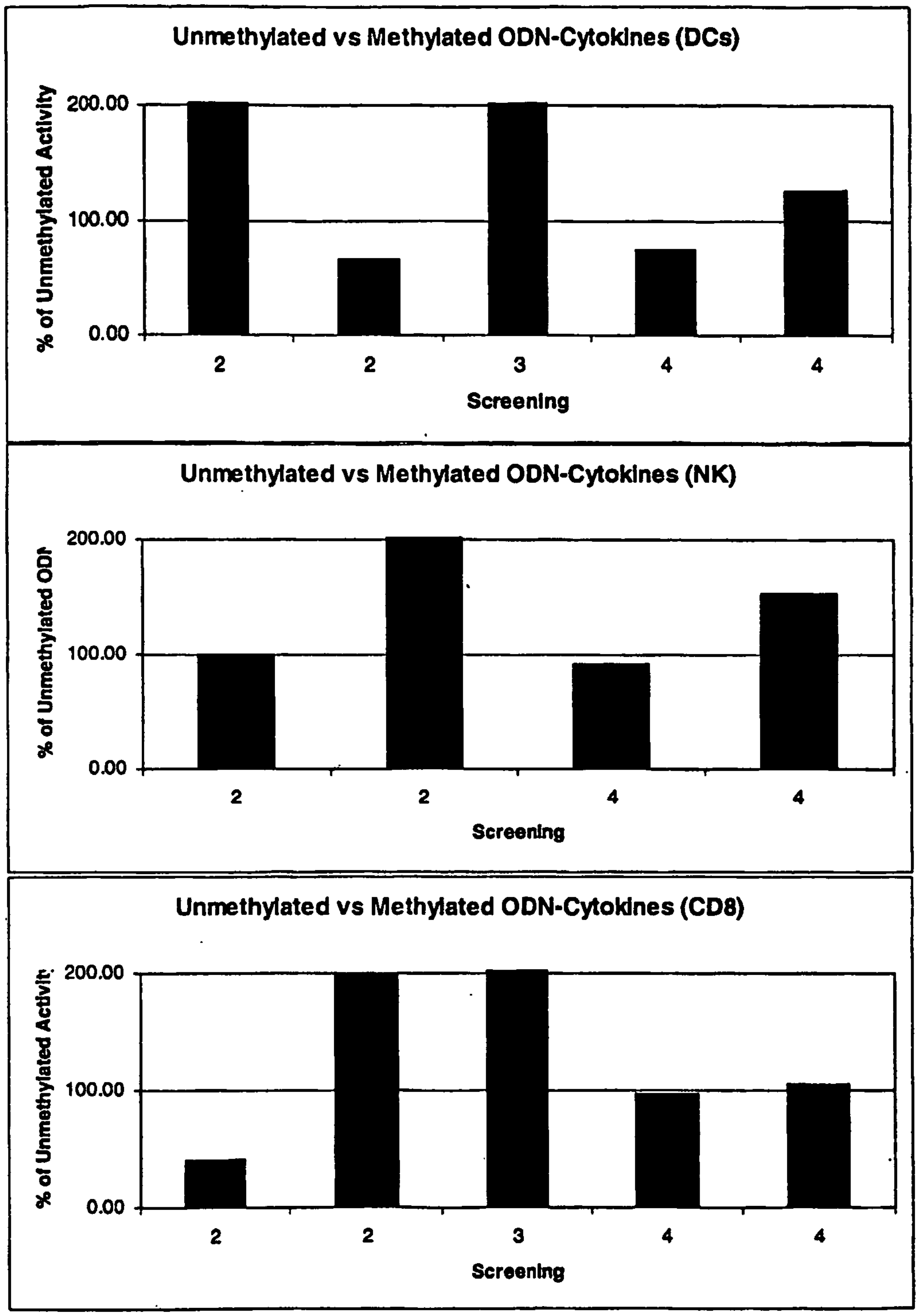


Figure 10

(a)

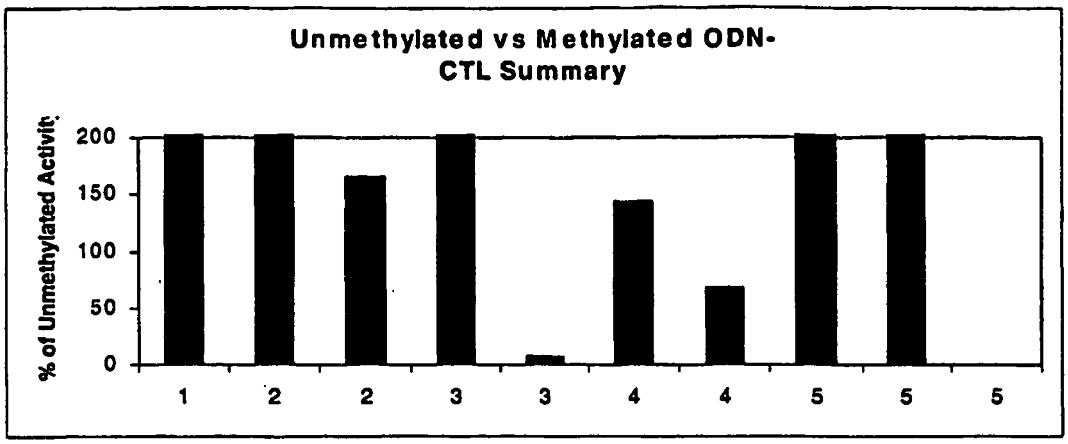


(b)

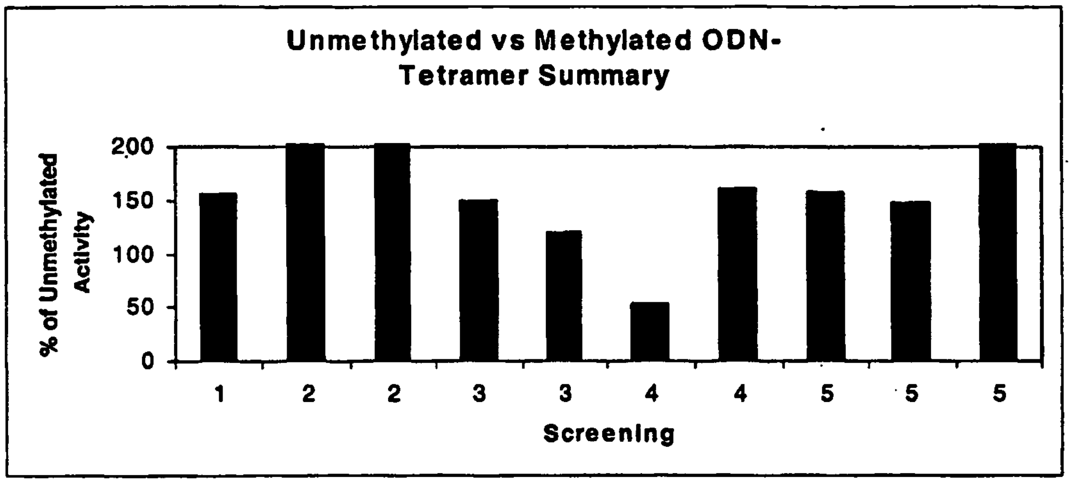


(c)

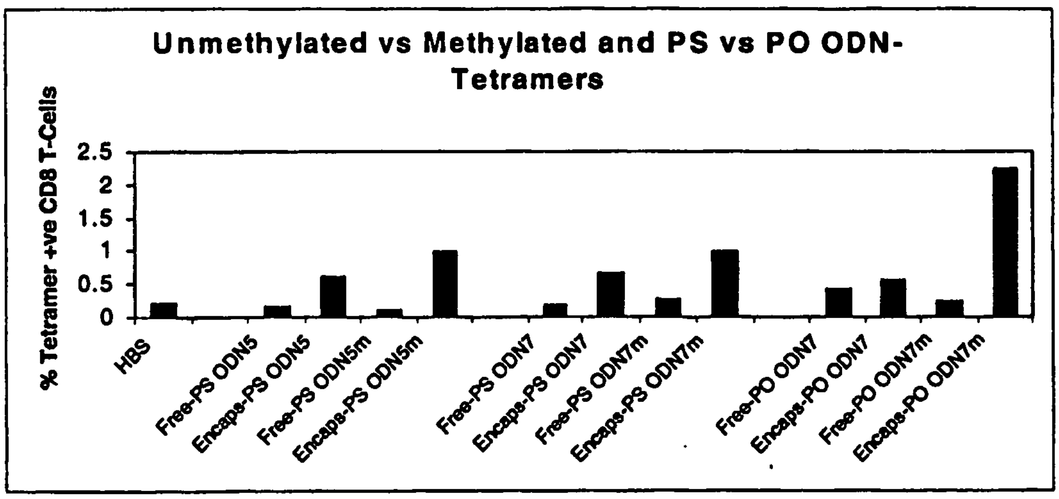


Figure 11

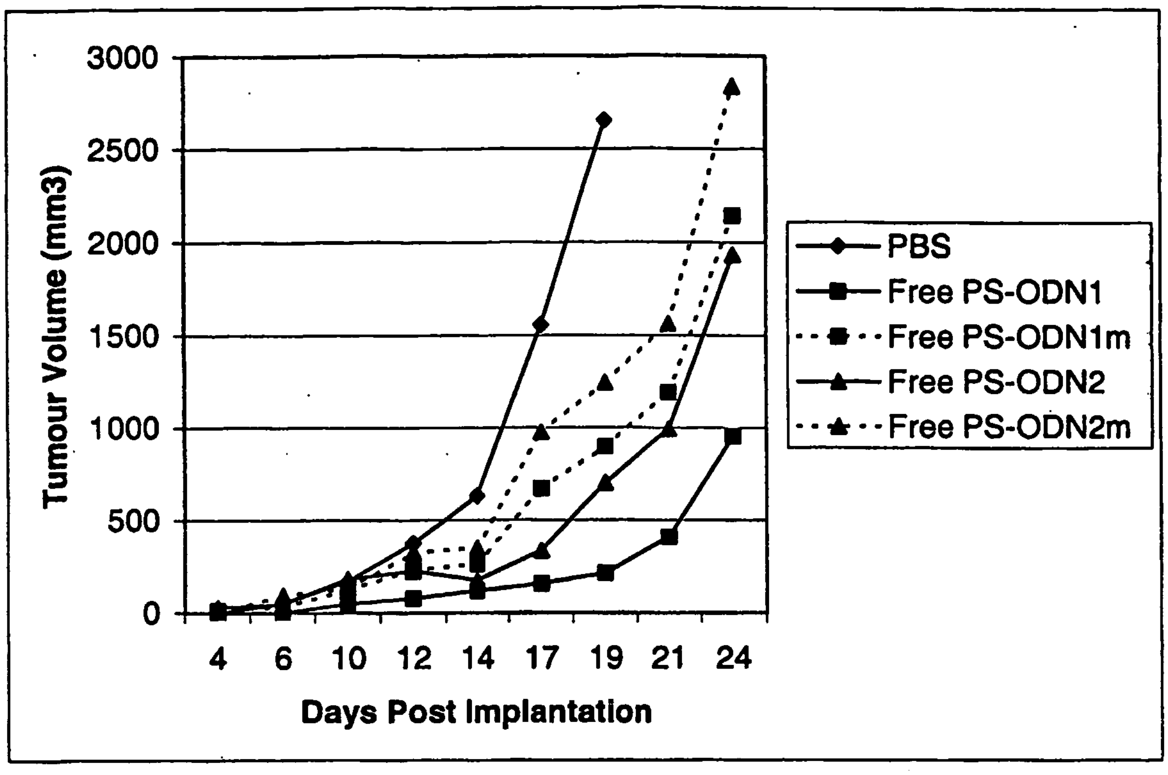
3000
2500
2000
1500
1000
500
0
Tumour Volume (mm3)
4
6
10
12
14
17
19
21
24
Days Post Implantation
PBS
Free PS-ODN1
Free PS-ODN1m
Free PS-ODN2
Free PS-ODN2m

Figure 12

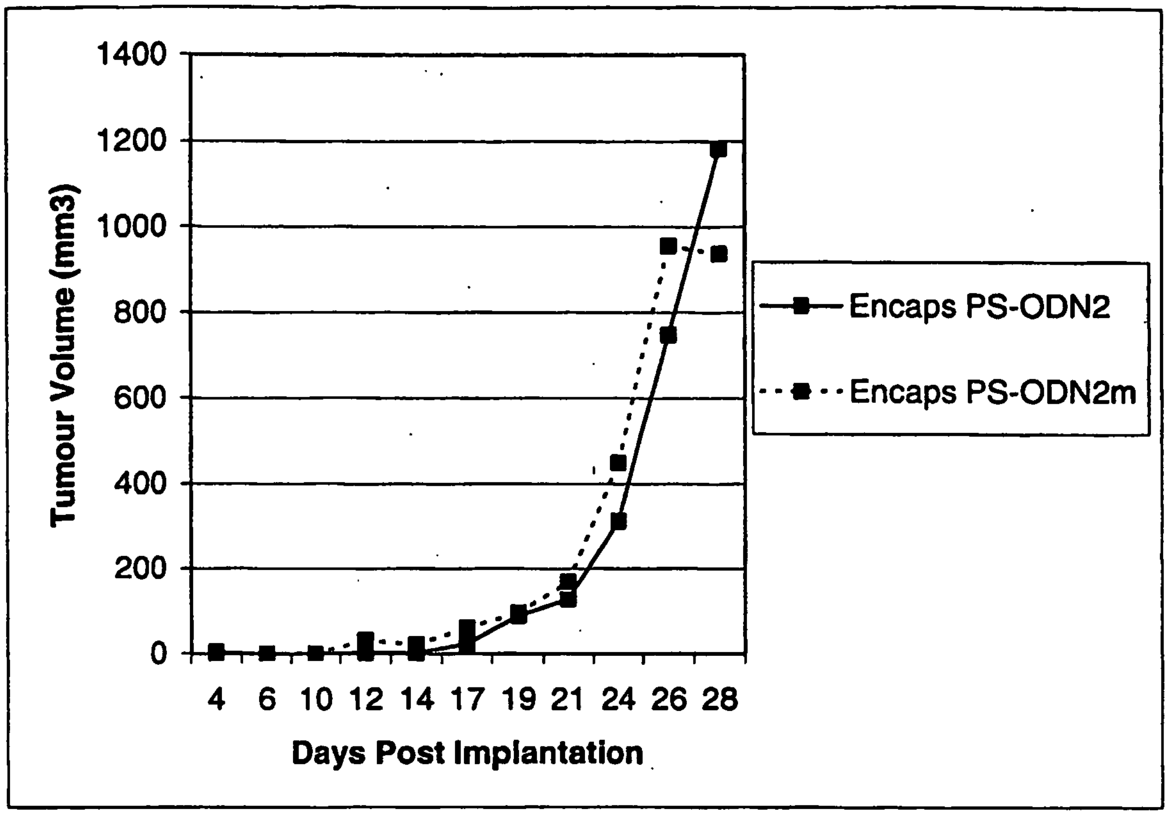
1400
1200
1000
800
600
400
200
0
Tumour Volume (mm3)
4 6 10 12 14 17 19 21 24 26 28
Days Post Implantation
Encaps PS-ODN2
Encaps PS-ODN2m

Figure 13

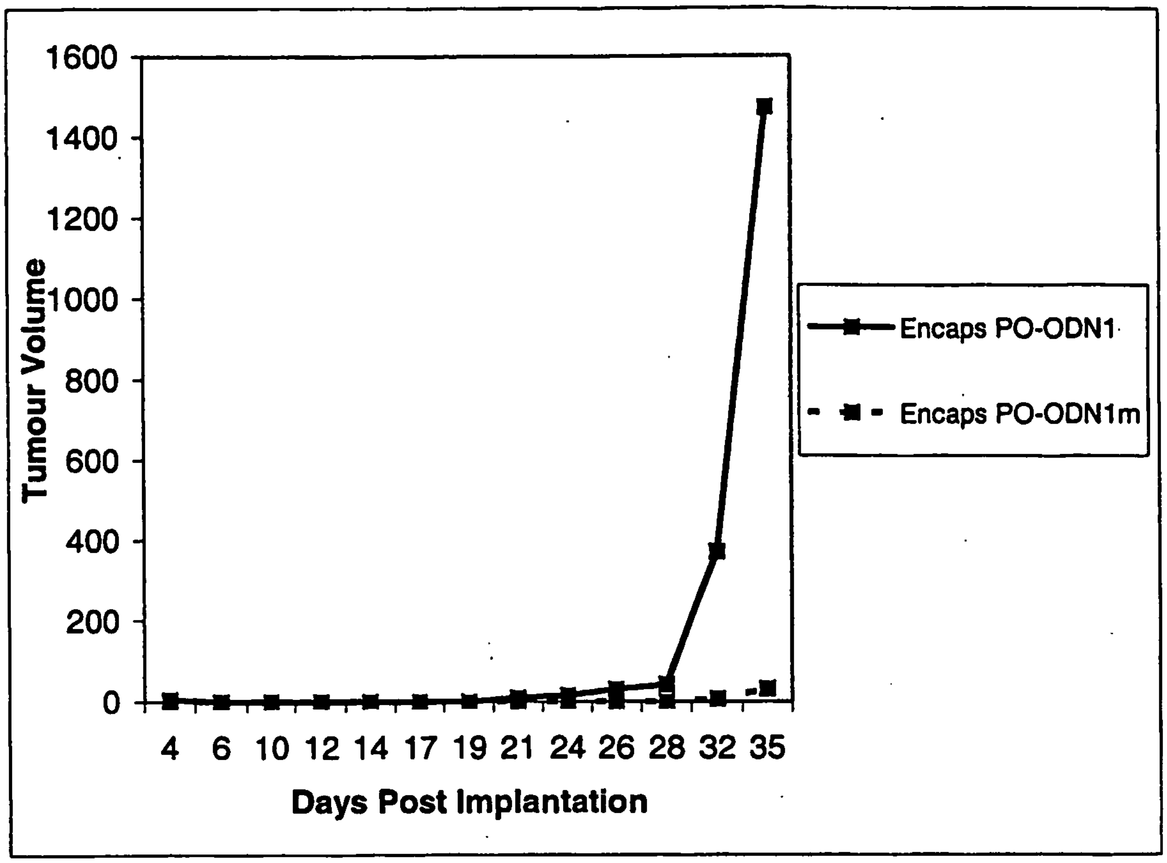
1600
1400
1200
1000
800
600
400
200
0
Tumour Volume
4 6 10 12 14 17 19 21 24 26 28 32 35
Days Post Implantation
Encaps PO-ODN1
Encaps PO-ODN1m

Figure 14

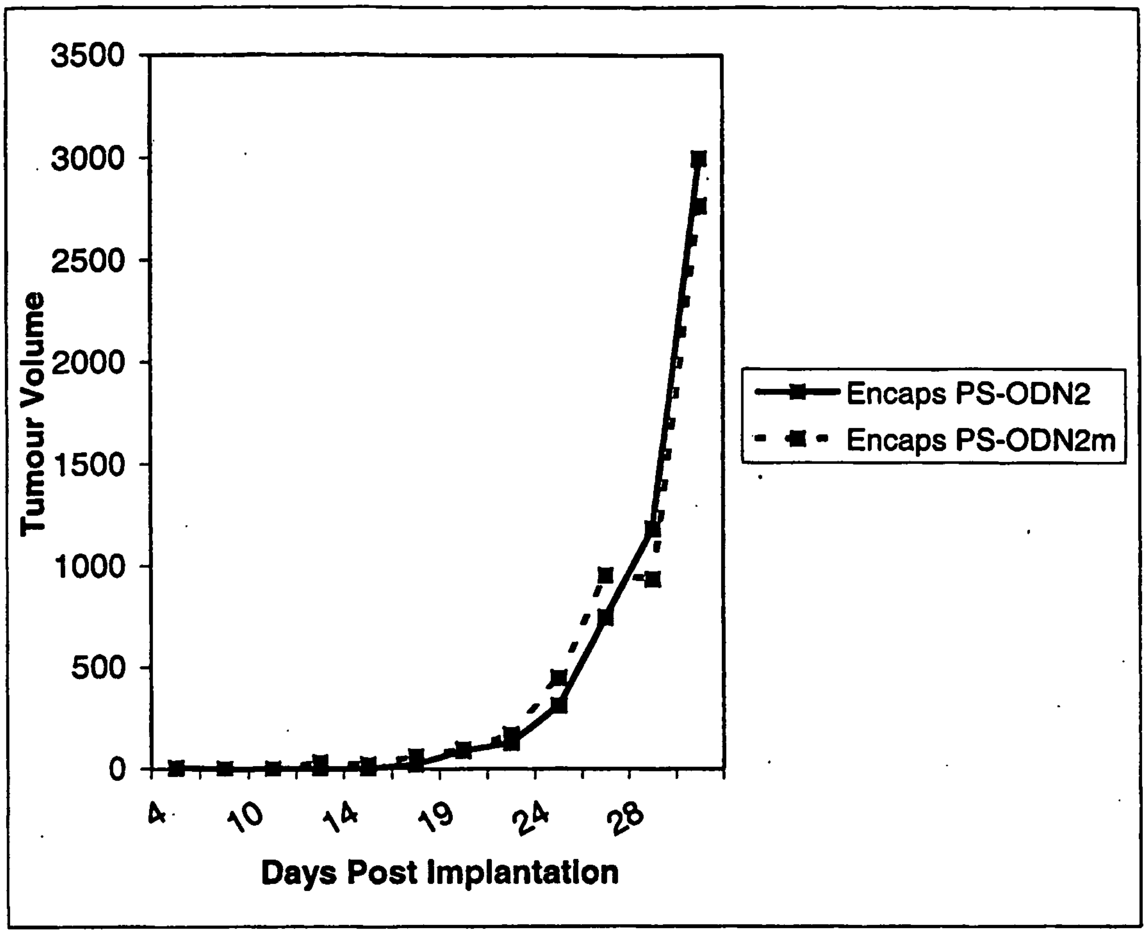
3500
3000
2500
2000
1500
1000
500
0
Tumour Volume
4
10
14
19
24
28
Days Post Implantation
Encaps PS-ODN2
Encaps PS-ODN2m

Figure 15

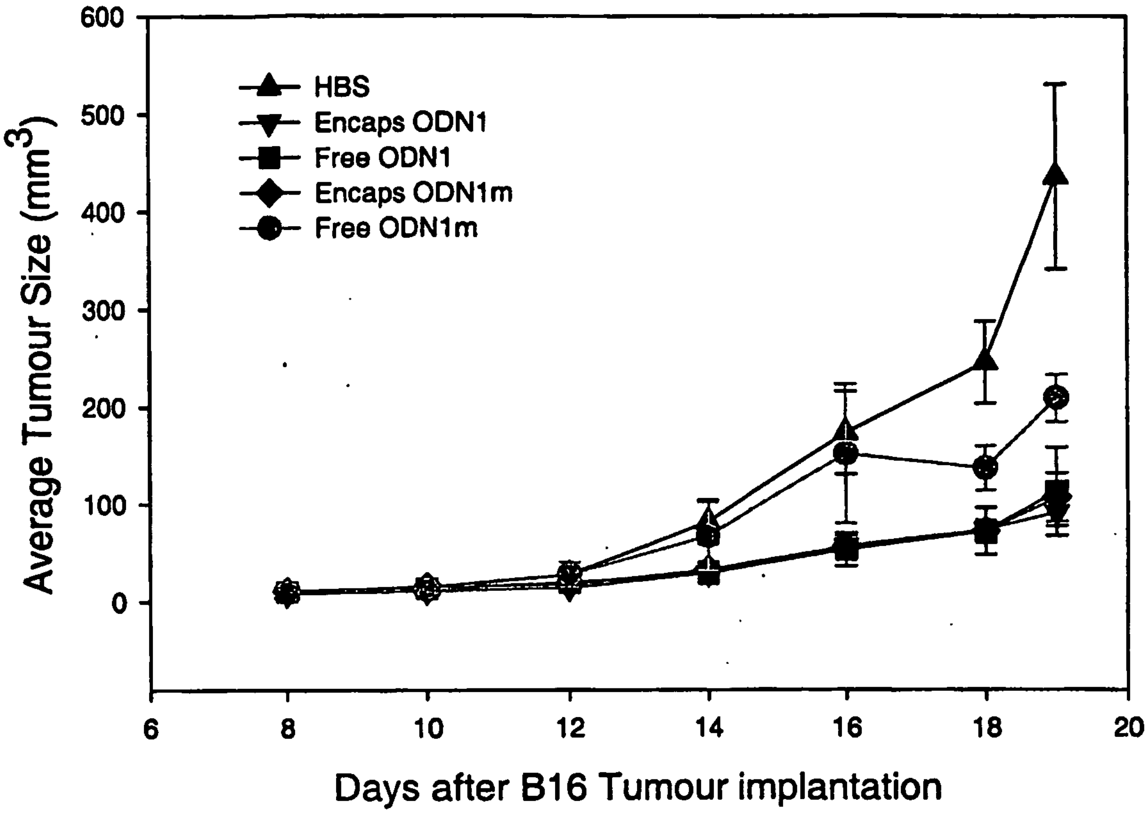
HBS
Encaps ODN1
Free ODN1
Encaps ODN1m
Free ODN1m
Average Tumour Size (mm3)
600
500
400
300
200
100
0
6
8
10
12
14
16
18
20
Days after B16 Tumour implantation

Figure 16

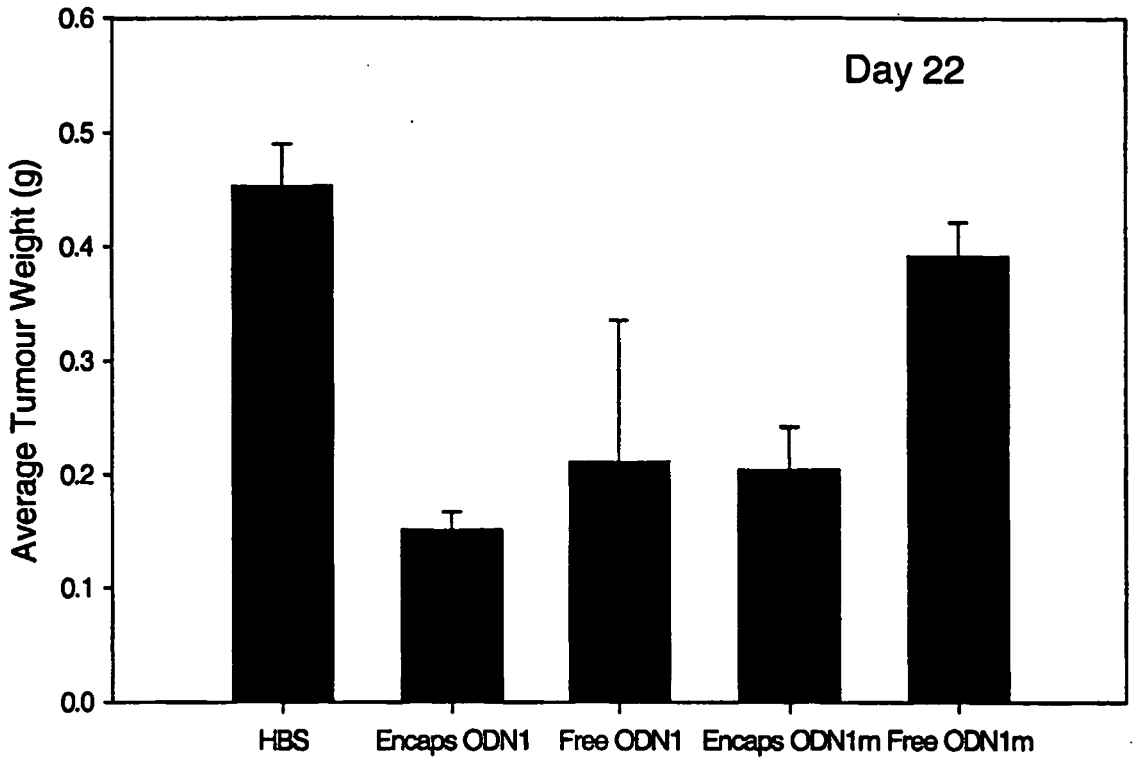
Day 22
Average Tumour Weight (g)
0.6
0.5
0.4
0.3
0.2
0.1
0.0
HBS
Encaps ODN1
Free ODN1
Encaps ODN1m
Free ODN1m

Figure 17

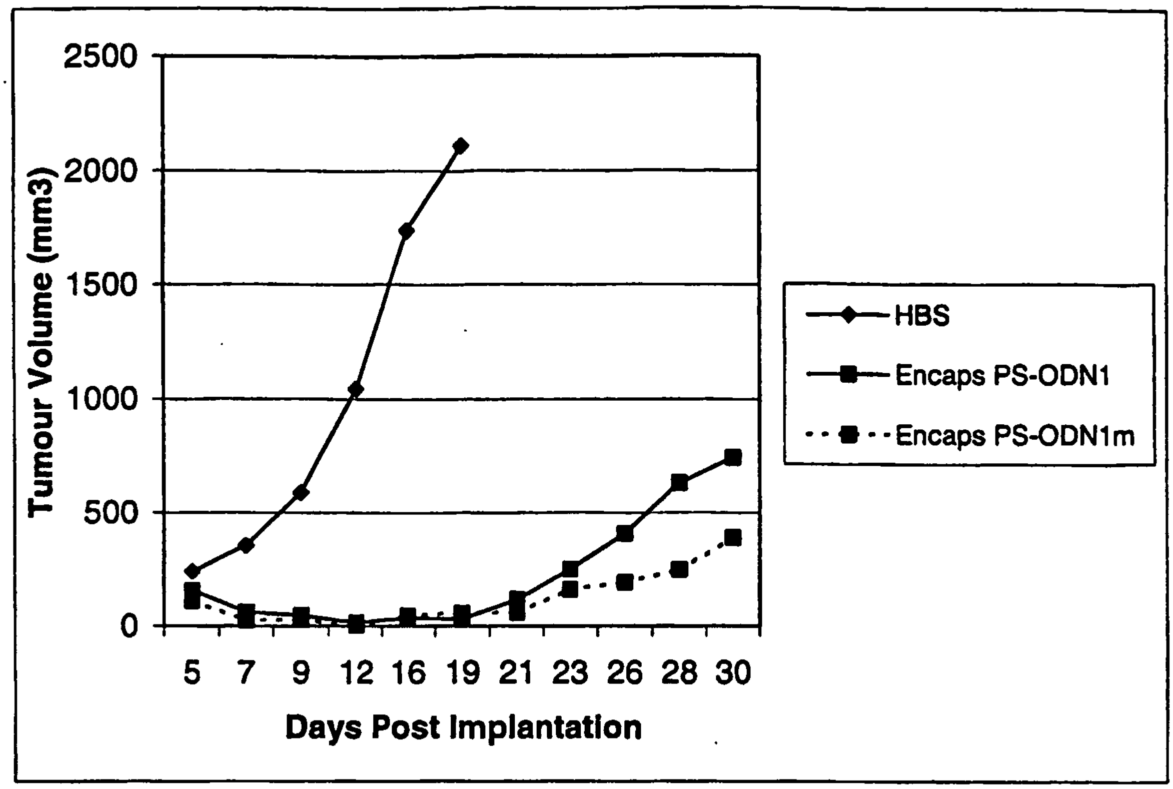
2500
2000
1500
1000
500
0
Tumour Volume (mm3)
5 7 9 12 16 19 21 23 26 28 30
Days Post Implantation
HBS
Encaps PS-ODN1
Encaps PS-ODN1m

Figure 18

(a)

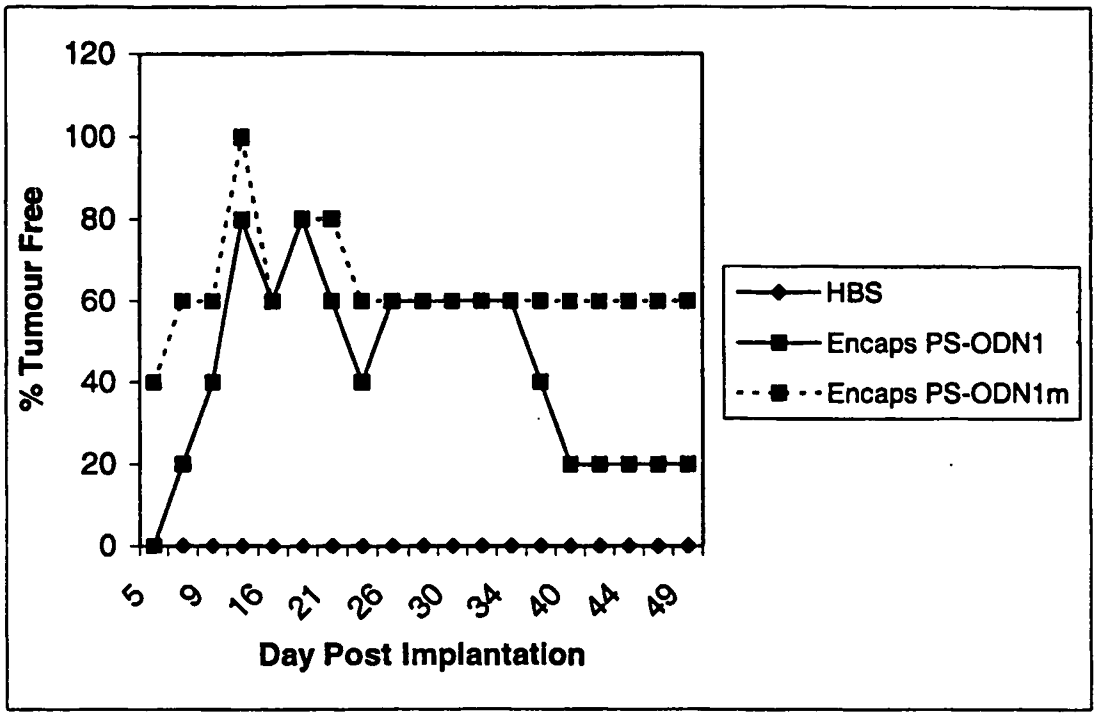
120
100
80
60
40
20
0
% Tumour Free
5
9
16
21
26
30
34
40
44
49
Day Post Implantation
HBS
Encaps PS-ODN1
Encaps PS-ODN1m

(b)

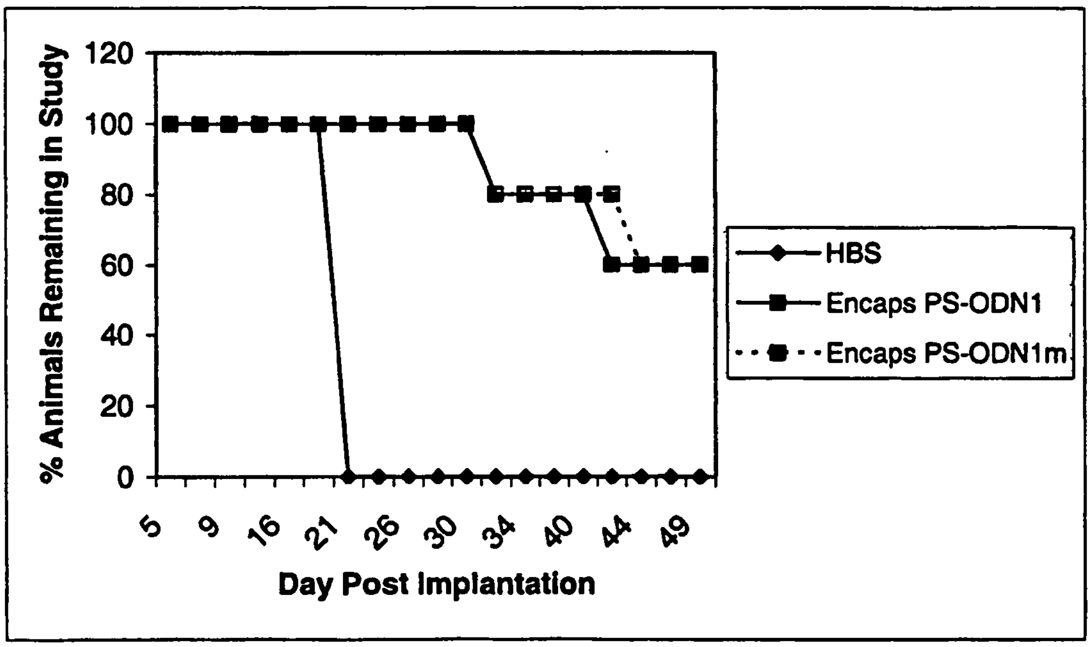
120
100
80
60
40
20
0
% Animals Remaining in Study
5
9
16
21
26
30
34
40
44
49
Day Post Implantation
HBS
Encaps PS-ODN1
Encaps PS-ODN1m

Figure 19

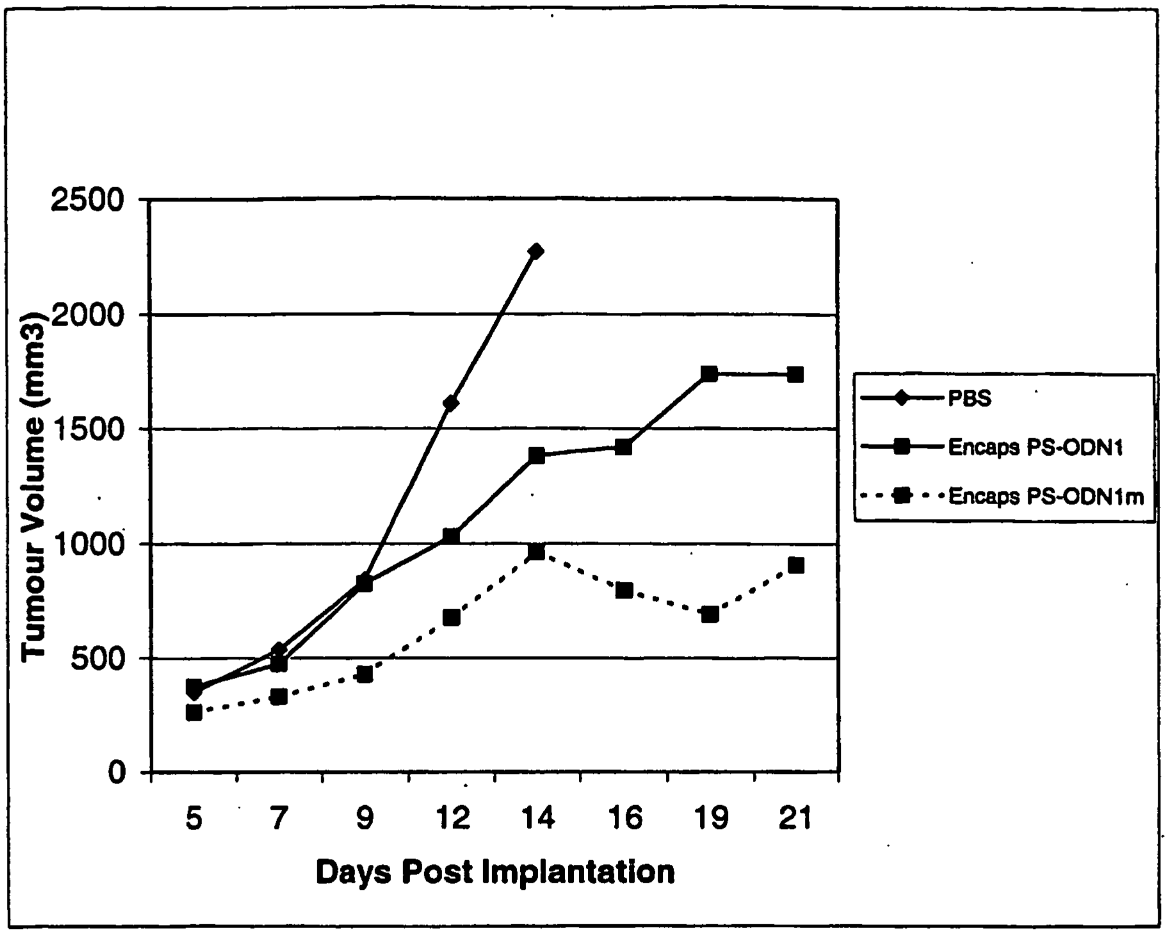
2500
2000
1500
1000
500
0
Tumour Volume (mm3)
5
7
9
12
14
16
19
21
Days Post Implantation
PBS
Encaps PS-ODN1
Encaps PS-ODN1m

Figure 20

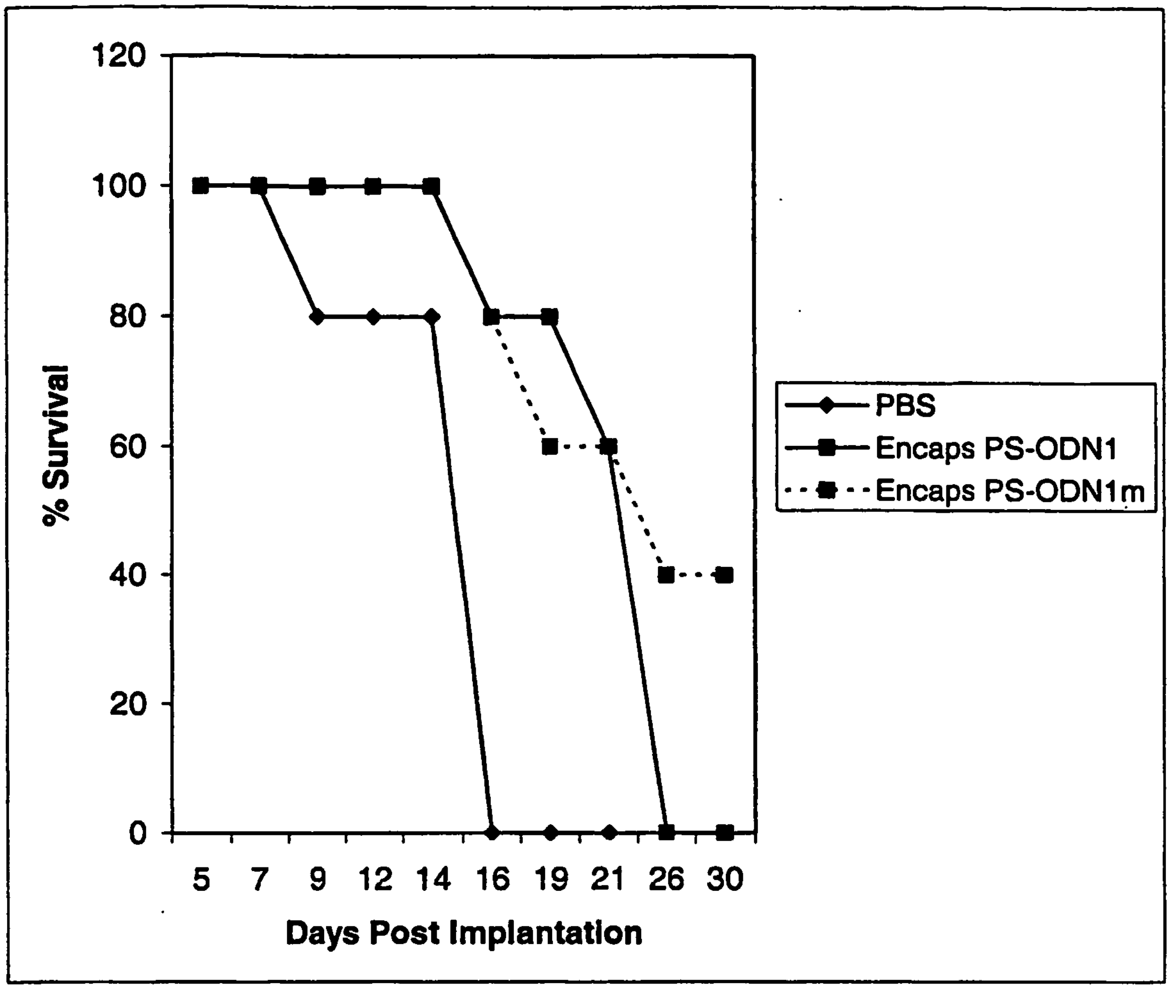
120
100
80
60
40
20
0
% Survival
5 7 9 12 14 16 19 21 26 30
Days Post Implantation
PBS
Encaps PS-ODN1
Encaps PS-ODN1m

Figure 21

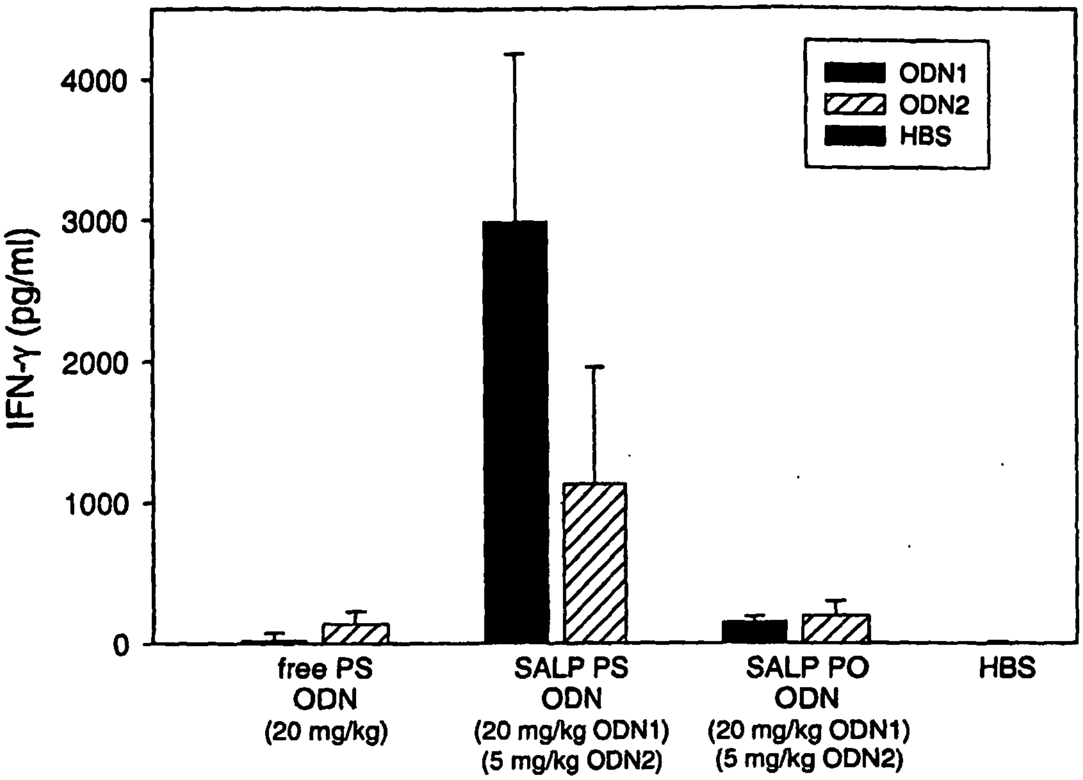
IFN-γ (pg/ml)
4000
3000
2000
1000
0
ODN1
ODN2
HBS
free PS ODN (20 mg/kg)
SALP PS ODN (20 mg/kg ODN1) (5 mg/kg ODN2)
SALP PO ODN (20 mg/kg ODN1) (5 mg/kg ODN2)
HBS

Figure 22

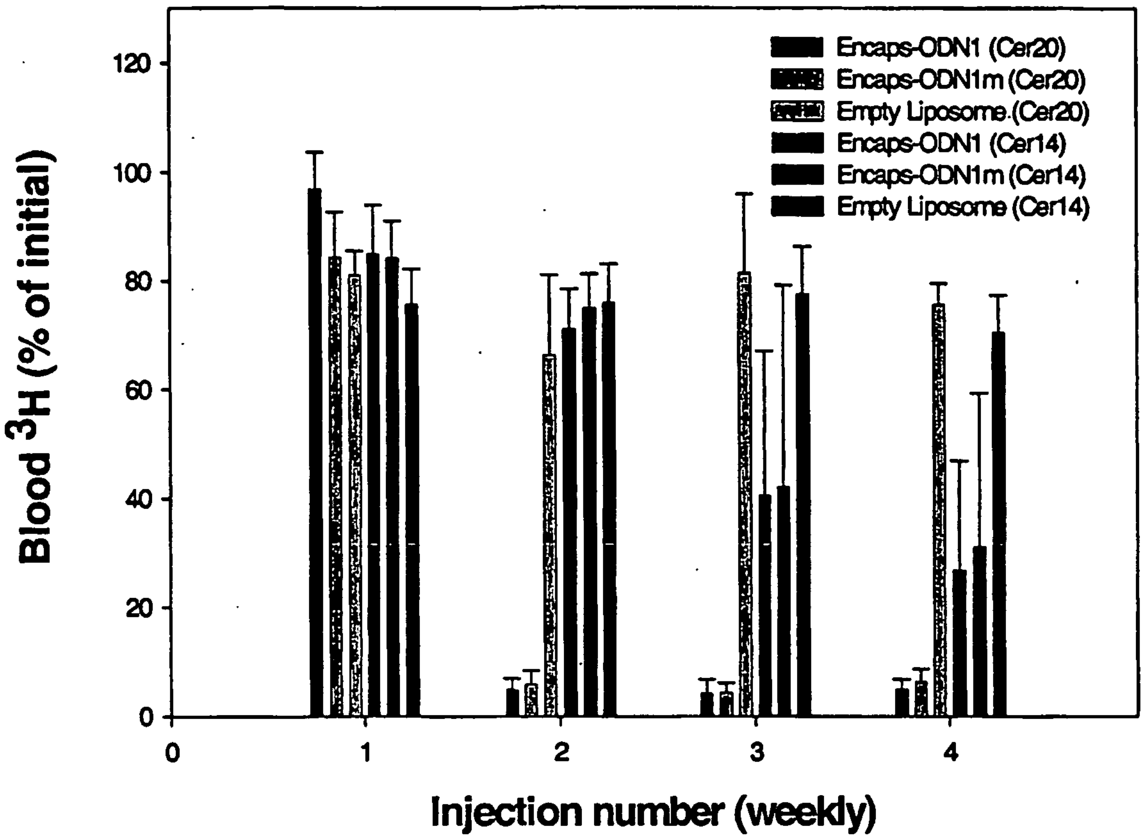
Encaps-ODN1 (Cer20)
Encaps-ODN1m (Cer20)
Empty Liposome (Cer20)
Encaps-ODN1 (Cer14)
Encaps-ODN1m (Cer14)
Empty Liposome (Cer14)
Blood 3H (% of initial)
Injection number (weekly)
0
20
40
60
80
100
120
0
1
2
3
4

Figure 23

(a)

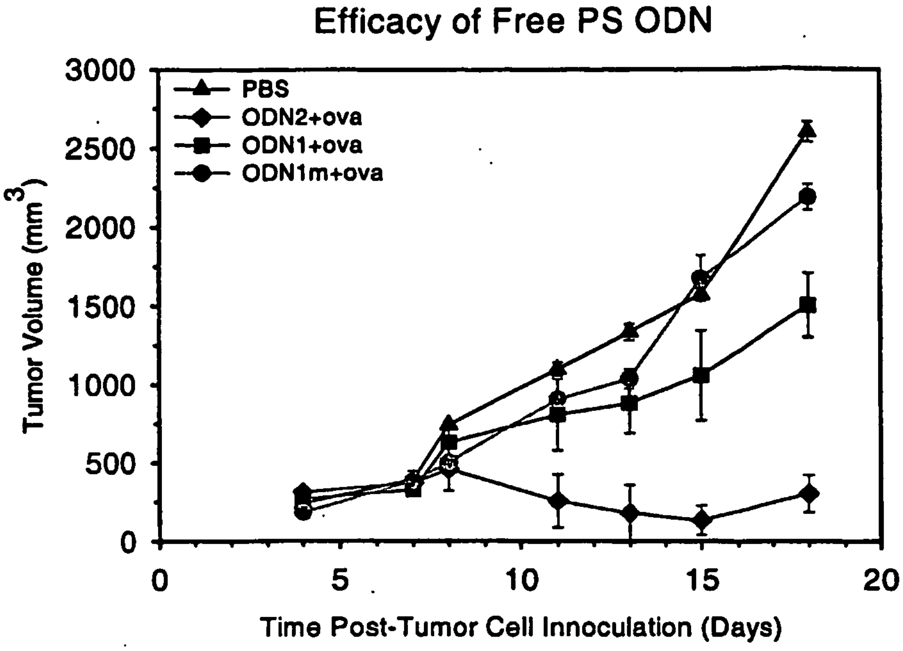
Efficacy of Free PS ODN
PBS
ODN2+ova
ODN1+ova
ODN1m+ova
Tumor Volume (mm3)
3000
2500
2000
1500
1000
500
0
0
5
10
15
20
Time Post-Tumor Cell Innoculation (Days)

(b)

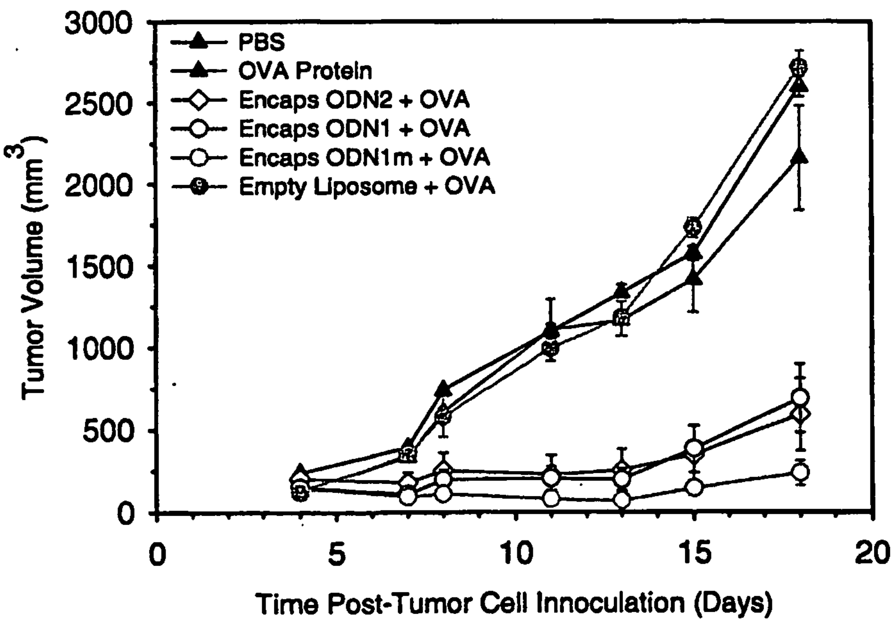
PBS
OVA Protein
Encaps ODN2 + OVA
Encaps ODN1 + OVA
Encaps ODN1m + OVA
Empty Liposome + OVA
Tumor Volume (mm3)
3000
2500
2000
1500
1000
500
0
0
5
10
15
20
Time Post-Tumor Cell Innoculation (Days)

Figure 24

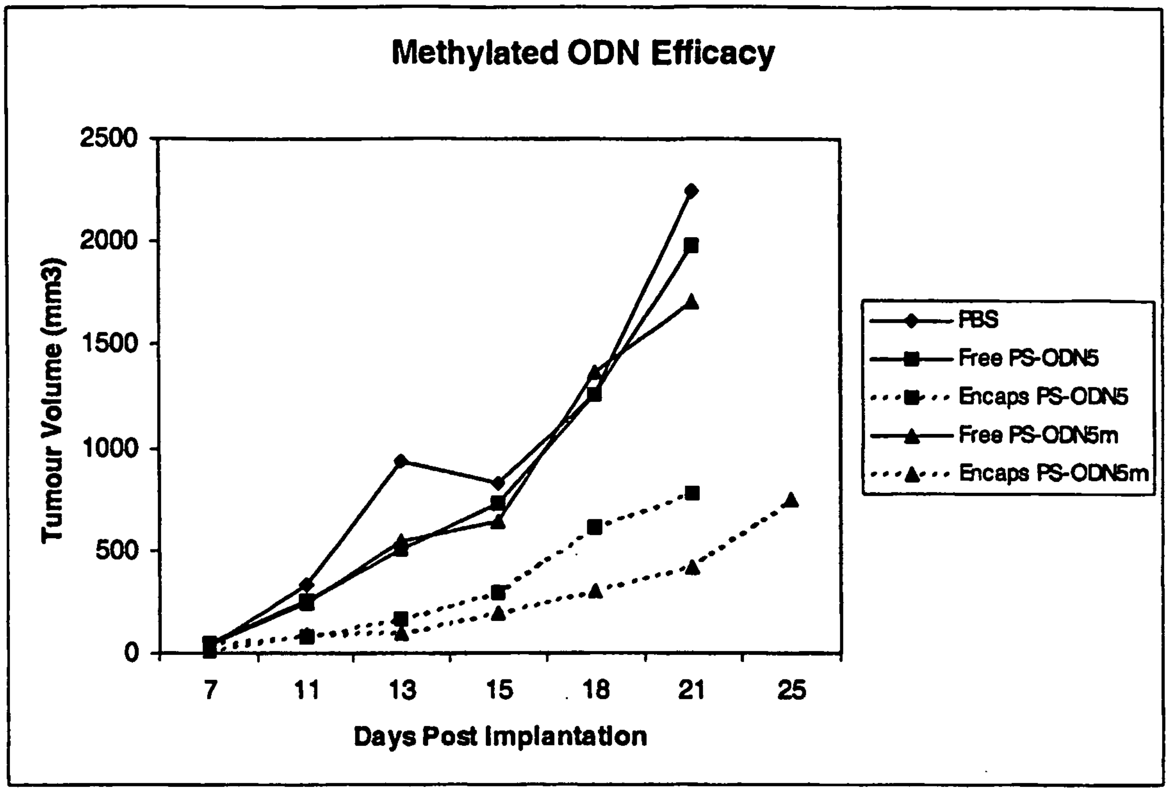
Methylated ODN Efficacy
Tumour Volume (mm3)
2500
2000
1500
1000
500
0
7
11
13
15
18
21
25
Days Post Implantation
PBS
Free PS-ODN5
Encaps PS-ODN5
Free PS-ODN5m
Encaps PS-ODN5m

Figure 25

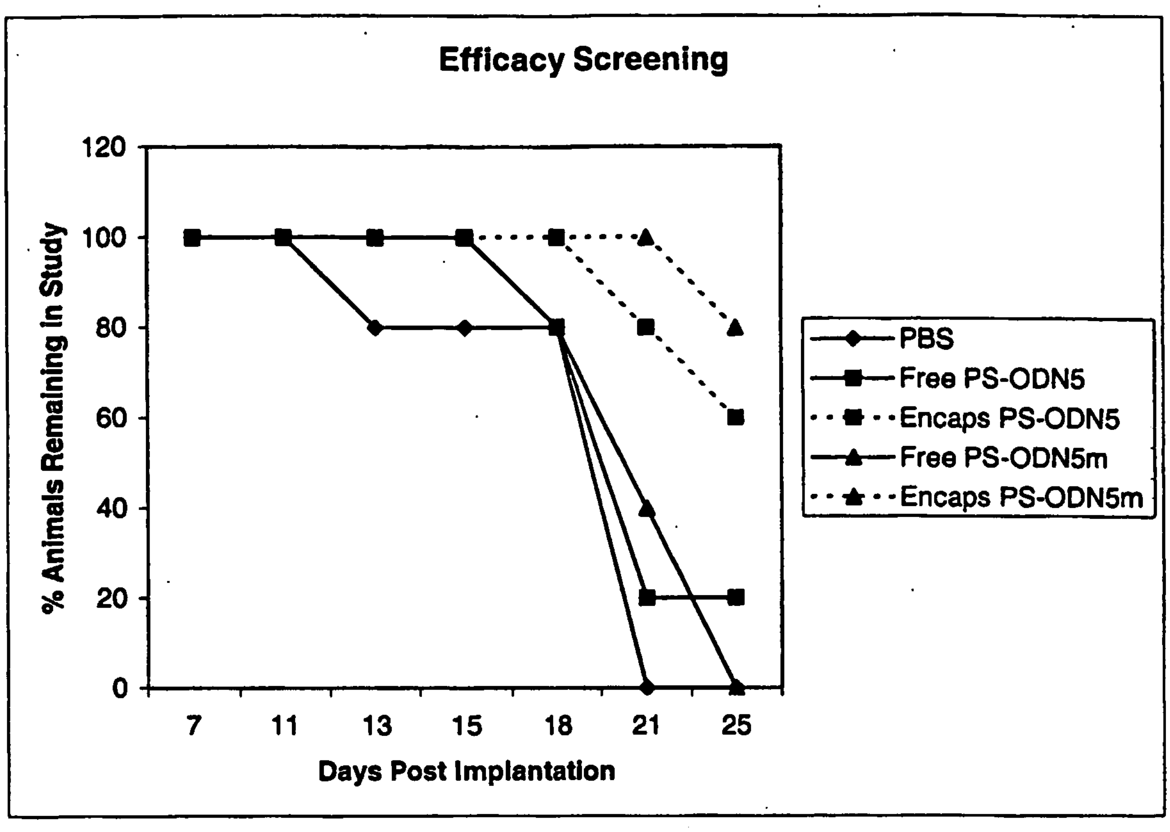

Efficacy Screening
% Animals Remaining in Study
120
100
80
60
40
20
0
7
11
13
15
18
21
25
Days Post Implantation
PBS
Free PS-ODN5
Encaps PS-ODN5
Free PS-ODN5m
Encaps PS-ODN5m


Figure 26

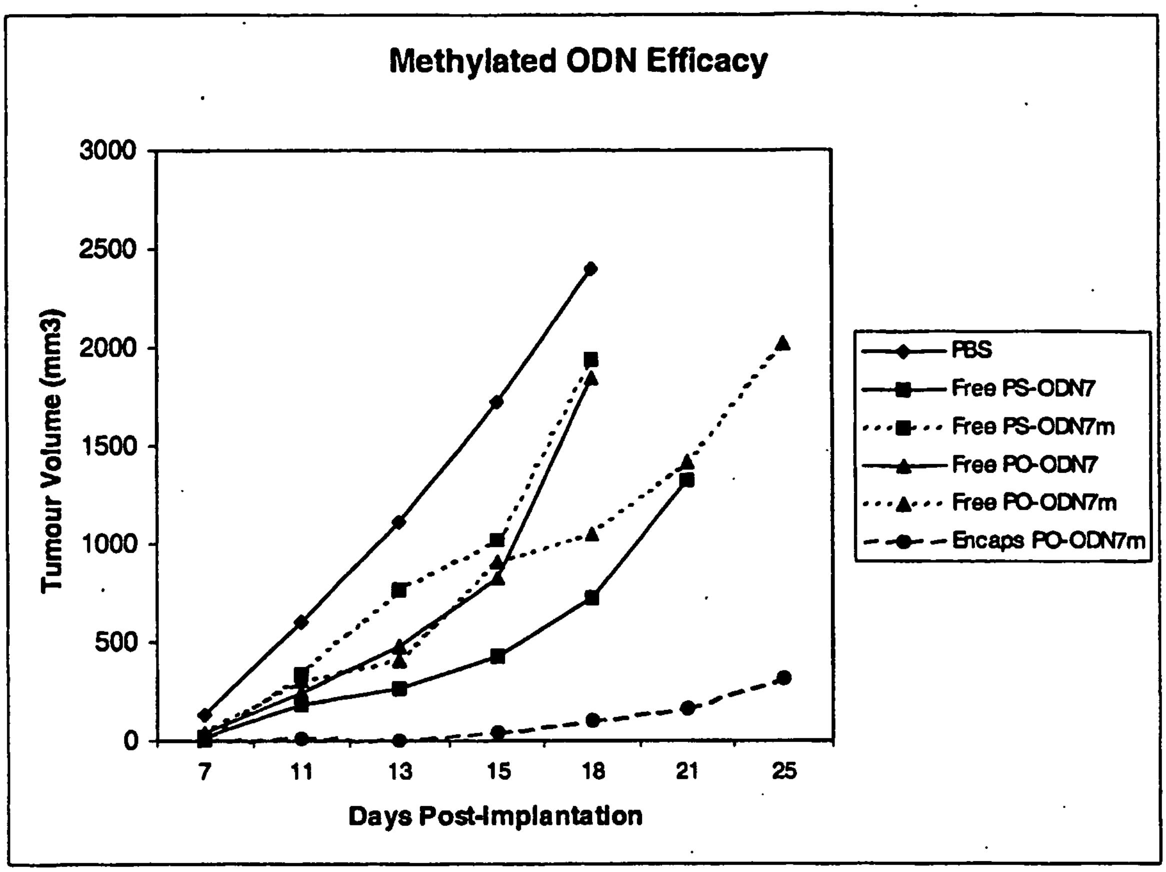
Methylated ODN Efficacy
3000
2500
2000
1500
1000
500
0
Tumour Volume (mm3)
7
11
13
15
18
21
25
Days Post-Implantation
PBS
Free PS-ODN7
Free PS-ODN7m
Free PO-ODN7
Free PO-ODN7m
Encaps PO-ODN7m

Figure 27

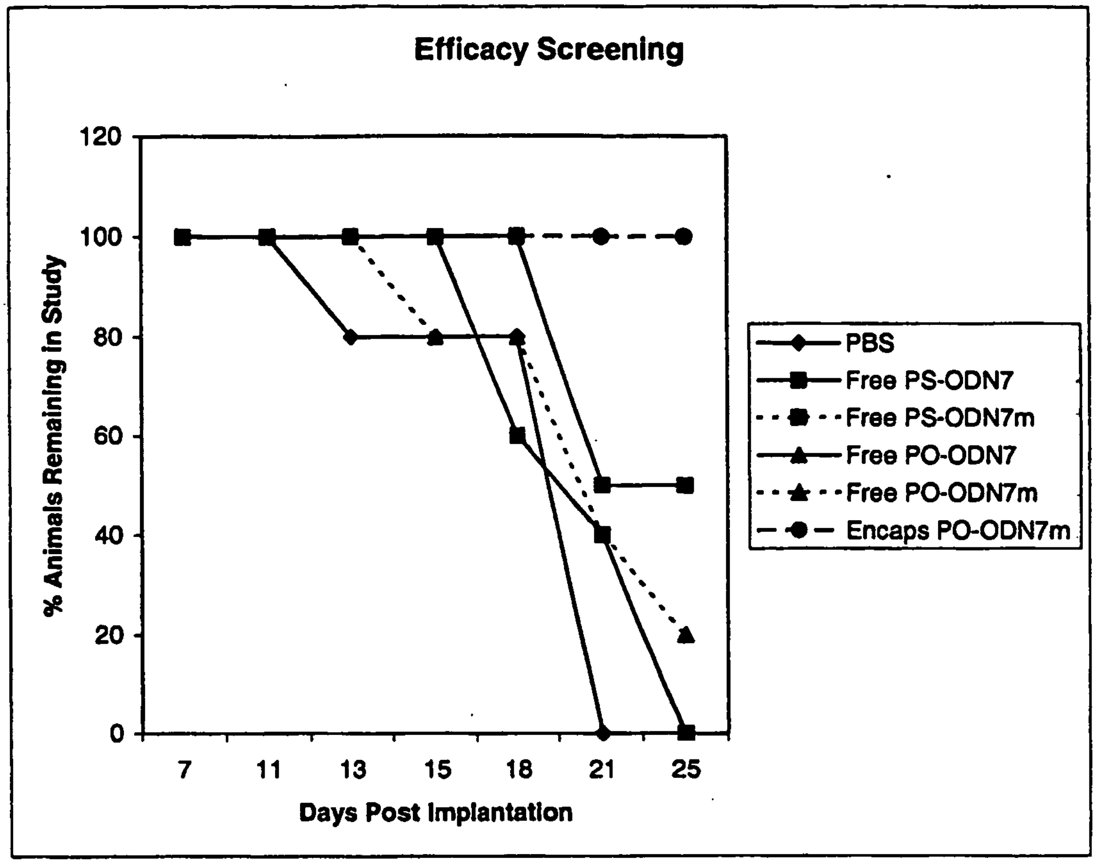
Efficacy Screening
% Animals Remaining in Study
120
100
80
60
40
20
0
7
11
13
15
18
21
25
Days Post Implantation
PBS
Free PS-ODN7
Free PS-ODN7m
Free PO-ODN7
Free PO-ODN7m
Encaps PO-ODN7m

Figure 28

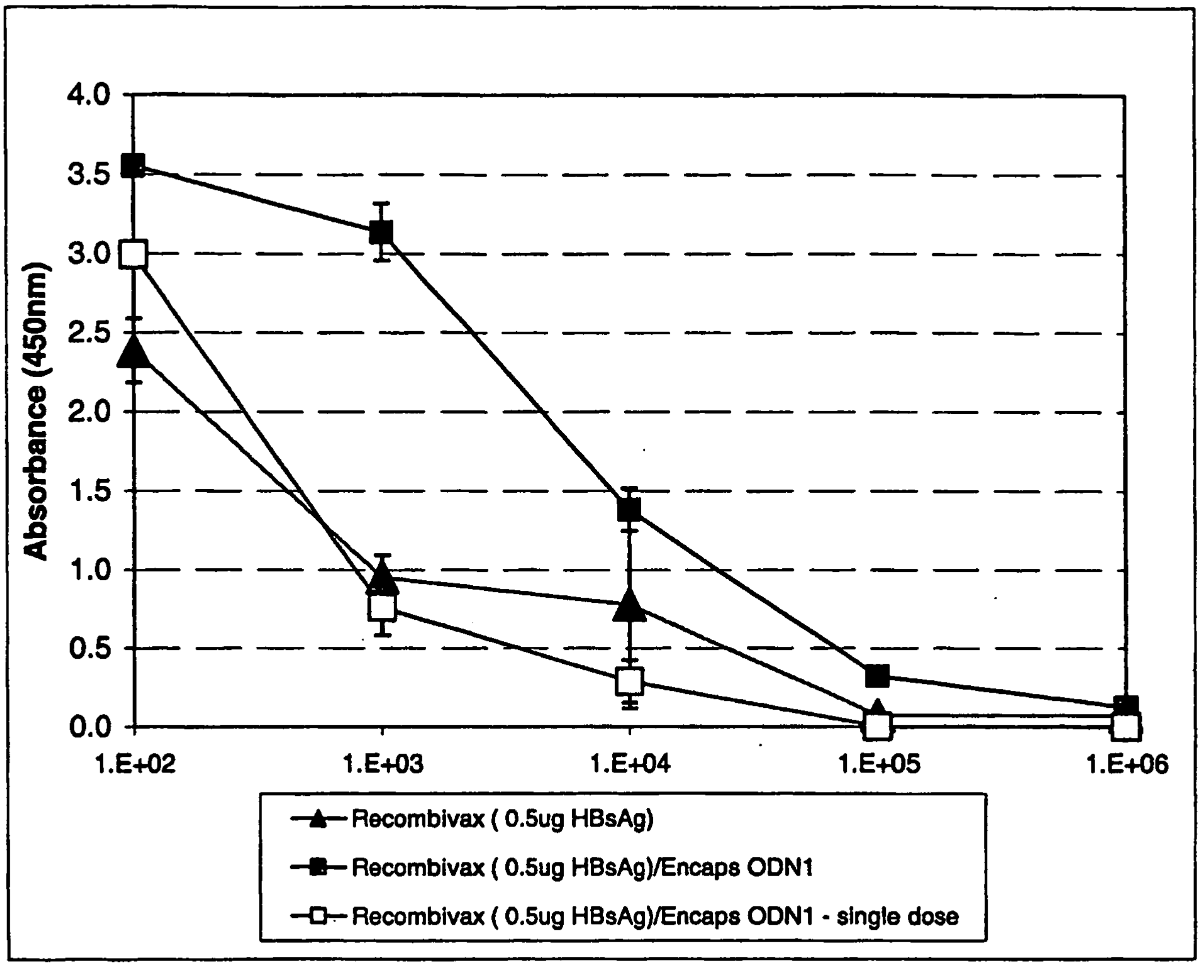
4.0
3.5
3.0
2.5
2.0
1.5
1.0
0.5
0.0
Absorbance (450nm)
1.E+02
1.E+03
1.E+04
1.E+05
1.E+06
Recombivax ( 0.5ug HBsAg)
Recombivax ( 0.5ug HBsAg)/Encaps ODN1
Recombivax ( 0.5ug HBsAg)/Encaps ODN1 - single dose

Figure 29

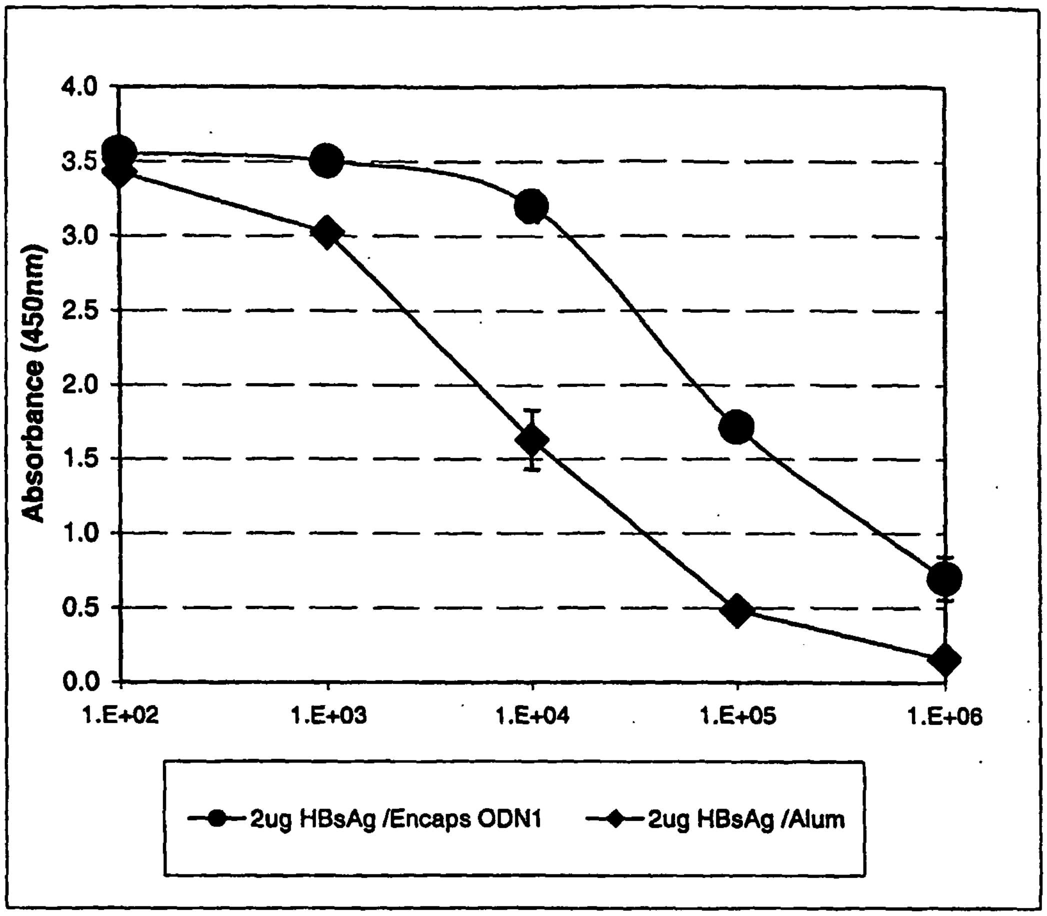
4.0
3.5
3.0
2.5
2.0
1.5
1.0
0.5
0.0
Absorbance (450nm)
1.E+02
1.E+03
1.E+04
1.E+05
1.E+06
2ug HBsAg /Encaps ODN1
2ug HBsAg /Alum

Figure 30

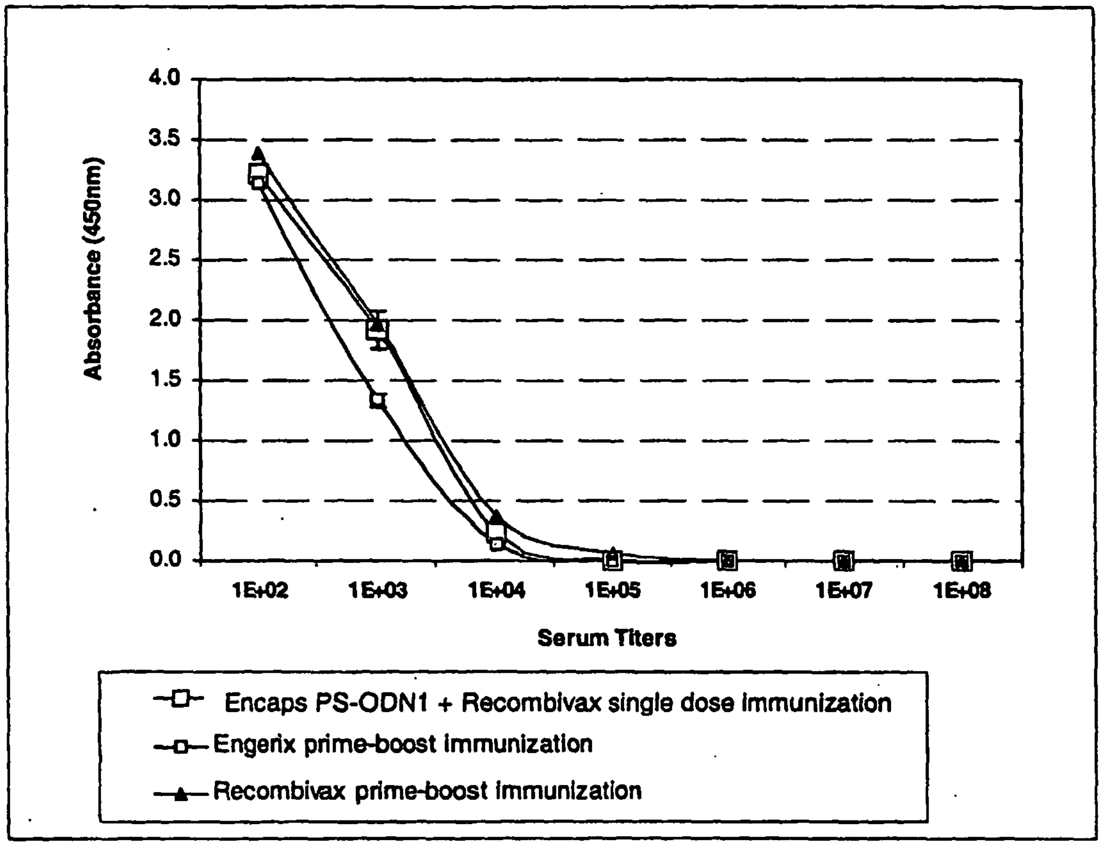
4.0
3.5
3.0
2.5
2.0
1.5
1.0
0.5
0.0
Absorbance (450nm)
1E+02
1E+03
1E+04
1E+05
1E+06
1E+07
1E+08
Serum Titers
Encaps PS-ODN1 + Recombivax single dose immunization
Engerix prime-boost immunization
Recombivax prime-boost immunization

Figure 31

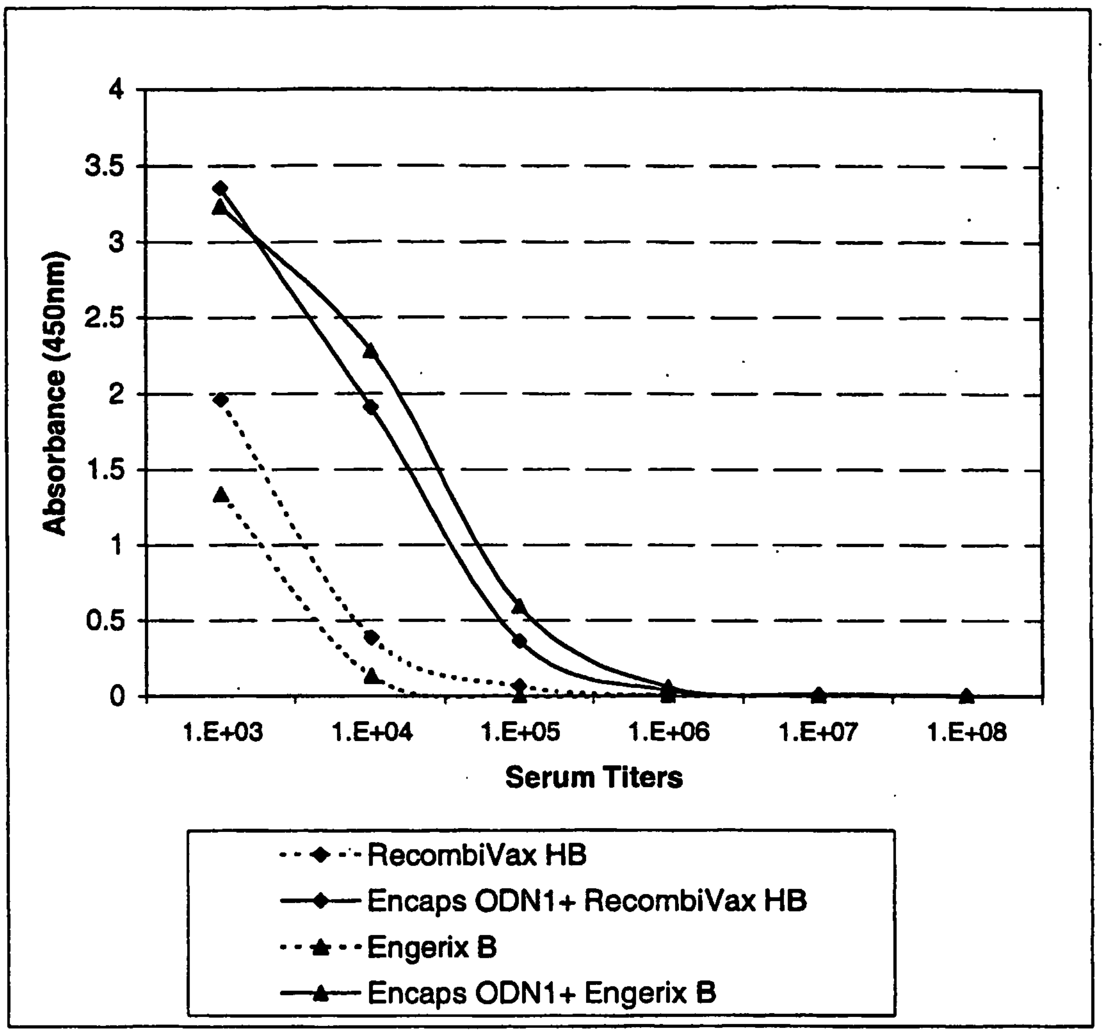
4
3.5
3
2.5
2
1.5
1
0.5
0
Absorbance (450nm)
1.E+03
1.E+04
1.E+05
1.E+06
1.E+07
1.E+08
Serum Titers
RecombiVax HB
Encaps ODN1+ RecombiVax HB
Engerix B
Encaps ODN1+ Engerix B

Figure 32

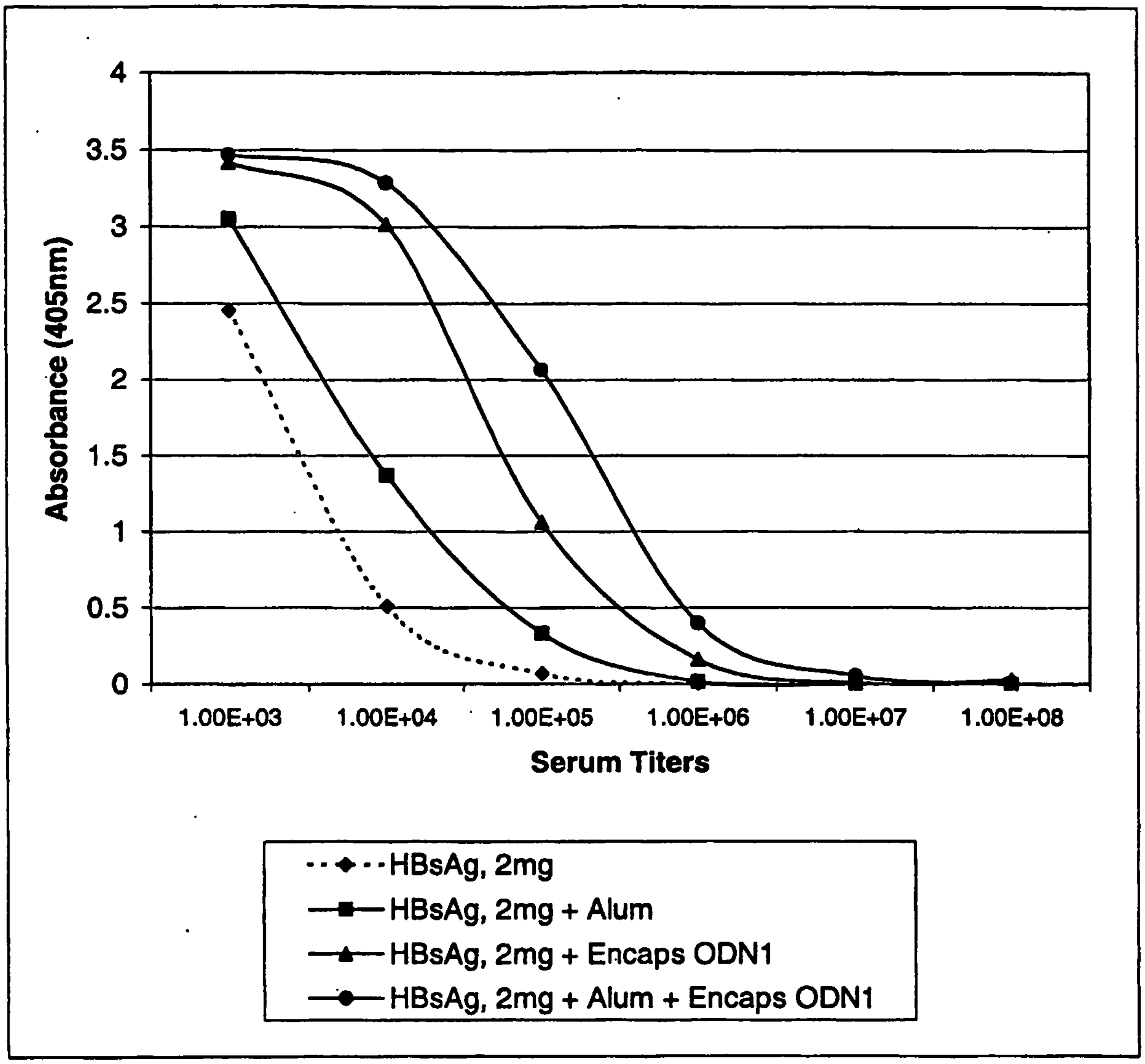
Absorbance (405nm)
4
3.5
3
2.5
2
1.5
1
0.5
0
1.00E+03
1.00E+04
1.00E+05
1.00E+06
1.00E+07
1.00E+08
Serum Titers
HBsAg, 2mg
HBsAg, 2mg + Alum
HBsAg, 2mg + Encaps ODN1
HBsAg, 2mg + Alum + Encaps ODN1

Figure 33

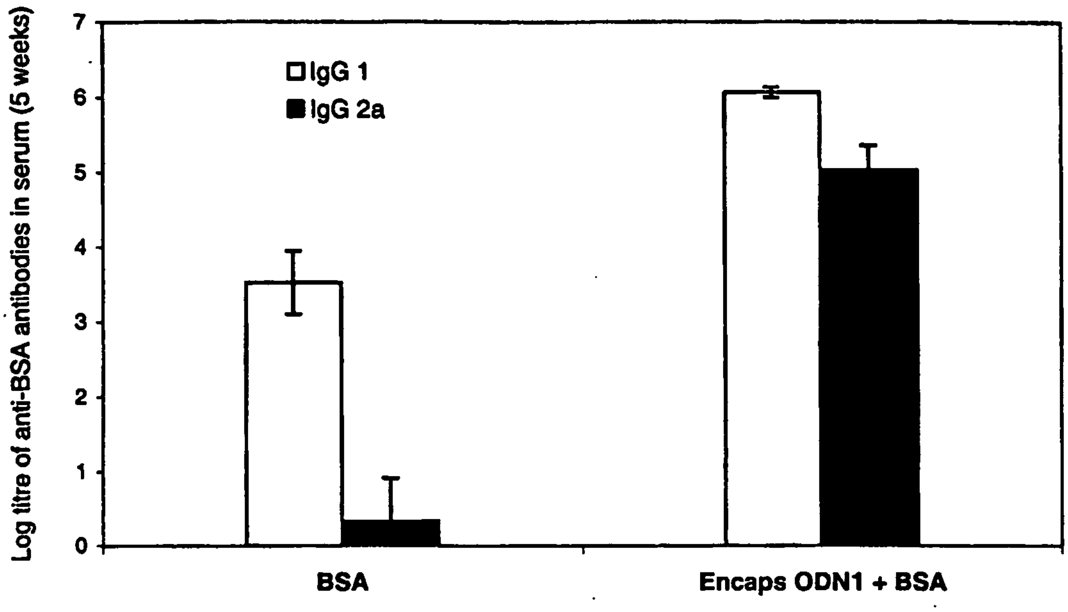
Log titre of anti-BSA antibodies in serum (5 weeks)
7
6
5
4
3
2
1
0
IgG 1
IgG 2a
BSA
Encaps ODN1 + BSA

Figure 34

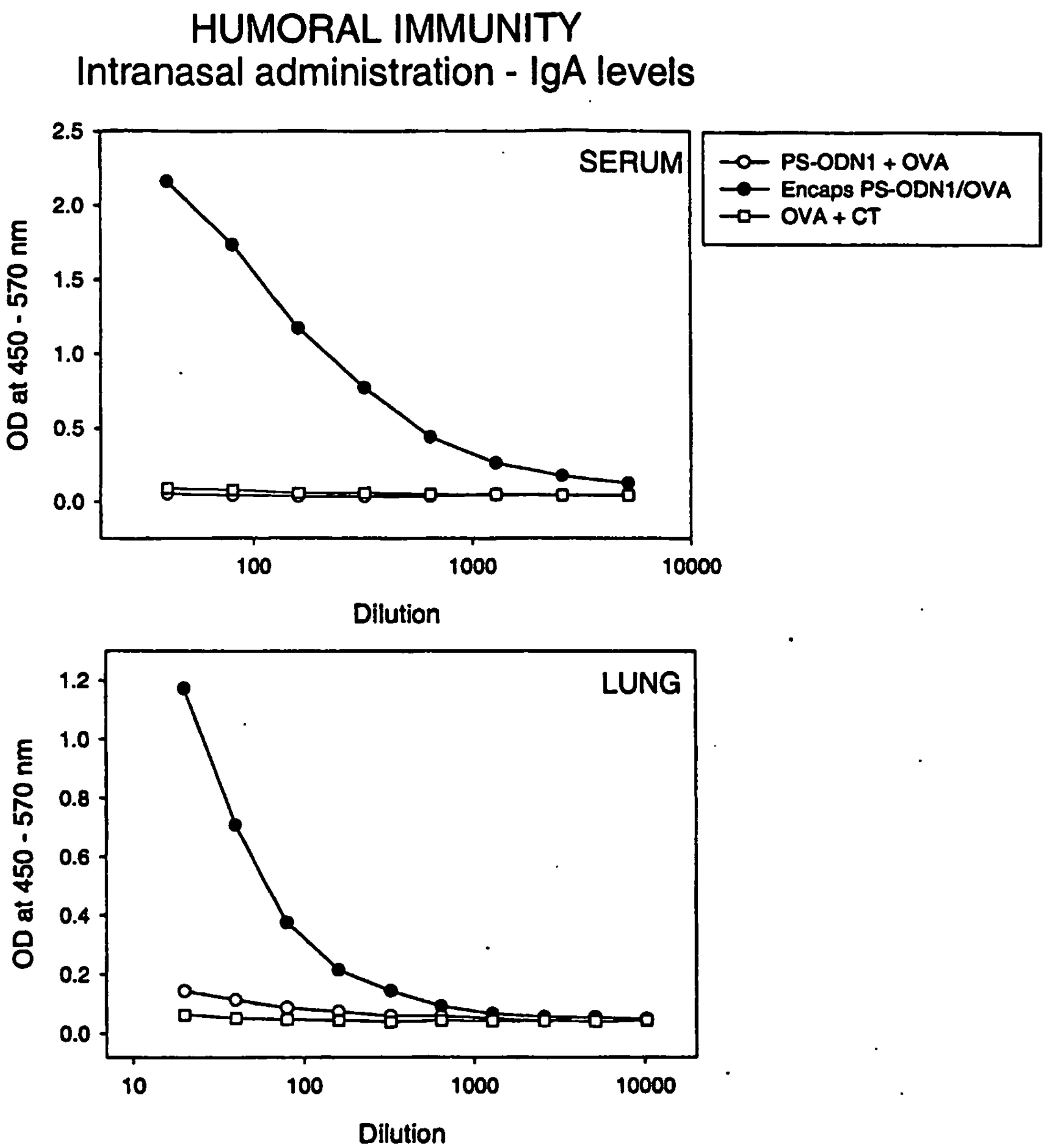
HUMORAL IMMUNITY
Intranasal administration - IgA levels
SERUM
LUNG
PS-ODN1 + OVA
Encaps PS-ODN1/OVA
OVA + CT
OD at 450 - 570 nm
Dilution
2.5
2.0
1.5
1.0
0.5
0.0
1.2
0.8
0.6
0.4
0.2
10
100
1000
10000

Figure 35

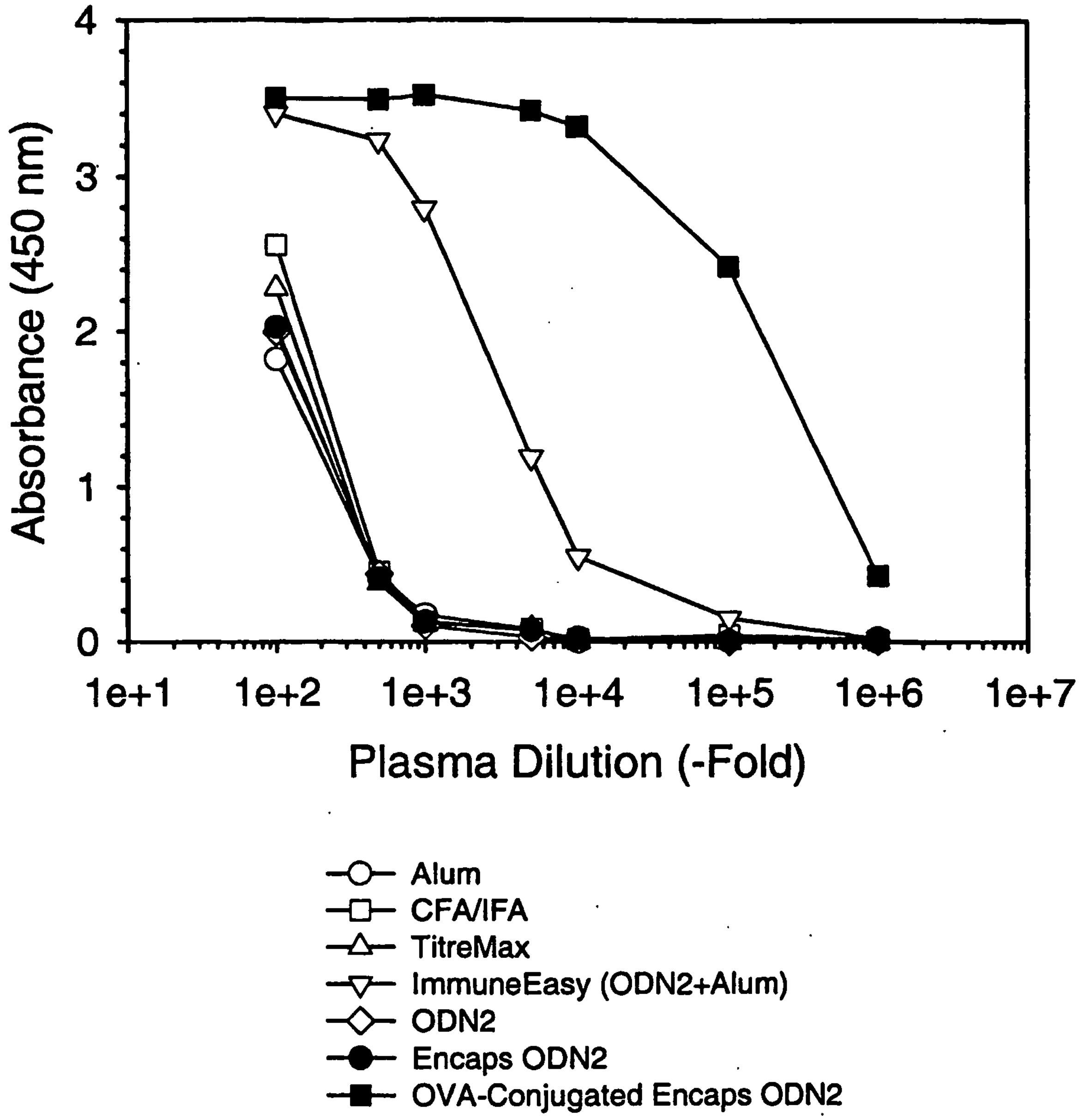
Absorbance (450 nm)
4
3
2
1
0
1e+1
1e+2
1e+3
1e+4
1e+5
1e+6
1e+7
Plasma Dilution (-Fold)
Alum
CFA/IFA
TitreMax
ImmuneEasy (ODN2+Alum)
ODN2
Encaps ODN2
OVA-Conjugated Encaps ODN2

Figure 36

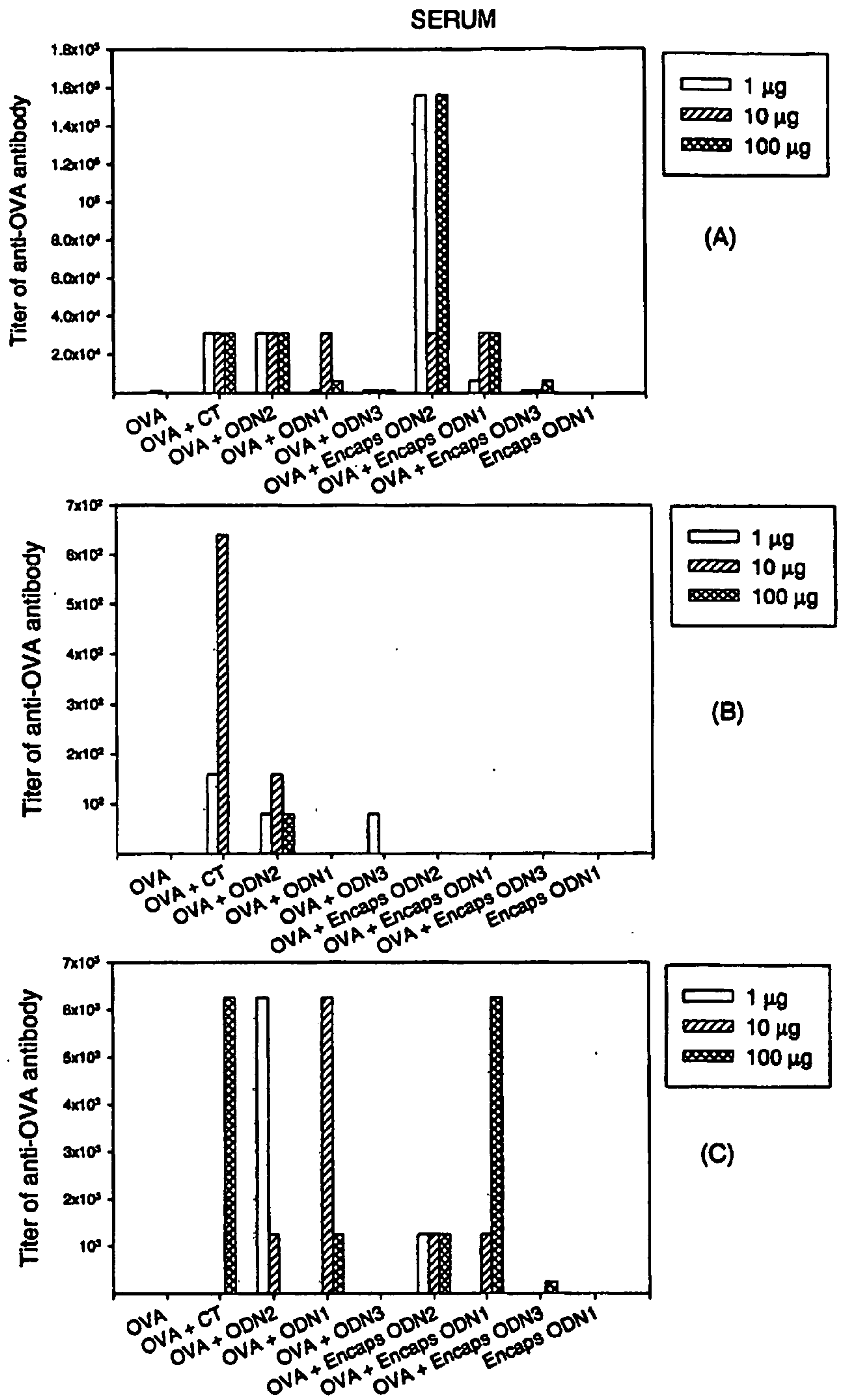
SERUM
Titer of anti-OVA antibody
1 µg
10 µg
100 µg
(A)
OVA
OVA + CT
OVA + ODN2
OVA + ODN1
OVA + ODN3
OVA + Encaps ODN2
OVA + Encaps ODN1
OVA + Encaps ODN3
Encaps ODN1
Titer of anti-OVA antibody
1 µg
10 µg
100 µg
(B)
OVA
OVA + CT
OVA + ODN2
OVA + ODN1
OVA + ODN3
OVA + Encaps ODN2
OVA + Encaps ODN1
OVA + Encaps ODN3
Encaps ODN1
Titer of anti-OVA antibody
1 µg
10 µg
100 µg
(C)
OVA
OVA + CT
OVA + ODN2
OVA + ODN1
OVA + ODN3
OVA + Encaps ODN2
OVA + Encaps ODN1
OVA + Encaps ODN3
Encaps ODN1

Figure 37

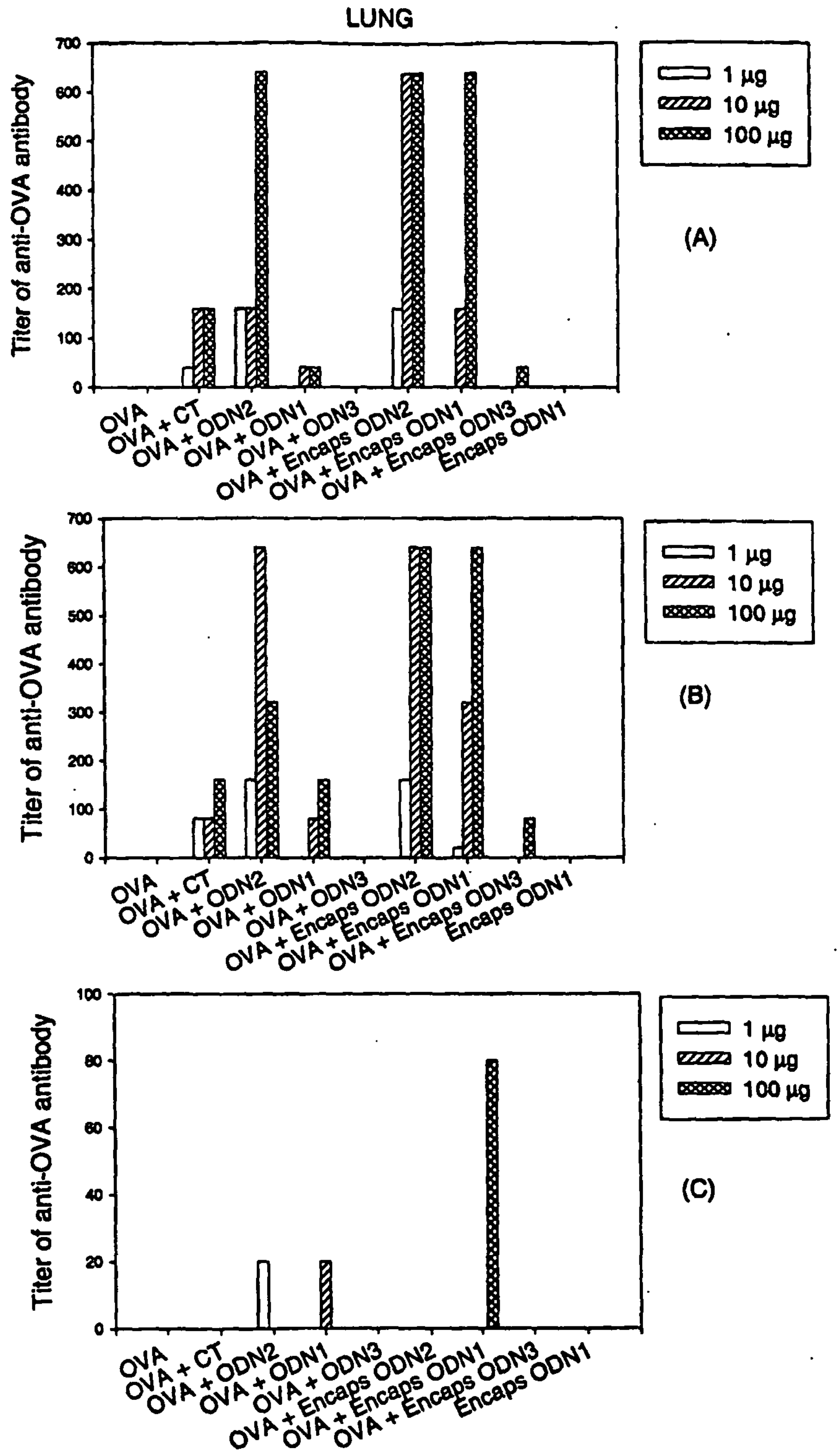
LUNG
Titer of anti-OVA antibody
700
600
500
400
300
200
100
0
1 µg
10 µg
100 µg
(A)
OVA
OVA + CT
OVA + ODN2
OVA + ODN1
OVA + ODN3
OVA + Encaps ODN2
OVA + Encaps ODN1
OVA + Encaps ODN3
Encaps ODN1
(B)
(C)
80
60
40
20

Figure 38

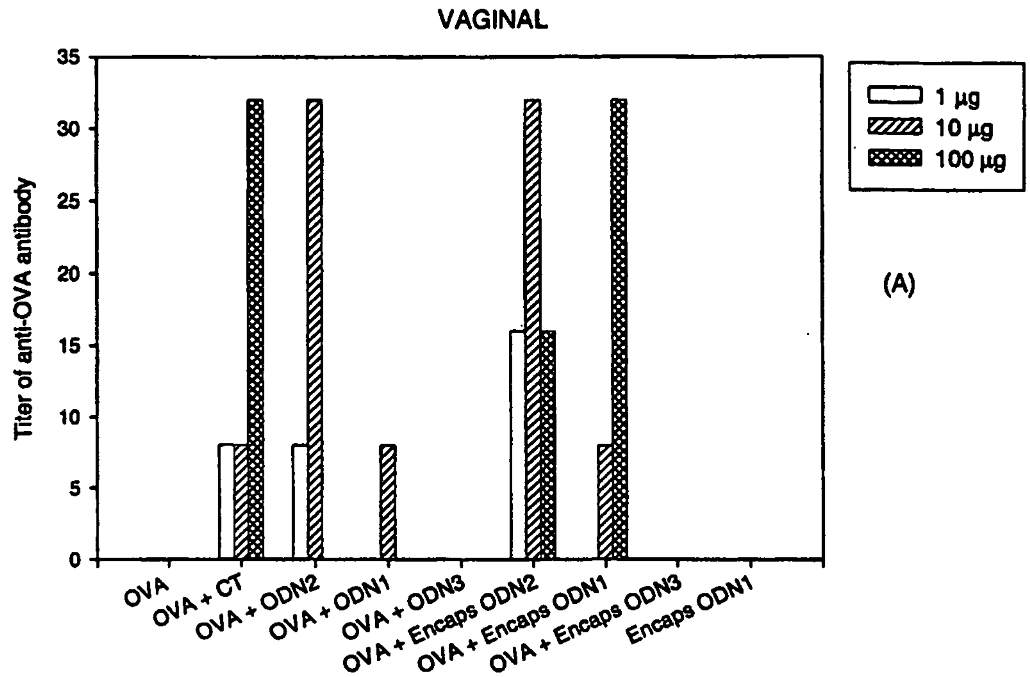
VAGINAL
Titer of anti-OVA antibody
35
30
25
20
15
10
5
0
1 µg
10 µg
100 µg
OVA
OVA + CT
OVA + ODN2
OVA + ODN1
OVA + ODN3
OVA + Encaps ODN2
OVA + Encaps ODN1
OVA + Encaps ODN3
Encaps ODN1

(A)

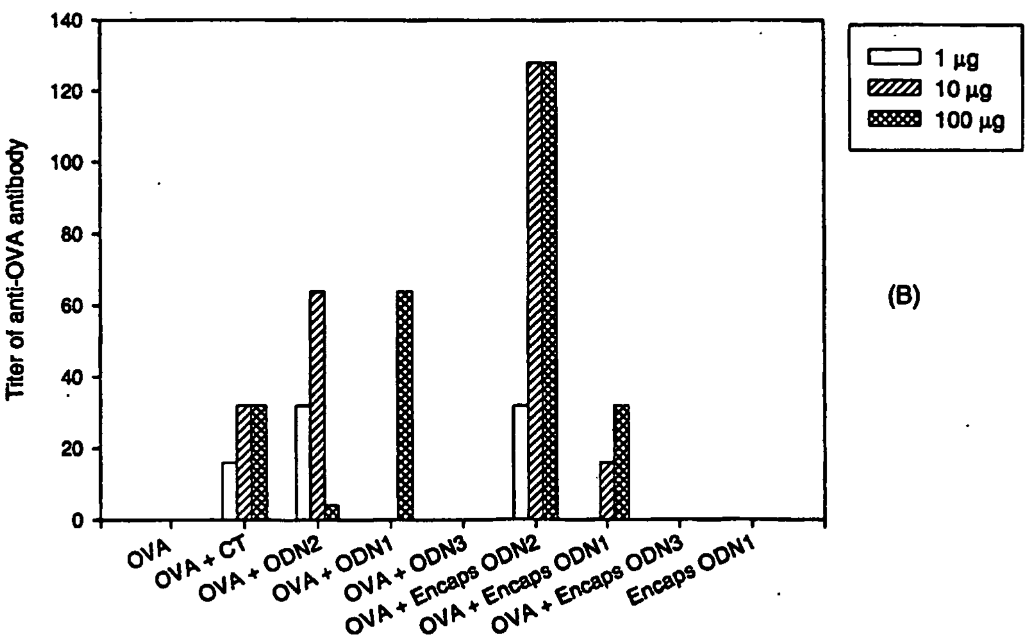
Titer of anti-OVA antibody
140
120
100
80
60
40
20
0
1 µg
10 µg
100 µg
OVA
OVA + CT
OVA + ODN2
OVA + ODN1
OVA + ODN3
OVA + Encaps ODN2
OVA + Encaps ODN1
OVA + Encaps ODN3
Encaps ODN1

(B)

Figure 39

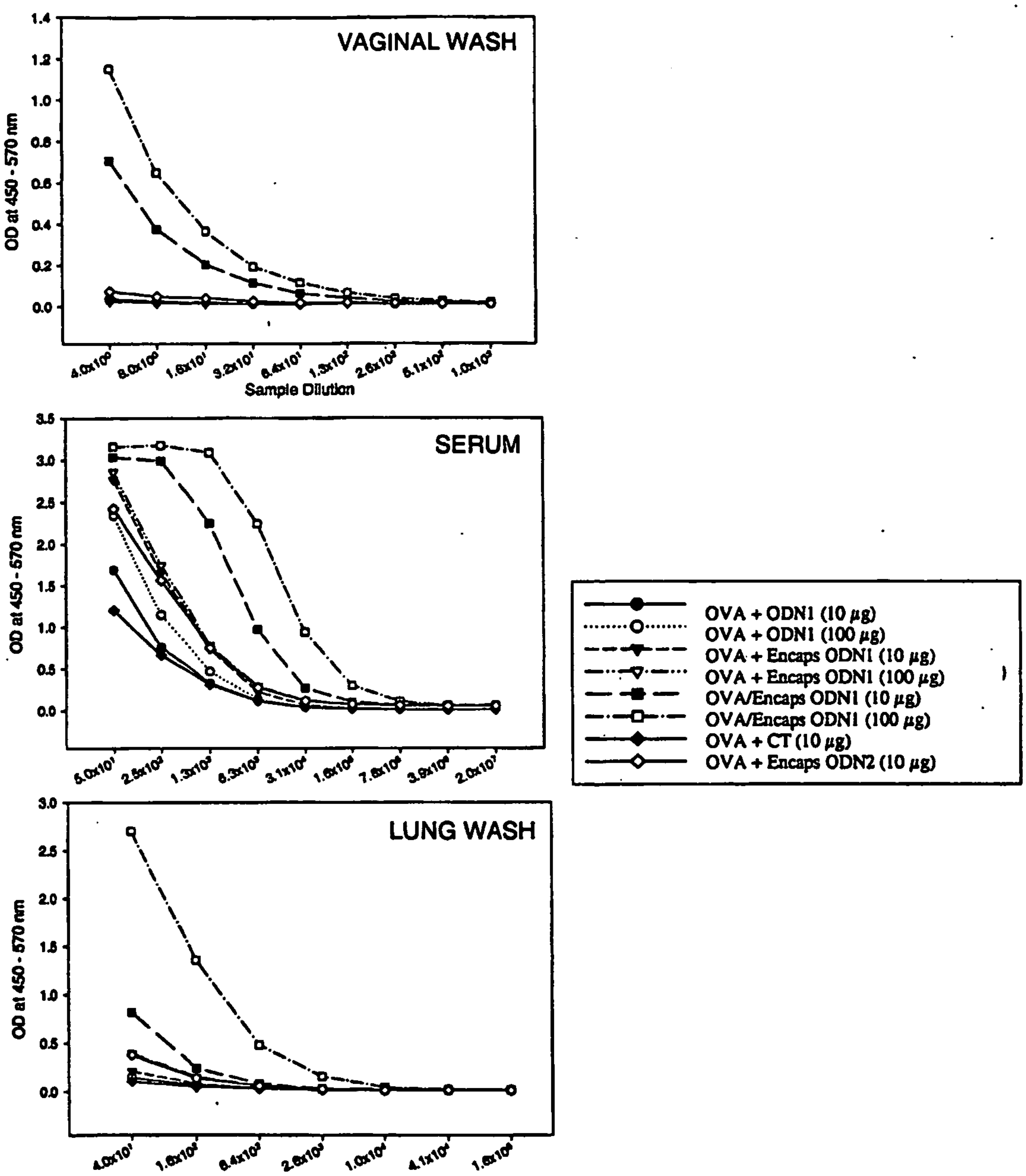
VAGINAL WASH
OD at 450 - 570 nm
Sample Dilution
SERUM
LUNG WASH
OVA + ODN1 (10 µg)
OVA + ODN1 (100 µg)
OVA + Encaps ODN1 (10 µg)
OVA + Encaps ODN1 (100 µg)
OVA/Encaps ODN1 (10 µg)
OVA/Encaps ODN1 (100 µg)
OVA + CT (10 µg)
OVA + Encaps ODN2 (10 µg)

Figure 40

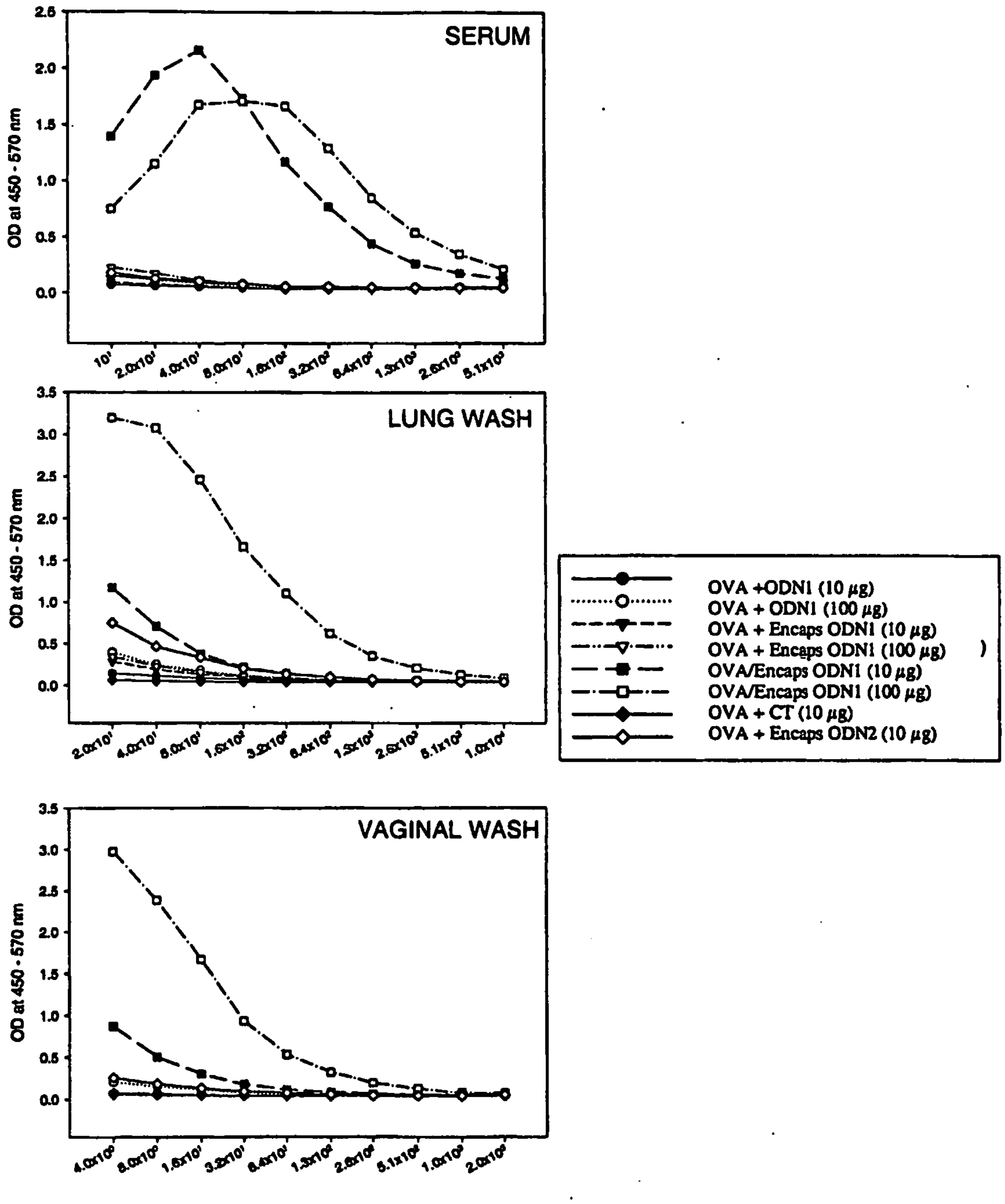
SERUM
LUNG WASH
VAGINAL WASH
OD at 450 - 570 nm
OVA +ODN1 (10 μg)
OVA + ODN1 (100 μg)
OVA + Encaps ODN1 (10 μg)
OVA + Encaps ODN1 (100 μg)
OVA/Encaps ODN1 (10 μg)
OVA/Encaps ODN1 (100 μg)
OVA + CT (10 μg)
OVA + Encaps ODN2 (10 μg)

Figure 41

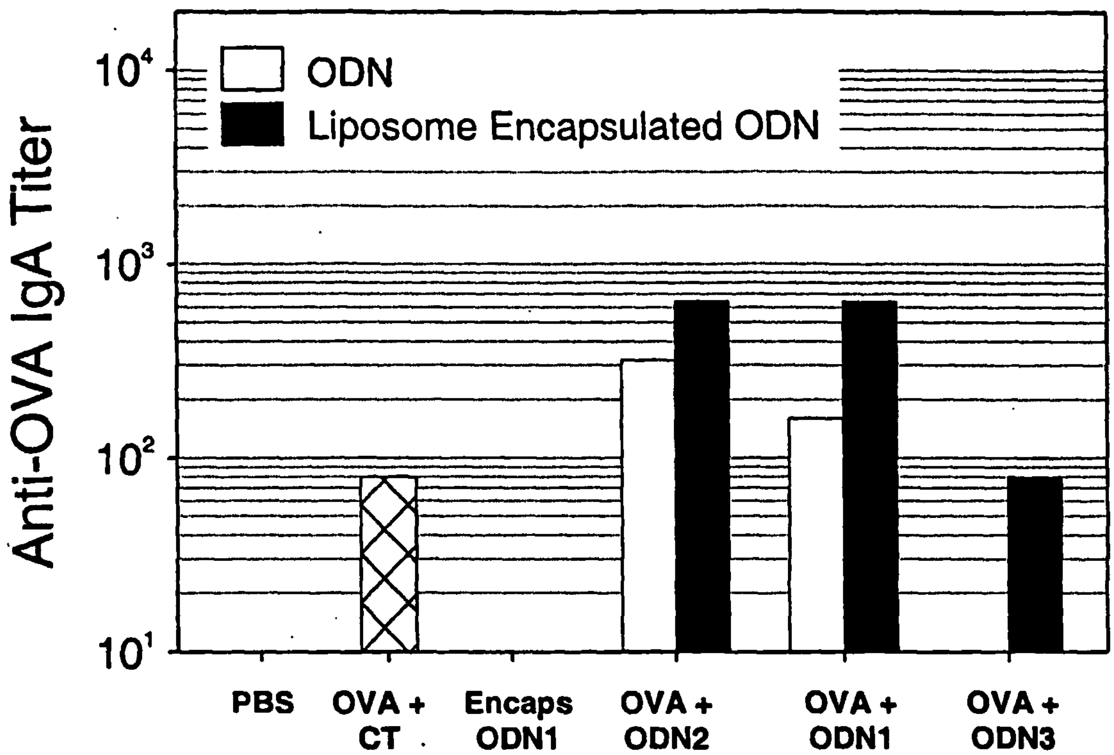
Anti-OVA IgA Titer
$10^4$
$10^3$
$10^2$
$10^1$
ODN
Liposome Encapsulated ODN
PBS
OVA + CT
Encaps ODN1
OVA + ODN2
OVA + ODN1
OVA + ODN3

Figure 42

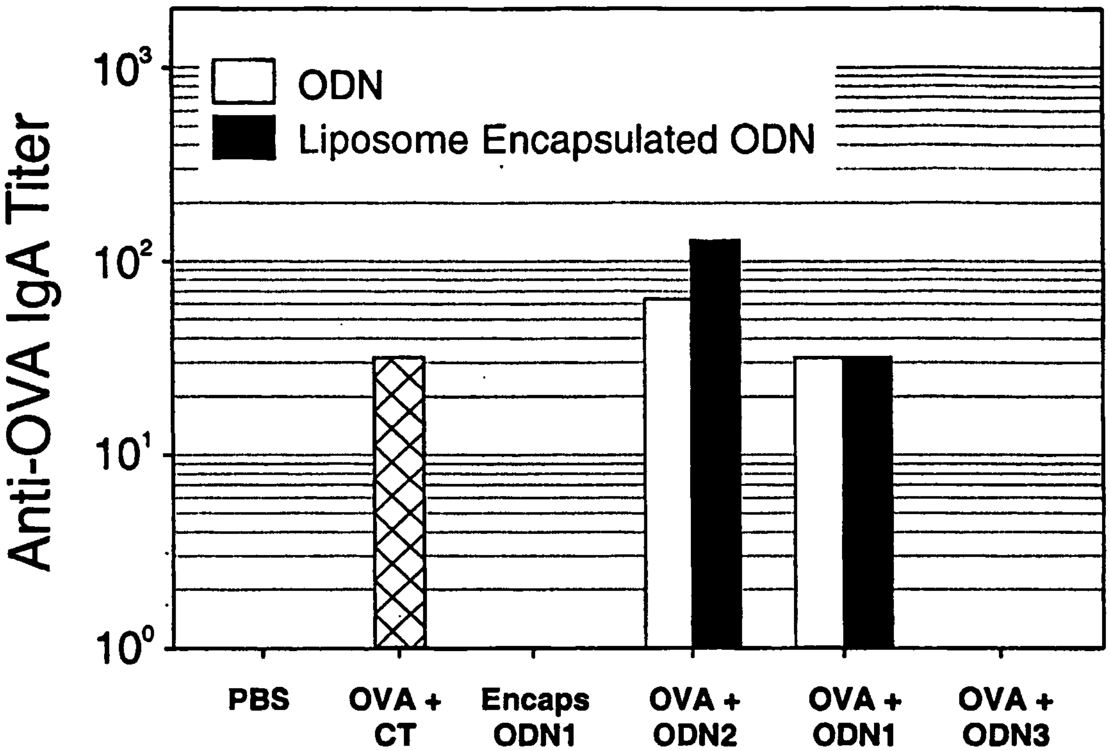
Anti-OVA IgA Titer
$10^3$
$10^2$
$10^1$
$10^0$
ODN
Liposome Encapsulated ODN
PBS
OVA + CT
Encaps ODN1
OVA + ODN2
OVA + ODN1
OVA + ODN3

Figure 43

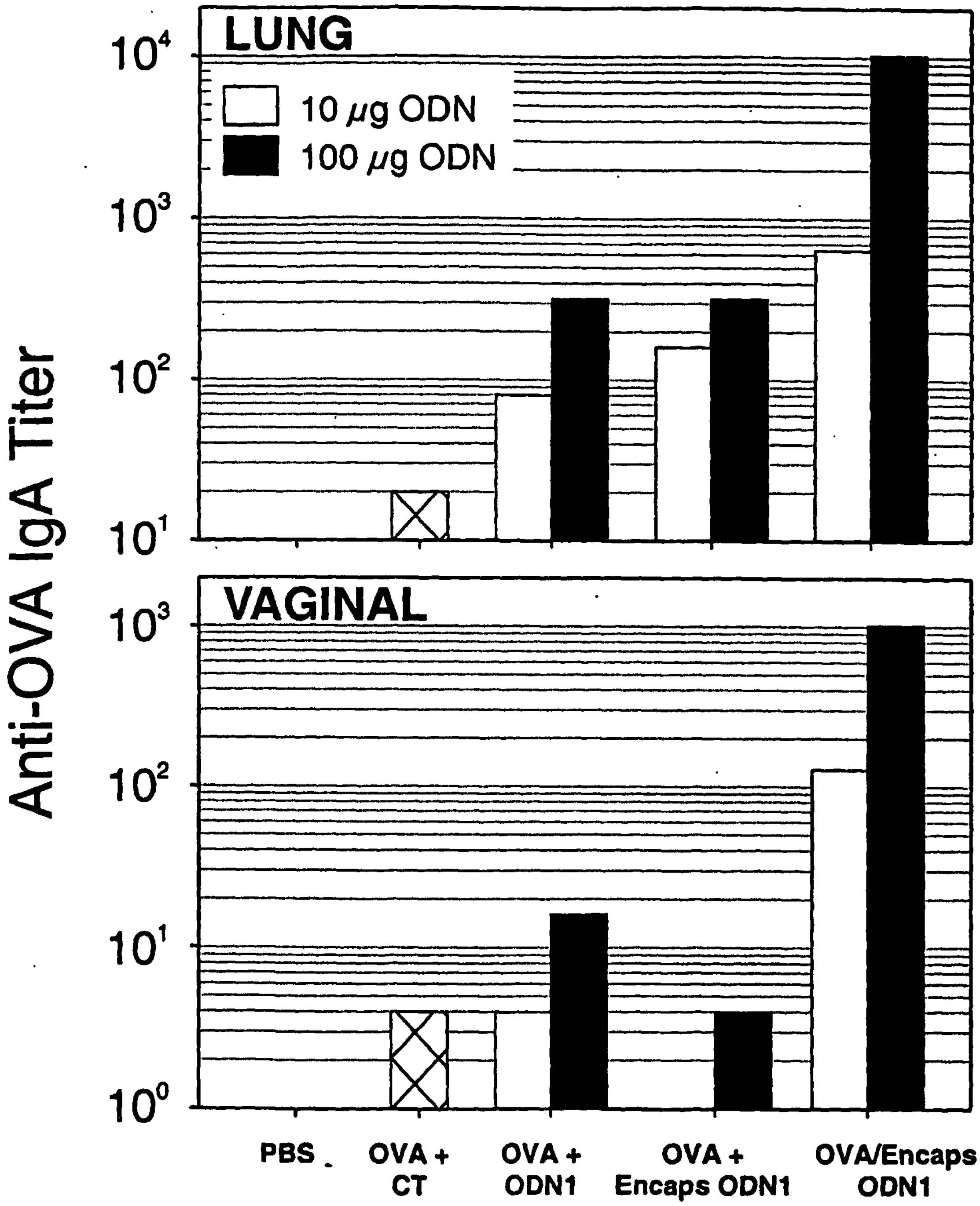
Anti-OVA IgA Titer
LUNG
10 μg ODN
100 μg ODN
10^4
10^3
10^2
10^1
VAGINAL
10^3
10^2
10^1
10^0
PBS
OVA + CT
OVA + ODN1
OVA + Encaps ODN1
OVA/Encaps ODN1

Figure 44

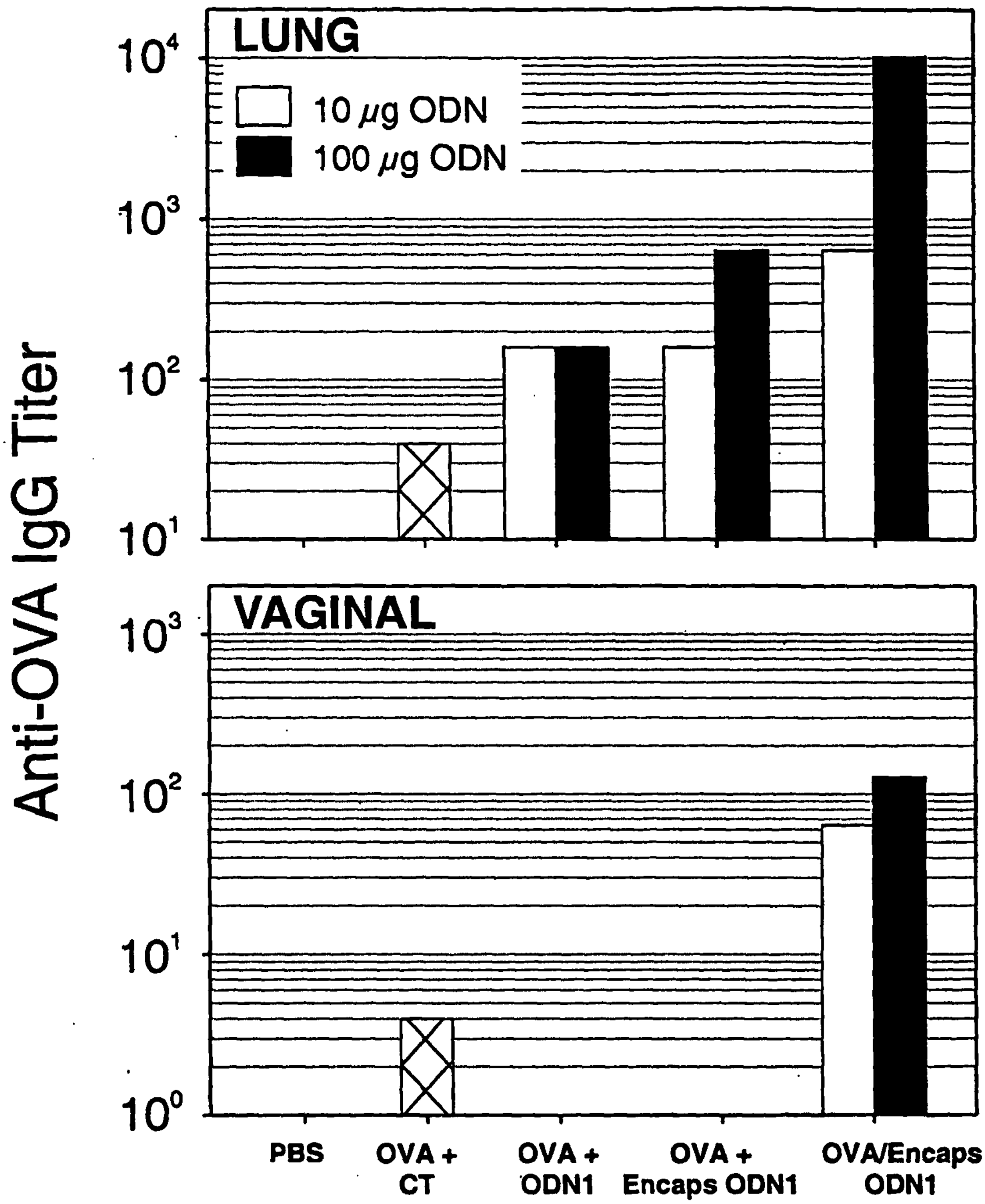
LUNG
10 μg ODN
100 μg ODN
VAGINAL
Anti-OVA IgG Titer
10^4
10^3
10^2
10^1
10^0
PBS
OVA + CT
OVA + ODN1
OVA + Encaps ODN1
OVA/Encaps ODN1

Figure 45

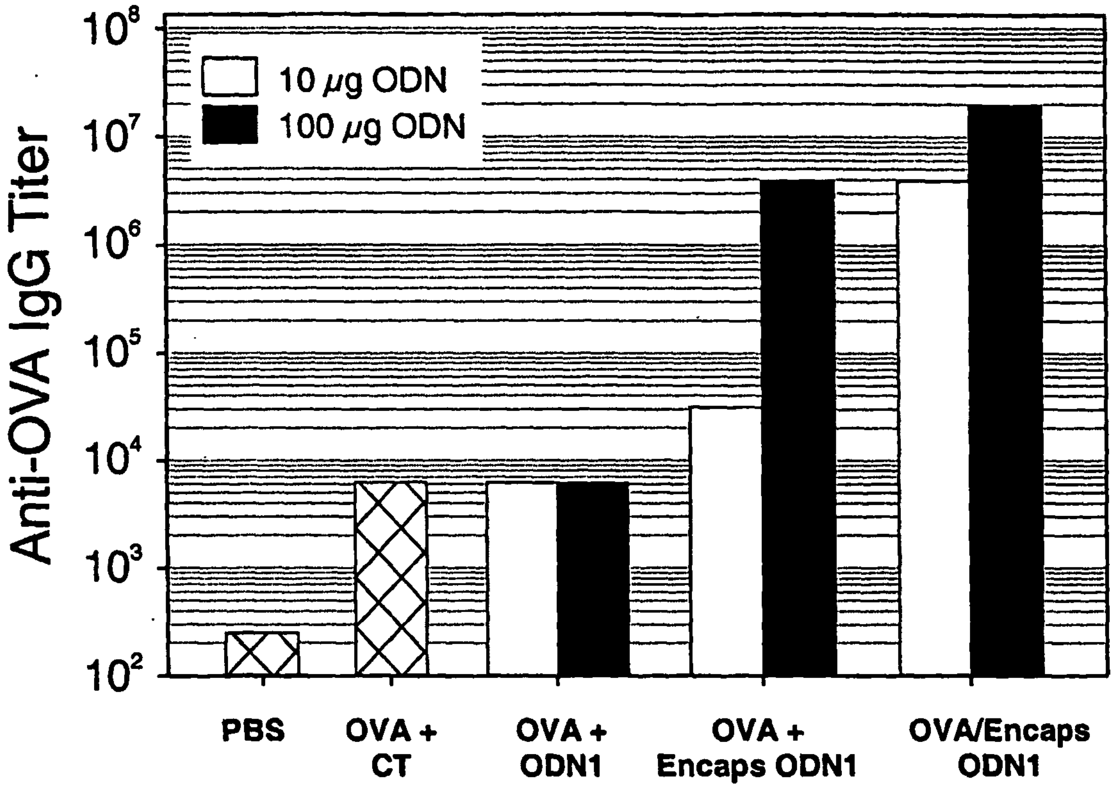
Anti-OVA IgG Titer
$10^8$
$10^7$
$10^6$
$10^5$
$10^4$
$10^3$
$10^2$
10 μg ODN
100 μg ODN
PBS
OVA + CT
OVA + ODN1
OVA + Encaps ODN1
OVA/Encaps ODN1

Figure 46

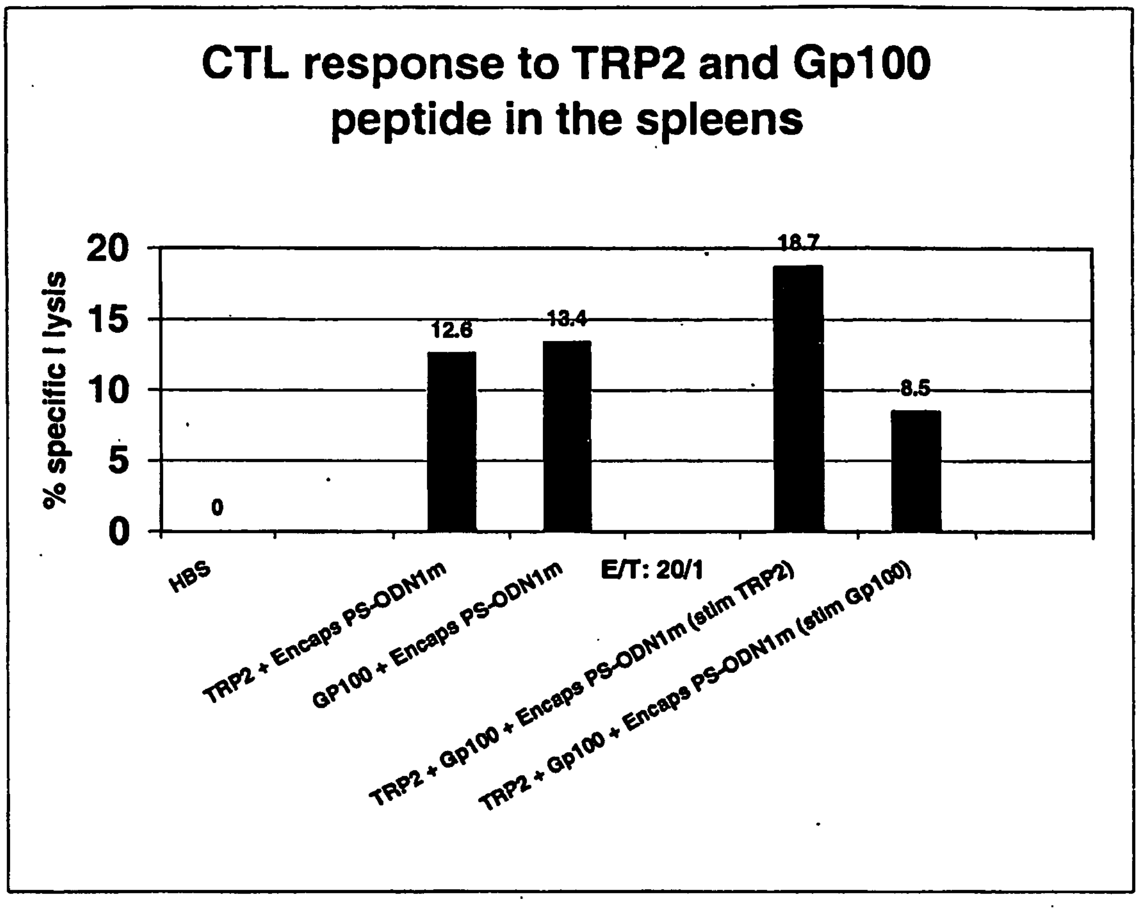
CTL response to TRP2 and Gp100 peptide in the spleens
% specific l lysis
20
15
10
5
0
0
12.6
13.4
18.7
8.5
HBS
TRP2 + Encaps PS-ODN1m
GP100 + Encaps PS-ODN1m
E/T: 20/1
TRP2 + Gp100 + Encaps PS-ODN1m (stim TRP2)
TRP2 + Gp100 + Encaps PS-ODN1m (stim Gp100)

Figure 47

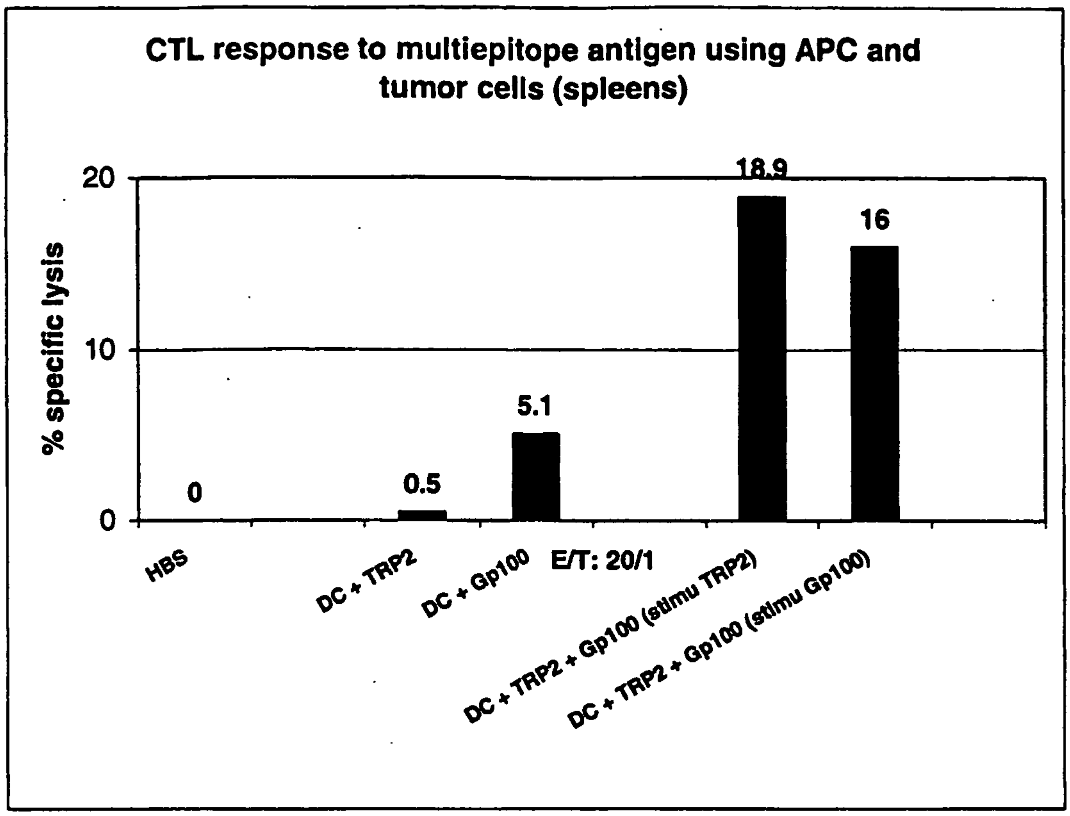
CTL response to multiepitope antigen using APC and tumor cells (spleens)
% specific lysis
20
10
0
18.9
16
5.1
0.5
0
HBS
DC + TRP2
DC + Gp100
E/T: 20/1
DC + TRP2 + Gp100 (stimu TRP2)
DC + TRP2 + Gp100 (stimu Gp100)

Figure 48

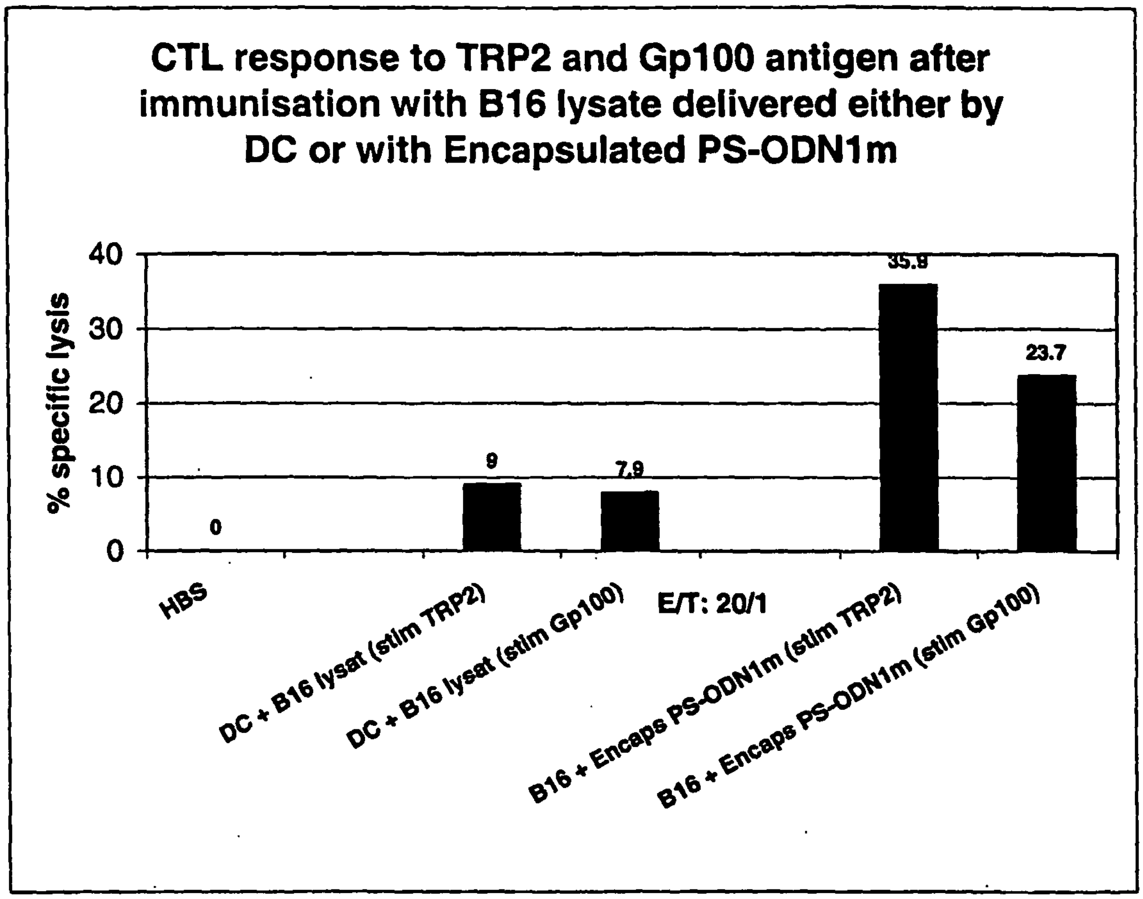
CTL response to TRP2 and Gp100 antigen after immunisation with B16 lysate delivered either by DC or with Encapsulated PS-ODN1m
% specific lysis
40
30
20
10
0
0
9
7.9
35.8
23.7
HBS
DC + B16 lysat (stim TRP2)
DC + B16 lysat (stim Gp100)
E/T: 20/1
B16 + Encaps PS-ODN1m (stim TRP2)
B16 + Encaps PS-ODN1m (stim Gp100)

Figure 49

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4469863 A
- US 5023243 A
- EP 092574 A
- WO 9611266 A
- US 379343 P
- US 649527 A
- WO 02069369 A
- WO 0115726 A
- US 6406705 B
- US 6465439 B
- US 6379698 B
- US 6365611 B
- US 6093816 A
- US 5785992 A
- US 6287591 B
- US 6287591 B1
- US 4320121 A
- US 5820873 A
- US 5885613 A
- WO 9851278 A
- US 218988 A
- US 094540 A
- US 6320017 B
- US 6417326 B
- US 674191 A
- GB 2147243 A, Takahashi
- US 4235871 A
- US 4501728 A
- US 4837028 A
- US 4737323 A
- US 5705385 A
- US 5976567 A
- US 140476 A
- US 5981501 A
- WO 9640964 A
- US 5075109 A
- US 4452775 A
- US 4675189 A
- US 5736152 A
- US 3854480 A
- US 5133974 A
- US 5407686 A
- US 6429199 B
- US 6287591 A


### Non-patent literature cited in the description


- **MOSMAN et al.** *Annu. Rev. Immunol.,* 1989, vol. 7, 145-173
- **PAUL et al.** *Cell,* 1994, vol. 76, 241-251
- **O'GARRA.** *Immunity,* 1998, vol. 8, 275-283
- **GLIMCHER ; MURPHY.** *Genes Dev.,* 2000, vol. 14, 1693-1711
- **ABBAS et al.** *Nature,* 1996, vol. 383, 787-793
- **SAMBROOK, J. et al.** Molecular Cloning,: A Laboratory Manual. Cold Spring Harbor Laboratory Press, 1989
- Directed Mutagenesis: A Practical Approach. IRL Press, 1991
- **JONES, J.** Amino Acid and Peptide Synthesis. Oxford Science Publications, 1992
- **AUSTEN, B. M. ; WESTWOOD, O. M. R.** Protein Targeting and Secretion. IRL Press, 1991
- **J. F. COLLINS et al.** *Comput. Appl. Biosci.,* 1988, vol. 4, 67-72
- Molecular Sequence Comparison and Alignment. **J. F. COLLINS et al.** Practical Approach Series: Nucleic Acid and Protein Sequence Analysis XVIII. IRL Press, 1987, 417
- **E. G. SHPAER et al.** *Genomics,* 1996, vol. 38, 179-191
- **UHLMANN, E. ; PEYMAN, A.** *Chem. Rev.,* 1990, vol. 90, 544
- **GOODCHILD, J.** *Bioconjugate Chem.,* 1990, vol. 1, 165
- **YAMAMOTO S. et al.** *J. Immunol.,* 1992, vol. 148, 4072-4076
- **BEAUCAGE, S. L. ; CARUTHERS. M. H.** *Tet. Let,* 1981, vol. 22, 1859
- **GAREGG et al.** *Tet. Let.,* 1986, vol. 27, 4051-4054
- **FROEHLER et al.** *Nucl. Acid. Res.,* 1986, vol. 14, 5399-5407
- **GAREGG et al.** *Tet. Let.,* 1986, vol. 27, 4055-4058
- **GAFFNEY et al.** *Tet. Let.,* 1988, vol. 29, 2619-2622
- **SAMBROOK. T. et al.** Molecular Cloning: A Laboratory Manual. Cold Spring Harbor laboratory Press, 1989

- **RANEY et al.** *Journal of Pharmacology and Experimental Therapeutics,* 2001, vol. 298, 1185-1192
- **KLIBANOV et al.** *FEBS Letters,* 1990, vol. 268 (1), 235-237
- **MARCH.** Advanced Organic Chemistry. Wiley-Interscience, 1992
- **PARR et al.** *Biochim. Biophys. Acta,* 1994, vol. 1195, 21-30
- **SZOKA et al.** *Ann. Rev. Biophys. Bioeng.,* 1980, vol. 9, 467
- Liposomes. Martel Dekker, Inc, 1983
- **HOPE et al.** *Chem. Phys. Lip.,* 1986, vol. 40, 89
- **C. G. A THOMAS.** *Medical Microbiology,* 1983
- **BENNETT, K.** Compendium of Veterinary Products. North American Compendiums, Inc, 1995
- **CREAVEN ; MIHICH.** *Semin. Oncol.,* 1977, vol. 4, 147
- **WHITE ; OLDEN.** *Cancer Commun.,* 1991, vol. 3, 83
- **NEWTON.** *Cancer Commun.,* 1989, vol. 1, 373
- **OLDEN, K.** *J. Natl. Cancer Inst.,* 1991, vol. 83, 1149
- **DENNIS, J.** *Cancer Res.,* 1990, vol. 50, 1867
- **OLDEN, K.** *Pharm. Ther.,* 1989, vol. 44, 85
- **WHITE ; OLDEN.** *Anticancer Res.,* 1990, vol. 10, 1515
- **LANGER.** *Science,* 1990, vol. 249, 1527-1533
- **SEMPLE et al.** *Methods Enzymol.,* 2000, vol. 313, 322-341
- **SEMPLE et al.** *Biochem. Biophys. Acta.,* 2001, vol. 1510 (1-2), 152-166
- **HARASYM et al.** *Bioconjugate Chemistry,* 1995, vol. 6, 187
- **HERMANSON et al.** Bioconjugate Techniques. Academic Press, 1996, 230-232
- Antibody conjugation methods for active targeting of liposomes. **ANSELL et al.** Drug targeting: strategies, principles and applications, Methods in Molecular Medicine. Human Press Inc, vol. 25, 51-68